# EUROPEAN PATENT APPLICATION

(11) **EP 2 151 502 A1**
(43) Date of publication of application: **10.02.2010**
(21) Application number: 08013669.0
(22) Date of filing: 30.07.2008
(51) Int. Cl.: C12Q 1/68

(54) **Genetic variations associated with feather pecking behaviour in avians**

(71) Applicant: Lohmann Tierzucht GmbH, 27454 Cuxhaven (DE)
(72) Inventor: The designation of the inventor has not yet been filed
(74) Representative: Vossius & Partner

(57) **Abstract**

The present invention relates to a nucleic acid molecule contributing to or indicative of low feather pecking. The present invention also relates to a nucleic acid molecule deviating by at least one mutation from the nucleic acid molecule of the invention, wherein said mutation is contributing to or indicative of high feather pecking and is a substitution, an insertion or a deletion. The present invention further relates to methods of testing an avian for its predisposition for low or high feather pecking comprising analysing in a sample the nucleic acid molecules of the invention for nucleotide polymorphisms which are linked to said predisposition. The present invention also relates to a method for selecting an avian or part of an avian for low or high feather pecking and to a kit for the detection of avians having a predisposition for low or high feather pecking comprising a nucleic acid molecule of the invention or a fragment thereof, in one or more container.

SEQUENCE LISTING

<110> Lohmann Tierzucht GmbH

<120> Genetic variations associated with feather pecking behaviour in
avians

<130> P1857 EP

<160> 101

<170> PatentIn version 3.3

<210> 1
<211> 29705
<212> DNA
<213> Gallus gallus

<400> 1
cattcctatg tgttttttgt aacaaaatta taccagctgt tttccttagt cttcaataag 60

acaaggccat ttatacatgg aacttatcag agttcattac tttgagctgt ctcaaggcat 120

tctttatcct ctcccctctc ctcggccctt ctcagatact gtctctctaa gcagggactt 180

catcccaggc accttgtaca ggaacaaccc atccggtttt tgatgggagc acccacccac 240

gcgcagtgaa cgccttccag ttcccagaga atctcactgt attcaaacag aaccaggttt 300

ttgtgttttt ccagttggag ggaggtggaa attactagat gggttctaag catggaaatc 360

tctgaagaca agggtgtctc ctttaaggaa gctactcaga tcacgggaaa agatagggtg 420

agaagaggga gctgctttta ctgcctcttg ttggctgtgc agcatcagct gcctgcagta 480

tgctgtgtga accttgggtc tgcaagtcca cagagccaat ccgtagcctt gcttcctaag 540

gaaatgagtt aagacaacca gaaggtgacg gccagccctc tgtcaggcct gcaggctggg 600

ccagagctcg tgctttgtgt gtgtggtggt gatgggagac tcacagggta acaaacacca 660

cagcagcact gtgcagatta acagggaaga ggtgactctg ctgcagccgg tcttcctgaa 720

gattcctgca cgtgaaggac agaaggcata aaaaggatac gactgatgaa cttcttcctt 780

tccgtcgaaa agaaaagaga acagagagaa taagatgagc ctaaatactc accaaaagcg 840

ccgcagaaac attctgccca gaactgccta caataggtcg gcagcagccc atgagaacac 900

ctgctgagtg gccctgaaag gctgcagccc atgagaacac ctgctaagtg gccctgaaag 960

gctgcagccc atgagaacac ctgctaagtg gccctgaaag gctgcagccc atgagaacac 1020

ctgctaagtg gccctgaaga gctgcagccc cttccagagg caccaacgct gacaagccgt 1080

aggccccgcc accagagccc caaggcccgt ttcccctcac aggcgggccc ggtgaccccc 1140

ccgctaaccc tacgccccta gctgaggcgc tgaggaacgc agtgtaaaca ggcacgccgt 1200

gttacgctgt gaaatgcctc tggttccgcg caggtgcgtt gcacggcgtt gcacggcgct 1260

cacctctcct cacggccgcc atgccggccc ccctcccccg ccgcccgcca gtcctccagc 1320

atcccggcag cccccgcgcc gcggccgttc gtgccgcccc gcccctctcc cttcgggtgc 1380

gatcggagcg ctcggcccgg ttcggtcgct ttcgggcggc ttcgggcagt gcccgttcgg 1440
gagctgccgt tcgggacggc gaaagggaat tcgggcgcgc tcggaaaagg ttcggcacat 1500
ctcggctttc ctcgcgaccg cctcacccgc cttcgggcgg gatcggaaaa gactcgagcg 1560
gactcgggat tcttcgaccc cgcgggccgg accgctcggg gactcggccc tgttcgggcg 1620
ggctcgggag ctgtcaggta tcgcttcggg ccgcgccgcc gcggcctcgg ccgtatcctg 1680
cgctgcgatt ggccgccggc aggaagcgcg ggcccagcgc ctcgggaccc gccgagcccc 1740
ttcggacgag gcctgaaagg actcgggaca cttcggccgg catcggaaac gctcggttcg 1800
acccggggag cgcactcgga aacgctcggc accgctcggc gggagcgccc gagcgacctc 1860
ggagcatcgc cccccccacc cggcggcgct cgggggggag cggcagggct cggggctgct 1920
cgggcgggct cggccccgct cccccccccc atcccgtcct cgcatggagg acgccgactc 1980
ggcggccaag cggctgggct tagcggaggc ggtggcggcc gcggtggcgg cggcggcggc 2040
agaacccgag cggcagcaac cgcaaggctc ggaggggaag gcggaacggc gggaggaggt 2100
gacggccgtg accgtgatgg cggcggacgc cgagcacatc gagatgggcg ccgagccgct 2160
gcccagcgcc gacgaggccg ccgcgttcgc aggtcagcgc gggccgcact gcgcatgcgc 2220
cgctctcttc agcgcagccc gcgctccgcg ctgcccgggg ccgcctcagt gcgggcggcg 2280
ctgccttcac gccgctcctc tctttcacca cacgctgtga gcgcagcggc ggcccgtccg 2340
aagtcactgc ggtggccctg tgtgcaaaat tcccgtttca gagtcgtatc aaaacgcagc 2400
cacgcgttgg tcactgggtc aggtgtaagg cggagcctaa aaacgctccg gggttcgttc 2460
tgctcttcag ccccgttccc tgctttaata aaaagagagt tgttctagaa taaagaagaa 2520
ataaacattt tttcccctta gttcatcgca ggttaagccg tgctcgaccc caagcctaac 2580
agagaacagt aataattgcc ctgactgaca ctggatggga aaaagagggg aatgtgcttc 2640
tatggggttc acacaccggg gtctgctttg cttcatttct gctttgcatt ccagaagtca 2700
ccacagtgac agtggccaac gttggtgcct ctgcagacaa cgtgttcacc acgtccgtgg 2760
caaacgcagc atccatttcg ggacacgtgc tggtaagtct gcccttcagc cgaggtgctt 2820
ccattggtgt gcacatgagg tcaggacatg tgcaggctgc tttgagccac agagctgagg 2880
caccctcagt gctttccctc tctgttttct gtgccattcc acccatgtat gtgtgctccg 2940
tcatggagct gacggcctgg aacacgtagc ctttgaatcc ggttggctga aagctcaaaa 3000
atgtacctgc taactaatgg tgtcttaggg tgcctttgag gagatctgca ggaatgctaa 3060
cttatttggt tctcccttta tttcttttgc agtctgggag aaccgccctc caaattgggg 3120
acagcttgaa tactgagaaa gccactctca tagtggttca cacagacggc agcattgtgg 3180
aaaccacggg gctgaagggg ccatcagcac ctctcacacc aggtataagg cagactgagc 3240
gtcctccggt ggagttgctt cctcttcact ttatttgtgt ggctggaaaa caggagaaac 3300

atcacacaca tgacctgaag ttgcatcgtg cactgtcccc tcttaacgta ctgctttttg 3360

gatttgttgc gtgcctttct aacccacccc cacaagcaga tcgattctaa cattgataat 3420

aaaagcccgg aactaggaga ttaatacttg ggttctttat gaagagccct tgaatgcggt 3480

tttgtctcca ttcaggcctt ttgctggtat ttagttgtga ctgtagctgg tgactttatc 3540

accaaggttg ctcccggctg cctggcagtc cccgttctgc ccacgagatg gcatgacgag 3600

ctaggaggtg ttccagccag ctgcaagtta cagcctgtgc ctttaatgtg ctgttaaaag 3660

catgttctct agcatgcaaa ctgctctatt tccagccatt ttcagaatgc taccttgtgt 3720

catacttctt gaggttggca gggcagaccc atggctctgg cttacatcga gcacctgttg 3780

cacacaagtt ctttgtggca ttctgaccac agaacgcatt tggctcagcc aaactacatg 3840

tgaaggtgtt gtcactgtgc cttttggttg ctgtcacctt cttccttttc tttttttttt 3900

tcccaaaggg ccgcaatcac ctcctacccc tttagcaact gtccaagaaa aaactggaac 3960

caagtataac tgggacccat cagtgtatga caatgagctc ccagtgaggt gccggaacat 4020

cagtgggatt ctgtacaaaa acaggctcgg ctcaggtaaa aacagccttc ttgtgtgagg 4080

aagatgttgc cttcttcact tactgcactt tccttttaat cagattgcct taaaggccca 4140

atcatgaaga aaataaggac ggtttcctag tcagcatagt cccatggata ttattgtgct 4200

cttctgtttt acccttttgc ttccttgttc tttattcaga ggctttttat ctgttaaagt 4260

acagcctgct gtgcagtgag tcttcccaat agtaggctga ctcttccaat gttctttttg 4320

tggacctctg agaagtacac ccttgtctct acaaagctct gcagagtccc tgctggattg 4380

gaaagcagag agcttgtctt ctaacgagcc tccttagctg tcttagcccc tctctttgct 4440

tttatctctg tgggaaagct gaagaagcag ttgcagaaac tgtcagcaag cctgactgca 4500

tagcatagtg tttgctttgt ggaaagagag ttttcttcgt gaccagacag attagaaatc 4560

taagcaaaaa ttatggaaag gcttaaattc atggtagtta tccaaaaaaa gacttatcct 4620

atgtggggat gagtaatatt ttctagccta ttgacgccgg tgatgcacaa aattggaatt 4680

cacggtgtgc acagtcattg tcacagccag gctgagaaat ggtcatgctt atccttcagc 4740

aatgggaagc agcagatgag caggtttggt agttaccata gattacctgc tgtttcgccc 4800

agtaatttca caatcacaga atacccggag ttagaaggga cccacaagga tcatcgagtc 4860

caacttgcca ttagccagag cctgcagaga cccttcctgg ggctgctctc cagcctttgc 4920

tccccagtct gtacacagag cccaggactg ccttgttcta ggagcagaat ctggcacacg 4980

cacatgttaa acttcatgca gctcatcatt gcccagcccc tgtctgtatt ctttagaaaa 5040

atggaaaagt aatccttagt ctttactgat acttttattt ttttcttttg attttaatac 5100

aggaggccgt ggtaggtgca ttaagcaagg ggacaactgg tacagcccca ctgaatttga 5160

agctatggca ggacgagcca gcagcaaaga ctggaagagg agcatccgct atgctgggag 5220

gcccttgcag tgccttattc acgtaagttt acgtttgttt tctcctgcct gctggaaggg 5280

gcaggttaat tctagacaga gtctgccctg cagtccgagc cgcgtgcaga gttgtgtgtg 5340

atggtgatgt ggggtgaaga agaagcaact cattgatttg gttgagggga actggccttt 5400

cttttattgc gtagggtccc cagaagggaa ttagtgttta tttctccctg ggagctgcca 5460

ggatctttga gcagagcaca gtcctgctct cgctgctttt ggcgcctgat gctgaacttg 5520

taatcttcca atttcaggat gggattttaa atcctcacgc tgcctcttgt acttgtgctg 5580

cctgctgtga tgacatgact ctggtgagtt gtgggtttta tatccattag tttaaaaaca 5640

attcatcaat caagctgcca tggctatctg tgatcaggtc aggagtgtcc tagaaacact 5700

gacaagtact gagtatttcc atatcagact gcttgctttt ttgtaaaatg atgctagagt 5760

tgttaagcag cagtatttag cttcacctca agcactgttt tgtgctcttt aggtgtgaac 5820

tggatggttt ccttgttgcg tagacctgaa actgcatcct tcccatcttc tttcactctt 5880

atttcttttc ttcaaccccg tggattggta gtgaaggatt tgcccagttc ttgtcctttc 5940

ataacagtgg gaaaaggctg aatctgagtg taactttttt actcacagag gcttttcagt 6000

ttaaagatcc agtagaaact tttggtctta ttgttgcaaa tctaccgtgg gaatacttac 6060

tgcttaaatc gtagctgcat ctagagaggt tggagtgaac tgatattccc ctgagaggtg 6120

cctcagctaa tgtctttttc tctttttttc ccccctagag tggacccatt cgactctttg 6180

taccatataa acgacggaaa aaagaaaatg aaatgtctgc aaatccagtc aagaaggaat 6240

cctccaaaaa tatcactctg cttcctgcca cagctgctac aacatgtagg ttctttgctc 6300

ctggtaaaaa tggccgagct agatatcttt ttgtcttatc agtggtcaac tgagaaaaat 6360

gtattatctc ttggcgcttc cttttatatg tcctttaata tctggtgggg ccgtgtggta 6420

atatttgtgc tatttcttga aatacatggg aaggtaggcc ctaagcaact gtaatgaaat 6480

ccacagtctg aaagctgtta gtgaaagagg cagaaggtgg tgctttcatc tgtaagctcc 6540

cctcttgcat tcagtacttg ctttgtcact tcgtgttttt gaagtcaaac catctgtgtg 6600

agaacagaaa ttaatttgca ggctggtcca gctcacaggt tccctatcac attcatgtcc 6660

tcctgttctc atctcatcat ctggaaggta acattacaaa gtgagctgag gaggaagcaa 6720

caggaataca aaaaagaaca gagcaagcca aacgtaagat tagattacta gcaacagcgg 6780

gaagagaaat tcataaagat tctgcagaga ttttgttgtt tccatccccc caaaacagct 6840

ttactctgta ctgttttgtt aacttgtgct gctggagggc tgcatgcttg cttgtgctag 6900

cactgggaga gagcagcact gcttgtcttg ctggcagcca tgtctttcct cagaaacaga 6960

accgtagcct cagtgtctta gagctgaact gtttgtataa aggtctttca ggctgagcag 7020

cgctctacag aatcagccca ttagtggtgc tggactaatg cagcttaaaa agggttttaa 7080

acacgttgga gctgcactgc tactttcatc cagatgtact ttgggacttg ttccctgcct 7140

tggccaagca tggttatatc actccatgta ggtggacagt tgaacttgtc cagtaaacaa 7200
aactcataga attagctgta atccattggg gtatttgaat caattttcaa gcagtaagaa 7260
ccattcttcc taaagtagtg tagtatatta gggccagcaa tgccttcaga agccatgcag 7320
ggctttgatt cccatttctg atgttagtgg ccttaggggg gggatccaaa cttggctgtc 7380
acagctcgtt gactgatctg tgagttctgt gactttggga acagggatct cagcagctag 7440
acagctgagg cctgagtgct gattatgaaa gtgcttctgc tatttccagt caccgtgact 7500
ccttctggac agatcactac ctctggtgct ctcacctttg accgcacgtc gacggtggaa 7560
gccacagcaa ttatctcaga aagtccagcc cagggggatg ttttcacagg agccactggt 7620
aaggattgag ctgcatgcct gcttgacatc catgagaagg tagggaaaaa aagaagcctt 7680
ttgtatgctg catgaaggat tttagttttt aagaacttac acagatgctg tatgtaacct 7740
gctgtcacaa aagaagtgtg aatgttctgt ccagatgctg cagatgctta tgtgcacctc 7800
tattgatttc tttggtttta tggttatctc catggttttg ggtttttttt ttcctttttg 7860
ctacttgttc atcctaaaat accacccaaa aaagtagctg agtgtttaaa ccaagtggaa 7920
aatgctgaat aaatgctgga aaatagcaaa ataaagaaaa cagaagtgct taccataaat 7980
ttcagctctt tcagtccaat tcctgcatgg ggctgtgggt tctgcaggag cagctgagag 8040
agctagcatc ctctgagcac aatagcttgg actcctatct gtgtaaaata aagtgtgtga 8100
ggaggagact ggcagtaatc tttgcagaag ccttgagatg tgaaagcaga gagagctgaa 8160
atcagtgctc agttgctttg cttggtgtcc acgtgacatc ccgctgctgt ctctcacttg 8220
caacctctgg aattaccgta gagaatagga acctgaagaa aatgtgaaag caaaaaaaaa 8280
cccaccaacc caaagaggtg aaagaagctt aagacatacc tgaaagctgt gccatgcagt 8340
gatgtgtaac gttgttgttc tcctgcagtt caggatacca acgtccagca gccgtgccgg 8400
gtcgctcacc cagagcctca ctacccaact taccaggaca actgtcagat ctcacctttc 8460
cctgaagctg cgctgccgac atctcatccc aaaatcggta tgcttcgtgt cccttgtgtc 8520
aggcacaatt agcagtgcct gagtttacct gggagccttc ttttccagca aggggtagag 8580
attgaagcag gaaaataaac cttcattttt tccttgtggg tttcgcagtt aagatttgga 8640
tgaagagatt ttcttccccc cgtgctttag gcattctgtt attgacaatt ttgatttgtt 8700
ggctcttggc agaagaagct cttgtgatct tgtttcctaa atcttcgcag ctccctgcct 8760
ttcagtaggc ttgtgcttca aagattttgt cactgcttgg gcactgtggg tgggtatgta 8820
tttaatcttc gatccaagaa agccatctcc tcacttagat ctaaagctgt aggagccttt 8880
ctgaaatttt ccacatctct ttatgaatcc tctagcttaa ggtgggggca gtttggagca 8940
ggtttgctgc gctgctctgg tgtcccatgt cagttttcca tgtgttactg ctgacatctg 9000
tgctggaatg atagaagctg gttaaggagg gcagtggggg tctgccctgc tgcagtcaga 9060

cagactgcag taaaaaagaa aaggtgatac aagaaacaac gtgcaaatca gcccttcagt 9120
aggacagggt atgttgatga tcaggtagat atctcttcag atgtaattaa atcatgtgaa 9180
cttaatgatc tttcattcac tgttttattc agagcagcac agtgcattcc tggtttgtcc 9240
agaatgcaat cttgttggtt ttaagcctta ctcagctctt gatgctatta attaaacatt 9300
gattattttt cacccgtgaa gcaaagatca ttactttccc ttggctgata cacatctgct 9360
aaaacgagga gtcggaagta attcctgtta gataagttat caaataccgt gccatgataa 9420
atgctcctct gaaaatgcag cagaggaaaa cattcttctg caaacagtta ctgataaaag 9480
actgatttat cttgactcat atggaagtgc aaatagttct ccgccaatca tgggaatgga 9540
gcaaaattac tgttttaatt aagggccgtt gagtgtagaa tatcagctca cctgtgtgat 9600
gaaccttcag caaagtctga aaactctgca catccctcac acggtgacct gaagtacact 9660
gtgtggaaga gacatattcc ctctccccca aagattttgt cactgcttgg gcactgtggg 9720
tgggtatgta tttaatcttc gatccaagaa agccatctcc tcacttagat ctaaagctgt 9780
aggagccttt ctgaaatttt ccacatctct ttatgaatcc tctagcttaa ggtgggggca 9840
gtttggagca ggtttgctgc gctgctctgg tgtcccatgt cagttttcca tgtgttactg 9900
ctgacacctg tgctggaatg atagaagctg gttaaggagg gcagtggggg tctgccctgc 9960
tgcagtcaga cagactgcag taaaaaagaa aaggtgatac aagaaacaac gtgcaaatca 10020
gcccttcagt aggacagggt atgttgatga tcaggtagat atctcttcag atgtaattaa 10080
atcatgtgaa cttaatgatc tttcattcac tgttttattc agagcagcac aatgcattcc 10140
tgctctgtcc agaatgcaat cttgttggtt ttaagcctta ctcagctctt gatgctatta 10200
attaaacatt gattattttt cacccgtgaa gcaaagatca ttactttttc tcggctgata 10260
cacatctgct aaaacgagga gtcggaagta attcctgtta gataagttat caaatactgt 10320
gccgtgataa atgctcctct gaaaatgcag cagaggaaaa cattcttctg caaacagtta 10380
ctgataaaag actgatttat cttgactcat atggaagtgc aaatagttct ccgccaatca 10440
tgggaatgga gcaaaattac tgttttaatt aagggccgtt gagtgtagaa tatcagctca 10500
cctgtgtgat gaaccttcag cactgtctga aaactctgca catccctcac acggtgacct 10560
gaagtacact gtgtttttgg agacatattc cctctcccct aatctcttgt ggattctttc 10620
aattttcatg agtatttatc gctaagagtt caattttgga aaatcaaaac cggtaaagcc 10680
agatgacatt tgttcctgga attgctaact gatgtgagct gacactgcgt gctgttggaa 10740
tcctagatcc aggttttctg atgcagtctt tggaaaatgg aggactgcag agccacagta 10800
agagaggggc tcagggctaa gcaaggaaag tgcgattagg taccaggaga ggaggtcaca 10860
tttttttctg gtggcttcct acttggtata atcaatgtgc agttactagc agggccatgg 10920
ttggggttgt gaaaaaataa aggctgcaac acctcagtag tttgggtggt cctttaatgt 10980

gactttttgt atgcttagga gaagtactgc tgggtttctt taacattcgc cttcgtatct 11040
atgtcattct agatgacaca tgagaaaaca agtgaaggag tccaaagaaa ttacagcagt 11100
ggaggttttc tttttaggag catctgaact ggtggttttg gaaactcatg tcatacatgt 11160
aatctggagt aatgataact gtcttgcttt gatcccagtc ttcagccagg gttgctgtca 11220
gatcagagtt caggaagcat tagatgtgaa ccttgtaaca aaacaaaaca cgaaaatcac 11280
ttcatggaaa cgcagtccct aattctttgt gttttatatt cctttctttg tgctcagtgt 11340
tgaccccaat ccctgccctg tcggtgcccc cccagacccc caccaaagcc atatccccaa 11400
cagtggtgaa tgggctggaa gtgacggaac agcggagctg gctctacttg gaagagatgg 11460
tcaactcact gctcaacacg gcgcagcagc tcaagtctct tatcgagcag gccaagcagg 11520
ccagctcttc cttccgcgag gccgctgtca cgcaagccaa gattcaagct gatgtggaga 11580
gaaaagaggt aattatgcca caggaggtga aatgggacgt ctttttggca gagttatgga 11640
ggtcttttcc aattgtaatg attctgtgat tttatgggtt gtactttgct ttttttccac 11700
tgggtatagc tttggtaacc atgacaaatt gtttctcaat gtcttgagag cagagaaaaa 11760
ggtaagttat tcccacgata ggaaaaggag tagagtacct gggattccag cagctgtggg 11820
tagcagtaat atcagttgtt tgttgtttcg ggtcctgagc tctgccatca gacattcatg 11880
catttagggg gcatggattg ccatatgtcc tggtatcggg aagatcatga gtgctcagcc 11940
ctgcagtggc tgcttccatt ctctcaagtg aggagggcta tcaaataggc ttttggtggc 12000
ctgaagaggc agagaggtaa taagaacagc aagctgtgga gtttattggg aagataacca 12060
gcaaaattta gaggcatctc gctgagcgtt ctgcaagcaa agctgatctc tcttgtgtcc 12120
tgtgataaga ctgcaaagcc agctggcatg atgagagtat tggcttttac ccagcagctc 12180
tttcctcctc tgtagggaat tatctgcaag gtctgcccta ttctccctgc ttcttttctc 12240
cttttttaat gcatggtaga gaccctaggt tcctcttaac acctaactcc atgcgctgct 12300
gaacgcattc tccttctccc acagatcccc agctgctcct gtgactttga gggaggctgg 12360
agaggggcaa tgtactgtga cactccgacc cttgtttgta gctgaggaac tccctttcca 12420
tgcaccacac tatttgctgt tacgttattg ctaatgtcag cttccactga attcatagca 12480
ggagggtgca gcaagaggcc ttgaagaaat gtgtgtggga ggattttgct gagaacccta 12540
tgacagtccc tgcagcagct ttgatgttct ttgccacgtt gtcactgcta aactcttctg 12600
tacgctgcat ccctgttgtg caggagtgtg atacactggg agtctctttc tcccttgcct 12660
tcagtctgga tagagcggtt atttgttctg ctgtccagat ttgggctgtc agctgctaaa 12720
agctttgtag atcgtgggac agcacaaaat tggaagcttt gacattttat agatggacat 12780
cttactttgt ttaactttac cctctgctct tcttccagtt tacataccgt gatcccctct 12840
gtttatcctt accgagctta atcgaaacag tgcttcagag tccatccctc tcaccctctg 12900

atccaaatcc atctcacttc agtgtacatc ttcttgcctc aggaacagga cttaaaatca 12960

ctcctcggta ttgatcttta agtgctgact tcacaaggca aggatgagca tgtgcttatt 13020

tccagaggaa gtccttttcc cagtaactgt tgaattccta attactctct ttgtgaatct 13080

taccactctt attggtaatg ggttccccaa atgttggtcc cggtggtatt ttttaaaatg 13140

cccagataat aattctcaag cttgctgctg tgttatggtt atgtgttatc tgccagcagc 13200

tgggcaatcc tgcaagtagg tattctaatc cattccagtg agttccatgt tttttgaaaa 13260

atgagttttc tgggagcaca cctggcattg caagcagtta cagtaaatgc tgaataatct 13320

cctgtcagct tttgtgttgg tcagagttgc cttcccagtt tcgtgaatgc ttccatggtt 13380

tttaacatgt ctctttccct ttttgtctct ggtccagcca cttcttctgt cttttaagtt 13440

gtgtgagggg gagaaagagg tgtcatccta ctttgtggtg ttgctgattt ttcttggacc 13500

catttaatac acagttgtgc cagtgtgttc ctacaaattt agaattgaaa ggttgtatgg 13560

ttatatctga gctgcagagc caaggcttta caacagatta aattataata gagctgtaga 13620

ctcagctctt cctggaagag tgactgcata gagagcagga ttaatgaaat ctgttagtgg 13680

gagcaagctt gttcccaact gtatatacaa acttcagtgc gatgactgca aataatatgt 13740

atgtttggtt ttcacagcag tgcagagcgg tcagcagcct gtctcttgag gatattgagt 13800

tgaagagctt ttgatcctgt aactgtctgc atccctccct gctaaggagc catgccttct 13860

ggcagaagag cgaggcagag ggctcaggac agcacaccct ttacctgccc ttcagagaaa 13920

gtggccccaa gttgtactct ctaatgacag cattaggtgc ctgtgggagt tacagagccc 13980

tgaaaggtga gaggtaggct ttttccccaa gctttcctag agccagagag gcatttgtgc 14040

tgcctgcaga cttttgtcac tacctgtgaa gtgaaagagc aggcatcact ctttcatcct 14100

gcttccactt tctaatggtg ttatccagga gtgttcagtg gagtattagg aacgttcccc 14160

ttttctacaa gattttgaag gtggaaatgc caacatcaga gagtagctgt gacaggaaga 14220

ggatttgcta aaagtagcaa ataagaacca taataaattg taagtgcagc ataatttaga 14280

agcatcaacg gtcacatttc tttcttaaga tggcatttct aatctgaatt ttgcagatta 14340

attgcatttc caaacgcatg gtcctaccta gagacagcat cttcaaaagc aacagagttg 14400

ccgaatgggg aagcattgag tatttaggct gcacagaagt ggaatataaa agcctgtctt 14460

catataagat tagaaaaaat atccaaaaat atacttgtca gatgtaaaag aatggaagac 14520

atgagatgtt gtaatttgga agtccccaca tgcactctat tactattttt tgtgcttttt 14580

aatttctgta tgtaacataa caaaaagcca cgatagggtc tctgggtata agaagttgtg 14640

gaaaggcacc agctagtcaa gtcctcaaga tatggactat ttgtaacctc taaatctaca 14700

gttccagcat ttgtgagaat atctcctttg cagtcatttc ttttaagaca gagctgttca 14760

ggagcctaag gaagaggctg aaataacctt tcatgttgca tatgctgttt gccgcatctc 14820

atgtgactct taatgaggac agctccttaa ttaatgagca cagccctcac agagttggtt 14880

cagacacgaa actgactcta tttgggaaat gtggatgcca taaagcttgt gctttcagtg 14940

agtaaacacg cagctggtgc tcagcctgtg tgtggctttg tgctggcagg gacaccaagt 15000

gtgtccatca gtaacattga tgttatctgg aagttttgag gaatgatgtc ctaaaagaaa 15060

accaagccat gtgctctcca gctcagtgtt tatctaaaat gcatcattac acaaaattaa 15120

aatatcaaaa tacttgtagg ctttatagaa ttgcaacatt ttgcatggga ttcactaagt 15180

acaactgctg tgtcagaact ctgcacagct gttcagagag agctctactc tgttgtaatt 15240

aaatggctcg tagatgagga tattggatcc tttggcttct gctgggattt tggctttact 15300

gcagttctta attaagggag aagaaaacaa ttaggaagag ggtgtatggg gcaagaaagg 15360

cttagggtct cttggaagag atgtttgacc acttgtagta ggattggccc caaaggaaca 15420

gagttaaggc tgtactttgc acaaagatct ttggaaatgt aatttcttct gagagtgcac 15480

gaagccgttg tttgtaatag atagacttca gaagggggag aacacatgaa cccaccgagt 15540

tctgtgctgt agaacacagc tggagggtgc agatcttcag gaaatgtgtt aggcagtgtt 15600

tctgtgtaag gataaaaaaa aatcagagtt gaaatctaac atgttttaat tgcaattgtc 15660

ctctttttgt gatgcaggcg tgtctacaga gccctatcca tttgaaaaca aggtggaaga 15720

ttaaatccgt tagcagctgg gccatctgaa ttattttttt agacagatta aaacagttca 15780

gggctggaga gtatgaaatt aacctaaaaa ggattgggca cttaagaaaa gtagttccag 15840

taagcaagtg atgacttata tcctccaaag cagctgtgga aatggctgag agctggggct 15900

catcagtttg ctggaaatga ttttgcaaga aatctgtgtg tgtcaagctt ggtcgtctct 15960

gttctcactc ctttcccttt tccacagttt ctcaaagaag tgactgattc acaccgtggg 16020

ccaggcagaa ccagagggct cagagataaa agggaggtag aatggagtta catttccatt 16080

agcaggattt ggggacccag agcagagctg aagggggtgc catgggtctg acaagcacag 16140

gaggtggctg gacttcctct cggcttcctt tctctttggc aatcccactg gaaaacagga 16200

taagccacat ttgtggagga tgcagtggat tctggagctg tggcagctct cagaactgga 16260

attctgtatc ttcagcccaa gaactgtctc tgtgcaggtg tcacagaggg gctgtagtag 16320

cagggtatat ttatgcagat attttcaaga aatgctatct cacacgtgct ttggcatctg 16380

tacgtacact ctcctcacca agaagaagtt taaaaacact gtgcagtgca tatgtactgt 16440

cttgctaata agcttgatct tgcctctttg tctaattgtg gtagaaccag gtcatagctc 16500

ttgcaccaga cagctttccc tgcctcctcc tccatcaccc cagagagctt tttcatctca 16560

tctttgtgtc acagtaacaa aaaggttctc aaggttgcaa cttcacagga gaataaatac 16620

tgagatgtca ggtcgtggag gcacttaaat caagccttta atctcctcac ctgttgggtc 16680

atatcactcc ttgataaacc tgctttgcct gaaggtgggg gtttgttttt ttccttactt 16740

tctaactgtt cctcatggag tttagatcag gtttagcatg taaccaaatc ggggccagtt 16800
ttagatccct ttgtttgagc actttgcctc tcagcagcac tggctgttaa gcatggcagg 16860
acaagggtgc aggagcactg agctcgactt cctttgcagc gcatccgaag cgtactttaa 16920
aattcacctt cagtgggctg ttaatggata aactgtctga aagcgttatc atcttttttt 16980
ttaaaggaag aataatcatt atgcttgatg ttgtttttga agtcttgggt gtgttaattt 17040
tgtcttgact ttttttttcc taacccaatc ccataccttc tgggcagttc tagcccacag 17100
gattctccca tgcgtgttgg tggatcagga tcacaatgct cggccctctt tgcccaggaa 17160
gatggcacgt ggccctattt ccatgacatt ttccttgggt ttcctttgct aatttgtcac 17220
tgtcttgtgt ggaatgcagt tccttgtaca cagtgaagcc acatgtcttg agattggatg 17280
aagtaaggct tgaataaatg tatgtaattg acagtttgtc accttgttac tcctggcttc 17340
ttcatctgac tccgatgtct gagtccctct tctgtaaggg aggagtgagg ttccctttgg 17400
tcctcacaga actgcatgaa ccctccgtac agacctcact tttcaagccc ttgttgcata 17460
ttgtggctgt gaaatgacac acttgctgtg ttttttcctt gcagcaatat cagaaccagt 17520
tatttcagca aacagaagat gtggatggga aaacagaaat catcatcaag gtaagctctg 17580
gttgactcat gttttatgag gagtccgttg tcttgttgca tttaaagatc cagaaatcat 17640
gtattctgtg gctggcctca tcttctgagc cttccctggc aggacaggtg ctctgttctg 17700
gcaaagtgtg ttctatttcc atattttcac agtcccttat ggctctctgg caaggttagt 17760
cagtctgagg cacctccaag taatggcagt gaagcaccaa ctggtgggta tcactgcagt 17820
gtgataggaa acttctttcc tttttgctct gtaagctagg tgtcaagccc cattttgata 17880
gcctcgattt atggctggac atcagaaaca caacagtggt tcagtcccac cacctgctaa 17940
ctgtaggtac ccctttgtgc tgtgagccag tggtgtgatg agctcggtgc tgatgggggc 18000
actgatggct ggattggtag tgctcaggag agaaatgagt cagcagcact gagttgtgct 18060
tgtgttgggc actgcactga tggtgcttct gaagctgaga aaagtttccc ttggcagcct 18120
ggcaccactg ctctgacgtg ctaatgatgt gtcagtaact taatgagctg ggaactgaat 18180
gttctgcaga gtgcttattt ttgtgataac tcagcgagat ctccacctca ccatgcaggc 18240
tatgttaatt gatgtcactg gtgtaagatt aaagtgctag acacaaaagc atcctttaaa 18300
aaccaggtgt gattttcaat ctccatatgc tacctcatcc tctgggctta agtcctgtac 18360
tcctgttaca gtcttcttac ttttactcct ctgccttctg cttcactaca gtcacccctc 18420
cactgttgtt ccctctgatg tggtcccttt cctgcttgtt ttcacagcag tcgtgtgtca 18480
actgtggccg cgaggcaatg aatgagtgca cgggatgcca taaagtcaac tactgctcca 18540
ctttctgtca gcggaaggta taagagcgtc tgtgagttgc aggtggagct gtgcagaggg 18600
gacaggtgac actggggtca gcctgtgtgc aggctgaggg cacagcacag acagaaacat 18660

gctgtggagt ggggggggtc actgtctgtg agccagggcg cagtgctggg gtgggtcaaa 18720

caggtaagga gtggccccaa gatctgcaca caccttctga aagaggttat caaagtgtga 18780

gtacaaccag cagtgaggtg tggacacctc actgtgtaaa cattggcgtg agctgggtgg 18840

gagtgctgta caagccctcc ctgctgctcc ctggaaacct gcactctact cagtacctct 18900

gatcctgtgc taccgaggct gtagttctct tatttttcac gctatttgtg atggtatgga 18960

tgataagatg aattgagcac acagacaaac gataaagctt taacatggga ggtggaaaca 19020

ggtatgaaga ggaagctgca aagttttgtg tttctcactg cttccttaca aagacctttg 19080

gcaccacttg ctttagagga gaggataatt tggtttagct acagctctgt gacctggctc 19140

tgtgacctct gggttcattc tttgtcaact gaatggagca cctgaagaat tttgactgtt 19200

tctttggcct gtaccagagc gtaatgatgg ccgtccctgc acagcaggaa ggctagaatt 19260

tcagaagaaa gattgcttct tgcagataaa ttacaaaatt atcaattatc aatggcctac 19320

cgtccccagg catctcaaag acagtaattt caccccgttg ttctgtcacg gaacatgctg 19380

ggaggtgtcg ctgagtggtc atgtgctggt caccctccga ggcagcttat aactgctctg 19440

tgaagaagcc tttaaaggga gcttatggat gagagggaga gcaactcttc ttacataggc 19500

agatagtgat aagacaaggg aggtgtgatt ttaaactaaa agagaagaga taaatgttag 19560

acgtgaggag gaaattctta gagggcagtg aggccctggc atgagctgcc cagagaagtt 19620

gtgggtgccc catctccggg ccatctcttc atggcctctc catctgctct tccctgcagg 19680

actggaagga gcaccagcac atctgtggtg aggccaacgc agtgacagta caggctgacg 19740

atgtgcatgt tgcggacaat gtgatggaga aagtctctgt ctgaacacac ctacctctga 19800

ccccatggca gcgtggtgca gccggctgga tcggcgtcct gatgtgagct gtctgtcctc 19860

atttctgtaa gactttttca tactgtgaca gtgtttgcct acttccagtc ctactgcaaa 19920

cctctggata acgcagagac tgagaaatcc ggatctggag gaggcaaact gcagcctccc 19980

gtctgggagc ctcctccagc ttgaggaagc ttagattttg ctagactgaa atctccactg 20040

tgtggataac aacaacctca tcgtctctct tgagatggtt taagaacagt tgctgcaatt 20100

agatttgctg ttgttctgtt ttccccatgc ctccctacct cttatttgaa aaaaggaatg 20160

atccctcatt tggtttattt aattgccttg aaaaagtaca aactcttaat ttgcctttga 20220

aaggactttc ttgttttgtt ttgtttttct gtgttccctc tgtgctgggg aaatttggaa 20280

cacattggag cttctgcact tgaatatgag tgaaagatca catcttgttc aataaaatgc 20340

gatgaaagca cagtgtaacc ccatggcagc accttggcac atggacaaat gcagcatgcg 20400

aggggtgtgt ctgcagggag ttttgcacag aggtgttggc tcttagtctt tgcaggacat 20460

cggcgtggat gtaacgtggc agcggggagg agcagctctc accacggcag catgccctgg 20520

ctgcagtgcc aaaatctggg caacctccaa ataagagtgc acagatagag ttctggcctc 20580

tcgctgctgc ttagtagctc ctaattaaag cagtggtgaa cattaagagg ttattggctt 20640
cattcgctac tccaaaatgc tttaattaat agtgtgctgc tttaataatt taggctatga 20700
ttgctgttat ttactttcta gttgctcact ggaatgagtt tccctcttgg tgctgagggt 20760
gaggaccagt acggatggaa ggaaggcagc aacgtgcccc cctcctttca aggagaccat 20820
tccttagtgg gaagccaagc atgagcttag tgggctgaga cagaaattac acatggagaa 20880
ctctggggag ggaaataggc aattctgtaa aatcccatcg cgagcaacat acccgctctt 20940
caagcttccc caaagtcatc cttcagtatt tacagtcatt tcttcttaca cgatgagtgc 21000
cagttttcta aatctttaag ataattactt aatggaaaag atgctcttaa ttgaacccac 21060
aacaatacaa ctaatttaca aggtttcttg cctccatcta ttgcccatta aggttctgac 21120
taaaggaagc agtgcgtcgt gctgaacacc gtgtgctgtt tggtttaagc acccatttgt 21180
tctcagctgc atcttgtatt ccccccagag gaacatccct gcagagcact gctaggctgg 21240
gagtagagct ctgtgttggt tcagagatga ctgcagctac acaaagacgt gagctcagcc 21300
atacccatgt ttgtacagat aactgagcgg caatttatac taatgcagaa agctctccag 21360
tatcgtcgct acgcagtttg tggatgagta tatccaatgc tttggttata gtctgaattt 21420
tgtttttatt gcatcttctg attaaggaga ttctgaagag catgagtggg acaacagtcc 21480
taaatgaaga tgtgtaggta cgactcatgt gtgaataaag ttctctagaa gcttctcatc 21540
agggccctgg catctgggag agctactctc tatagcacag tgaactcgta agactgcagt 21600
ataatcgttg tggcaacgta acatgtttat ttgttgatga taaattgatg aagcaatgtt 21660
atccttagtc gaagaaaatc tcaagacctc ccagcaagtc ttcttttatt tctaagcaaa 21720
caaattgccc cttggaaagc aacagccctg catgaaaatc attagtcaga agtctgccac 21780
ttgtgcaata tccgcagtct tgtggggtct cttgatgtct acgtcaagca ccaagtgtta 21840
aagaagtggc tacgcctgca gcttccagtg gggggagtat ccaagccgta accactgcac 21900
tcacctgtaa ctcctcacag tggagacaga gggctgggaa ccaccatcct ggagctgcca 21960
tcctggaaca atctttactc cttccctcct ccccaacaaa gacagcaaac cttaggcaga 22020
aatagatctt taatgaacta tacaaaaagt tatcctggtc attccctcca gttccagtga 22080
gctcagcaga agaggtgcaa gactttgcgg aagaagttcc tgaactcggc gttgaaaaca 22140
gtgtaaatga tggggttgac agcgctgttg acgtaaccca gccaggtgac gatgctggtg 22200
acttgagtgg gaatggtgca ggacttgcag agagcccttg taatgtggac cacaaaaaat 22260
ggtgtccagc agaagaggaa agcacctggc aggagaagat taaagagaga aagcgttgtg 22320
ttcaaaaggg ttggagttaa cgaatgcaga gctaccatct ctccgtgaca aaaatttggg 22380
gcaactacct aaatgttggt gaagaaaaga tgtgagtggt gtgtcatttt tggattagca 22440
tttaaaatac caggagtcac aaatgctgac agctgaagtg aaggttgaat taaaaaacac 22500

cacacacgtg ttttctccct ggagcgatgc tactctggat caaagggaga ccgaggcact 22560
ctctggaccg ggttcaacaa aatcctgacc cctgtaggac cacacagcac atttcccacc 22620
ctccttgacc agcctctgcc actggccact caccaacaac caccggcagt acccgcatgg 22680
ccttccgttc ccggccgttg atcttggctc gcttctgacc gtgtgccggg tgtgggtact 22740
tgaggtgtgg gtaagagaca gtctggagcc cattgctcat cacatagtcc ccagaaaggg 22800
ggtgctcagg gtgggcataa gggtggtact cctccggttc catgccgacc cgctctctct 22860
caatgaaagg tgaggggtga tagagcattc tgttgccccc gtagatgcca ccccttagct 22920
tggccttcct ggcttcttcc cagcgcttga gtccttggaa catggcgcag tagagcacca 22980
gcatgacggg gcaagggatg aagaaggagc agatggagga gtacacgatg tagttgtcat 23040
cctccagctg gcacaagctg gggtcccggt ttgggacgtt gttgaggcca aatatgactg 23100
gggatgccac agcaaaggcg aatatccacg tggtggagat gaggatcagc tgccgcaggt 23160
cgatctgtcg ccggttgtag ttcagtggga ctgaaacagc gataaacctg ccaacacagg 23220
tgggtcagtg ctcgtgtggg ggtagcacca ctggtaaggt aagcgaatgg aagcagcttg 23280
atgctgagac atgatccctc tcctctggtg taaggaggag gaaggcactt accgatccac 23340
actgatagca cagaggttga agatggaagc cgtgcacagc atcacatcca tggtcatcag 23400
ggcatcgcag agcacagtgc tgagggacca cacgccaccc tgaaactgaa cgacaagatc 23460
cccgggtcag taggtgcctt taacatgcaa ggaaacagct ctgtatacat gtgtataaaa 23520
tgcccctggg agcatccttg tggcagggct tgtcctctga ctcctgctcc cgtgttcctg 23580
gggcagctct gtaccctgtg ctgggtacca ggactgaggc cagggaccaa accaaaggct 23640
tctcccagct gtggccctga gcaacaccac tggactgaag gcttctattt gtgtcacatt 23700
tctgaaccat cctttgagac agctctgtct ggcgctacag cagagagaga ttagggttga 23760
aattccctca gggctcttcc ccagcttgaa gcacagtctt tctaagactg gtccaagtgc 23820
tttgtgtctc cctgtgcttt ttgggtatct ggtgacctgt gtgaacttga ggtttttgac 23880
attcatggtc ctccccattc acatttccag gaggaggcag ctctgccagg attagtatca 23940
gtcagtactg atgtcatgca caggattatg attagtggag ctgcaaagcc aaaggagact 24000
ctggagaaac tgtttggcta tttttatata cccatctcct tgagaggccc cgcaagcttc 24060
cagaagactg ctggactcag acctgggtgt ttcattcggg cttttctcta gggcagatca 24120
gctgctccaa gcagaatttg ggagctgaat cctgaaacac aaccctcaca ttttttacca 24180
aagcttcacg aggatcctga ccagcttcat tgcagaaaat ttgacttggc tgtggtgttc 24240
aaaacccatt tgtggtttca aagaatgaat gggttctgaa caggccccga gttgccaggc 24300
tgcagcacct tccaaggaca cagcctttgc tgtggggctc atgttgggca ccctgaaagt 24360
tgagcactcc cacaaaatgt gcccggaatc cctctggtgg aggagtgact ttggattaga 24420

ggcagagctg gctttgcttg gtcatgtggc tgagtggtac cttattacag caggttgcag 24480
aagattagcc catcaaggta cacagtgtaa agctttaggg atcacaaaca gctcaagccg 24540
cctttagcaa gtgctccaga acaactctta attacttttt ttttcacatt ctttgctttt 24600
ttagcatgag accgtccatc tttttttcca gtagtgctgg tgctgagaag aaacagccag 24660
acagttgcac agcaaagcat gcaagctgtc ttccttacca aaataaaaca aagctgcaaa 24720
tacaacccca caagccagct gatgtctttt gtaaggcatt ctctgtacag atctggcaag 24780
gtttcactct tatttcacgt gataaaagca catgcctctc tctgatgcca cacaggagct 24840
tcgtagctgt cctcttgcca gacctgcaag gaggatgact gggagcagag tgtgtgaaga 24900
ggactttggt gggtaaagga tccaaaatac aatgctcaga aaacagcagg ctgcaaacat 24960
taggagatcg tagaatcata gcatcgctaa ggttggaaaa gacttctaag atcccccacc 25020
cacccccacc acgtccctca gtgccacatc tccacgttct cggaactcct ctgggacagt 25080
gactttacca cacttcgggc agcctgtacc agtgcatcac cactctttca gaggagaatt 25140
tttccctaat atccaatctg agatgcccag ctggatttcc aggtacctgc ctaccctcag 25200
caaggccatg gttcaagggt ataacacgga gaaagtgcta atctgaagag cccgagcctg 25260
ctgggaagga tgtggcacca gcagtaatct tggtgtgtga gtgcaaagtc cactgcatct 25320
gattccaaag gtttcctgaa tgtccacaag aagctgaatg cctctgctgc ttctctgctg 25380
gcagagtgcc tgctggagag aatcatcctc cccgacctgc agaggtgcag ggtaagagtg 25440
cagagctgct aaaactgttt cctttttttc ctaatcgctt acacagatct tagtggcaaa 25500
gatgtttaca taaccgtacc tcgcacagct cctgccagca gcttcctggc tcggccccat 25560
ttcacagctc gggcaaggat tagggaatga gctgaacagg gtgaagcagg taggaggcac 25620
agccacagct gagcagcatg actacagact ggaagctcag tttcctgtgc tggttggaag 25680
atcagagctg accaaggaca acgacgtttt tattgaggga agaggggctt cacatccatt 25740
tcaacactcc ttaaggcccc aagcattaac ctgagctgct aaggcaggtt aaagacacag 25800
aaggattaca acgcagaact gatggttttg aacagccact gaagtgagtt gaagtgagtg 25860
cacttaaggg agaatcgttg ctagggcaca ggtaggacag ctaaagttcc taaagctcac 25920
ccatgtctgg ccacccatcc tgagcatcga gcaagtgtct gccatcccca gagcatggtg 25980
ggagacaccc gccatgctct ggccagctgg aaatgcttct caggggcaca gcagggtacg 26040
tcagctgggg acaggaaagg ggagagggga agggaagagg aagagaagaa gggaagaaga 26100
agggaagaaa cccaatcagc aagaagtagc taagaagaat gataaaacac agaggcttcc 26160
taaggaccaa aattgtgaca cgcagggaaa agcaaaagca cccaaacaaa gcagtatggt 26220
ggggctcttc ttggtgcagg ggaaagagag gggaatctat tgctctcctg tttgtcactg 26280
gtccttattg atccccagca gaatgtgtgc ctctagaaga gctctgggga gacagaggtc 26340

acaatgtccc agcaaatcct aggatgaggt ggagagacta aaagcatcct aagctctaat 26400
cctctgcctt ccttgcttaa gaaaatatag gcccagccct ggtgtggaag aggacaggag 26460
acactcaggc tgggacaccg ccactgtgat cttctgctgg tcttcaggga taggaacacc 26520
actctgcccc actcacgggg acagcccctt ccatccctca ggaagggctt acaaacccca 26580
tggcaatgaa aaatcacaag gacagccatc ctcctgagcc tgcaggtaca gctttctggg 26640
gccgctccct ctgcgtggca ttacatctca gctttcccta catctgaaaa gaggtcctct 26700
gtcttctaca cctccagctg cagcaagaat aggtgcaaaa agttggctct tttggcccat 26760
gggtgctttc ctccatttgt ctgaggactg atagagctgt gatgggagca gggagagcca 26820
tgtgcaggtc caacaaatcc ctctgaaatt gcaggaagat tggttgggcc agggagagga 26880
gcaacaaatc tccctgaaat ctcaaagcat catcagtcct caggcagaat caatttattt 26940
ctgtttcaca gcaagatgca aaaccattcc ggagcacctg aattgtacag tcctgcctct 27000
cactcccgct tccctttccc tcagctgtcg tggtctcaga ggggagcagg tttcagtggt 27060
gactcaaagt ctgccaaaca gagcacacca gccaggaaga gccctgcagg aagccaccaa 27120
agccacttta agggtcactg catctctgaa gaggcagcat cccctcctca acttgtcaca 27180
gcaacctgct tcctggctaa agacctcaca accctgctca ccttacagca gccagaacat 27240
agcatgggag ctcttcttaa acgttggctg gtggcaaatt gggatgctca cagtgcgacg 27300
gctccctgga gaagatatcc cggggcactg gggcttgggg aattcgggga ttggggcatt 27360
tggggcttgg gatttgagaa gcttggggca tttggggcac tgggtctcgg gaccttgggg 27420
cttgggggca cctggggcag ccaagacaag ggcagtaccc catccttgtg cggagccacc 27480
cgctgtcacc tccagtggtc caggatgcca gcagggaggg cagaaccgcc ccgttccccc 27540
cgccgccccc cgagcatctc tcacctcgga gtagacgtag aggggcagga cgaggagggc 27600
gagcagaagg tcggcgacgg cgaggctgac gatgaaatag ttggtggtgg tcttgagcgc 27660
ccgctccgtg cacacgctga ggcacaccag cccgttgccc ccgacgatga gcaggatgag 27720
gacgatgccc agcaccaggg cggcgatgtt gtggccggct ggaggaggag gaggaggcgg 27780
cggcggagcg gtgccgttgc acggggcggc cccggcgctg ccgttcccca tggcgccggg 27840
gcatggagcc gccgagccgc cgcctccccg cctcagcccc ccgcgggagg gagaagcgaa 27900
ggcgctccgc cggctccgaa ctttgcgagg tgatgggagg agcctccccc gggccgcccc 27960
ggggcagtaa gcggctcacc gggatgcggc gggggggtgg gggtgtggga aggaagaggg 28020
ggagctttga ggtccgttcc agcccagccg tgccgtggtt gtgtgctttc tgtacgttga 28080
acacagccgt gtccccctgt catgagcccg gccgcgctga tgggagttag gaaatagcgg 28140
aggcatcggt cccacgagcg ctgcgctgcg aatacagcgg tggatcaaag cgctgtcgga 28200
ttttagcaaa gccctgacaa agctagagga ggaaaggaaa aaaaaaaaaa agccacacaa 28260

accgacaggt tttcgagtag aaagttgttt caagtcagcc cgacctgaag attttaccca 28320

ggagattcaa agcagtgata ccaaccgtga tgagagaaat cattgctctg atctccaaga 28380

gcatttgaag gactgtgaag actctggaag tccagcaaat agcaaacact gcctcttttt 28440

aaatggagag ggacaagaga tcggggaact aaagagcaat ctgtgttgct ttgagtaaca 28500

gatcttcttt ggtgattttc ctatgaggtg ttgatggggg ggggttaatg ggtgtggggc 28560

actgcgatgt cccgacctgt gccctttgca catcactgca tcaacccttg tgctggcgga 28620

gaaacacgct ctgtctctag cagagccagg gctgaaggct ggggaaggac aggatggctg 28680

tgagccccat ttctagccca ggtgggctgg gagatgtgag acctgatcct gcatcggtgg 28740

gcatccttcc agctgtgctg ccatacctca gcgtgggtgg tggtaagagc cactgaccat 28800

cccatcctga tcagccttct gttgtgggag gcagcaggaa gggaaggcat tgcaagaaag 28860

gatcatgtac accataagga gtggggaaac tgcattactg cctgcttttt ataagacaag 28920

aggcttctca gagctgctaa aaggtccaga tatcacttcc atctaatcca gcaagagact 28980

ggcagaaaga ttcctcactc cagcctttta tgcctctcat ccccaaattc ctttactttt 29040

gtttgaaaga agacacaatc aatccccagc atcatgggaa agccagagcc ctgtttctag 29100

cagcaaggac ttggatctca gttccatcct ccccctgcag ccaacagcac ttaatcccac 29160

tgtctgtttt atccagctgc ctcaggctcc acagctgtgt gtgtgagaag gagaaattta 29220

ccttggctta agtactcagc gggcaatctg ccttgctaaa tattgtctcc agagatgtga 29280

tttccaagct tgtttccgaa atcagcagga gcaccatccc tctgggagct ccctcttacc 29340

cccagcatag cgttgccatc ttcctgttga tcccaatacc tgtgggtgag tctgtcctgc 29400

tccttgcagg tgacccaact tggatgtgga attaaacctg tagggcagaa cacagctctg 29460

tcctgctggc ttcccaccag ctgcagaggg gttccttcag gccttctgct ctgcctggag 29520

cttccacgtt cctctgggaa caattcacct tctgcacagg tgggcacaag gagggctgct 29580

catctgtcag gtcaagaaga gctggtttgg caatatcact tgtagcagag cccttctact 29640

ccaagcaaac agctcaaact agccaaggga ggccttcagg tgcattaatg aaactgtgga 29700

cggag 29705

<210> 2
<211> 546
<212> PRT
<213> Gallus gallus

<400> 2

Met Glu Asp Ala Asp Ser Ala Ala Lys Arg Leu Gly Leu Ala Glu Ala
1               5                   10                  15

Val Ala Ala Ala Val Ala Ala Ala Ala Ala Glu Pro Glu Arg Gln Gln

            20                  25                  30

Pro Gln Gly Ser Glu Gly Lys Ala Glu Arg Arg Glu Glu Val Thr Ala
        35                  40                  45

Val Thr Val Met Ala Ala Asp Ala Glu His Ile Glu Met Gly Ala Glu
    50                  55                  60

Pro Leu Pro Ser Ala Asp Glu Ala Ala Ala Phe Ala Glu Val Thr Thr
65                  70                  75                  80

Val Thr Val Ala Asn Val Gly Ala Ser Ala Asp Asn Val Phe Thr Thr
                85                  90                  95

Ser Val Ala Asn Ala Ala Ser Ile Ser Gly His Val Leu Ser Gly Arg
            100                 105                 110

Thr Ala Leu Gln Ile Gly Asp Ser Leu Asn Thr Glu Lys Ala Thr Leu
        115                 120                 125

Ile Val Val His Thr Asp Gly Ser Ile Val Glu Thr Thr Gly Leu Lys
    130                 135                 140

Gly Pro Ser Ala Pro Leu Thr Pro Gly Pro Gln Ser Pro Pro Thr Pro
145                 150                 155                 160

Leu Ala Thr Val Gln Glu Lys Thr Gly Thr Lys Tyr Asn Trp Asp Pro
                165                 170                 175

Ser Val Tyr Asp Asn Glu Leu Pro Val Arg Cys Arg Asn Ile Ser Gly
            180                 185                 190

Ile Leu Tyr Lys Asn Arg Leu Gly Ser Gly Gly Arg Gly Arg Cys Ile
        195                 200                 205

Lys Gln Gly Asp Asn Trp Tyr Ser Pro Thr Glu Phe Glu Ala Met Ala
    210                 215                 220

Gly Arg Ala Ser Ser Lys Asp Trp Lys Arg Ser Ile Arg Tyr Ala Gly
225                 230                 235                 240

Arg Pro Leu Gln Cys Leu Ile His Asp Gly Ile Leu Asn Pro His Ala
                245                 250                 255

Ala Ser Cys Thr Cys Ala Ala Cys Cys Asp Asp Met Thr Leu Ser Gly
            260                 265                 270

Pro Ile Arg Leu Phe Val Pro Tyr Lys Arg Arg Lys Lys Glu Asn Glu

275                 280                 285

Met Ser Ala Asn Pro Val Lys Lys Glu Ser Ser Lys Asn Ile Thr Leu
    290                 295                 300

Leu Pro Ala Thr Ala Ala Thr Thr Phe Thr Val Thr Pro Ser Gly Gln
305                 310                 315                 320

Ile Thr Thr Ser Gly Ala Leu Thr Phe Asp Arg Thr Ser Thr Val Glu
                325                 330                 335

Ala Thr Ala Ile Ile Ser Glu Ser Pro Ala Gln Gly Asp Val Phe Thr
            340                 345                 350

Gly Ala Thr Val Gln Asp Thr Asn Val Gln Gln Pro Cys Arg Val Ala
        355                 360                 365

His Pro Glu Pro His Tyr Pro Thr Tyr Gln Asp Asn Cys Gln Ile Ser
    370                 375                 380

Pro Phe Pro Glu Ala Ala Leu Pro Thr Ser His Pro Lys Ile Val Leu
385                 390                 395                 400

Thr Pro Ile Pro Ala Leu Ser Val Pro Pro Gln Thr Pro Thr Lys Ala
                405                 410                 415

Ile Ser Pro Thr Val Val Asn Gly Leu Glu Val Thr Glu Gln Arg Ser
            420                 425                 430

Trp Leu Tyr Leu Glu Glu Met Val Asn Ser Leu Leu Asn Thr Ala Gln
        435                 440                 445

Gln Leu Lys Ser Leu Ile Glu Gln Ala Lys Gln Ala Ser Ser Ser Phe
    450                 455                 460

Arg Glu Ala Ala Val Thr Gln Ala Lys Ile Gln Ala Asp Val Glu Arg
465                 470                 475                 480

Lys Glu Gln Ser Cys Val Asn Cys Gly Arg Glu Ala Met Asn Glu Cys
                485                 490                 495

Thr Gly Cys His Lys Val Asn Tyr Cys Ser Thr Phe Cys Gln Arg Lys
            500                 505                 510

Asp Trp Lys Glu His Gln His Ile Cys Gly Glu Ala Asn Ala Val Thr
        515                 520                 525

Val Gln Ala Asp Asp Val His Val Ala Asp Asn Val Met Glu Lys Val

530                 535                 540

Ser Val
545

<210>  3
<211>  378
<212>  PRT
<213>  Gallus gallus

<400>  3

Met Gly Asn Gly Ser Ala Gly Ala Ala Pro Cys Asn Gly Thr Ala Pro
1               5                   10                  15

Pro Pro Pro Pro Pro Pro Pro Pro Ala Gly His Asn Ile Ala Ala Leu
            20                  25                  30

Val Leu Gly Ile Val Leu Ile Leu Leu Ile Val Gly Gly Asn Gly Leu
        35                  40                  45

Val Cys Leu Ser Val Cys Thr Glu Arg Ala Leu Lys Thr Thr Thr Asn
    50                  55                  60

Tyr Phe Ile Val Ser Leu Ala Val Ala Asp Leu Leu Leu Ala Leu Leu
65                  70                  75                  80

Val Leu Pro Leu Tyr Val Tyr Ser Glu Phe Gln Gly Gly Val Trp Ser
                85                  90                  95

Leu Ser Thr Val Leu Cys Asp Ala Leu Met Thr Met Asp Val Met Leu
            100                 105                 110

Cys Thr Ala Ser Ile Phe Asn Leu Cys Ala Ile Ser Val Asp Arg Phe
        115                 120                 125

Ile Ala Val Ser Val Pro Leu Asn Tyr Asn Arg Arg Gln Ile Asp Leu
    130                 135                 140

Arg Gln Leu Ile Leu Ile Ser Thr Thr Trp Ile Phe Ala Phe Ala Val
145                 150                 155                 160

Ala Ser Pro Val Ile Phe Gly Leu Asn Asn Val Pro Asn Arg Asp Pro
                165                 170                 175

Ser Leu Cys Gln Leu Glu Asp Asp Asn Tyr Ile Val Tyr Ser Ser Ile
            180                 185                 190

Cys Ser Phe Phe Ile Pro Cys Pro Val Met Leu Val Leu Tyr Cys Ala
        195                 200                 205

Met Phe Gln Gly Leu Lys Arg Trp Glu Glu Ala Arg Lys Ala Lys Leu
    210                 215                 220

Arg Gly Gly Ile Tyr Gly Gly Asn Arg Met Leu Tyr His Pro Ser Pro
225                 230                 235                 240

Phe Ile Glu Arg Glu Arg Val Gly Met Glu Pro Glu Glu Tyr His Pro
                245                 250                 255

Tyr Ala His Pro Glu His Pro Leu Ser Gly Asp Tyr Val Met Ser Asn
            260                 265                 270

Gly Leu Gln Thr Val Ser Tyr Pro His Leu Lys Tyr Pro His Pro Ala
        275                 280                 285

His Gly Gln Lys Arg Ala Lys Ile Asn Gly Arg Glu Arg Lys Ala Met
    290                 295                 300

Arg Val Leu Pro Val Val Val Gly Ala Phe Leu Phe Cys Trp Thr Pro
305                 310                 315                 320

Phe Phe Val Val His Ile Thr Arg Ala Leu Cys Lys Ser Cys Thr Ile
                325                 330                 335

Pro Thr Gln Val Thr Ser Ile Val Thr Trp Leu Gly Tyr Val Asn Ser
            340                 345                 350

Ala Val Asn Pro Ile Ile Tyr Thr Val Phe Asn Ala Glu Phe Arg Asn
        355                 360                 365

Phe Phe Arg Lys Val Leu His Leu Phe Cys
    370                 375

<210>  4
<211>  20
<212>  DNA
<213>  Artificial sequence

<220>
<221>  source
<223>  /note="Description of artificial sequence: DRD4 primer"

<400>  4
cccttggcta gtttgagctg                                                  20

<210>  5
<211>  20
<212>  DNA
<213>  Artificial sequence

<220>
<221>  source
<223>  /note="Description of artificial sequence: DRD4 primer"

<400>  5
cagcaagaga ctggcagaaa                                                    20


<210>  6
<211>  20
<212>  DNA
<213>  Artificial sequence

<220>
<221>  source
<223>  /note="Description of artificial sequence: DRD4 primer"

<400>  6
ggcataaaag gctggagtga                                                    20


<210>  7
<211>  21
<212>  DNA
<213>  Artificial sequence

<220>
<221>  source
<223>  /note="Description of artificial sequence: DRD4 primer"

<400>  7
tgctctgatc tccaagagca t                                                  21


<210>  8
<211>  22
<212>  DNA
<213>  Artificial sequence

<220>
<221>  source
<223>  /note="Description of artificial sequence: DRD4 primer"

<400>  8
cgctatttcc taactcccat ca                                                 22


<210>  9
<211>  18
<212>  DNA
<213>  Artificial sequence

<220>
<221>  source
<223>  /note="Description of artificial sequence: DRD4 primer"

<400>  9
gggagggaga agcgaagg                                                      18


<210>  10
<211>  20
<212>  DNA
<213>  Artificial sequence

<220>
<221>  source
<223>  /note="Description of artificial sequence: DRD4 primer"

<400>  10
cgatctcttg tccctctcca                                                    20


<210>  11
<211>  20
<212>  DNA
<213>  Artificial sequence

<220>
<221>  source
<223>  /note="Description of artificial sequence: DRD4 primer"

<400>  11
agttggtggt ggtcttgagc                                                    20


<210>  12
<211>  18
<212>  DNA
<213>  Artificial sequence

<220>
<221>  source
<223>  /note="Description of artificial sequence: DRD4 primer"

<400>  12
cccgtgcaac ggcaccgc                                                      18


<210>  13
<211>  19
<212>  DNA
<213>  Artificial sequence

<220>
<221>  source
<223>  /note="Description of artificial sequence: DRD4 primer"

<400>  13
agaggggcag gacgaggag                                                     19


<210>  14
<211>  19
<212>  DNA
<213>  Artificial sequence

<220>
<221>  source
<223>  /note="Description of artificial sequence: DRD4 primer"

<400>  14
gaccactgga ggtgacagc                                                     19


<210>  15
<211>  20
<212>  DNA

<213>  Artificial sequence

<220>
<221>  source
<223>  /note="Description of artificial sequence: DRD4 primer"

<400>  15
attgcaggaa gattggttgg                                                    20


<210>  16
<211>  20
<212>  DNA
<213>  Artificial sequence

<220>
<221>  source
<223>  /note="Description of artificial sequence: DRD4 primer"

<400>  16
ttgattctgc ctgaggactg                                                    20


<210>  17
<211>  20
<212>  DNA
<213>  Artificial sequence

<220>
<221>  source
<223>  /note="Description of artificial sequence: DRD4 primer"

<400>  17
gtccttattg atccccagca                                                    20


<210>  18
<211>  20
<212>  DNA
<213>  Artificial sequence

<220>
<221>  source
<223>  /note="Description of artificial sequence: DRD4 primer"

<400>  18
tcctaggatt tgctgggaca                                                    20


<210>  19
<211>  20
<212>  DNA
<213>  Artificial sequence

<220>
<221>  source
<223>  /note="Description of artificial sequence: DRD4 primer"

<400>  19
ccttcccggt tcaaatactg                                                    20


<210>  20
<211>  20

<212>  DNA
<213>  Artificial sequence

<220>
<221>  source
<223>  /note="Description of artificial sequence: DRD4 primer"

<400>  20
cttcagtggc tgtttgaagc                                                    20


<210>  21
<211>  20
<212>  DNA
<213>  Artificial sequence

<220>
<221>  source
<223>  /note="Description of artificial sequence: DRD4 primer"

<400>  21
cctctctctg atgccacaca                                                    20


<210>  22
<211>  20
<212>  DNA
<213>  Artificial sequence

<220>
<221>  source
<223>  /note="Description of artificial sequence: DRD4 primer"

<400>  22
ctctgctccc agtcatcctc                                                    20


<210>  23
<211>  20
<212>  DNA
<213>  Artificial sequence

<220>
<221>  source
<223>  /note="Description of artificial sequence: DRD4 primer"

<400>  23
cttggctgtg gtgttcaaaa                                                    20


<210>  24
<211>  19
<212>  DNA
<213>  Artificial sequence

<220>
<221>. source
<223>  /note="Description of artificial sequence: DRD4 primer"

<400>  24
aactttcagg gtgcccaac                                                     19


<210>  25

<211>  21
<212>  DNA
<213>  Artificial sequence

<220>
<221>  source
<223>  /note="Description of artificial sequence: DRD4 primer"

<400>  25
tgaaccatcc tttgagacag c                                                21


<210>  26
<211>  19
<212>  DNA
<213>  Artificial sequence

<220>
<221>  source
<223>  /note="Description of artificial sequence: DRD4 primer"

<400>  26
gcttcaagct ggggaagag                                                   19


<210>  27
<211>  20
<212>  DNA
<213>  Artificial sequence

<220>
<221>  source
<223>  /note="Description of artificial sequence: DRD4 primer"

<400>  27
tgggactgaa acagcgataa                                                  20


<210>  28
<211>  20
<212>  DNA
<213>  Artificial sequence

<220>
<221>  source
<223>  /note="Description of artificial sequence: DRD4 primer"

<400>  28
cccaggggca ttttatacac                                                  20


<210>  29
<211>  20
<212>  DNA
<213>  Artificial sequence

<220>
<221>  source
<223>  /note="Description of artificial sequence: DRD4 primer"

<400>  29
aacacaggtg ggtcagtgct                                                  20

<210> 30
<211> 20
<212> DNA
<213> Artificial sequence

<220>
<221> source
<223> /note="Description of artificial sequence: DRD4 primer"

<400> 30
agcatcaagc tgcttccatt 20


<210> 31
<211> 20
<212> DNA
<213> Artificial sequence

<220>
<221> source
<223> /note="Description of artificial sequence: DRD4 primer"

<400> 31
ggagcgatgc tactctggat 20


<210> 32
<211> 19
<212> DNA
<213> Artificial sequence

<220>
<221> source
<223> /note="Description of artificial sequence: DRD4 primer"

<400> 32
ggtggttgtt ggtgagtgg 19


<210> 33
<211> 20
<212> DNA
<213> Artificial sequence

<220>
<221> source
<223> /note="Description of artificial sequence: DRD4 primer"

<400> 33
cgttgtgttc aaaagggttg 20


<210> 34
<211> 20
<212> DNA
<213> Artificial sequence

<220>
<221> source
<223> /note="Description of artificial sequence: DRD4 primer"

<400> 34
tttttgtcac ggagagatgg 20

<210>  35
<211>  19
<212>  DNA
<213>  Artificial sequence

<220>
<221>  source
<223>  /note="Description of artificial sequence: DRD4 primer"

<400>  35
gagacagagg gctgggaac                                                    19


<210>  36
<211>  21
<212>  DNA
<213>  Artificial sequence

<220>
<221>  source
<223>  /note="Description of artificial sequence: DRD4 primer"

<400>  36
gagatggtag ctctgcattc g                                                 21


<210>  37
<211>  20
<212>  DNA
<213>  Artificial sequence

<220>
<221>  source
<223>  /note="Description of artificial sequence: DRD4 primer"

<400>  37
atctcaagac ctcccagcaa                                                   20


<210>  38
<211>  23
<212>  DNA
<213>  Artificial sequence

<220>
<221>  source
<223>  /note="Description of artificial sequence: DEAF1 primer"

<400>  38
ccagctgttt tccttagtct tca                                               23


<210>  39
<211>  22
<212>  DNA
<213>  Artificial sequence

<220>
<221>  source
<223>  /note="Description of artificial sequence: DEAF1 primer"

<400>  39
cctcttccct gttaatctgc ac                                                22

<210>  40
<211>  20
<212>  DNA
<213>  Artificial sequence

<220>
<221>  source
<223>  /note="Description of artificial sequence: DEAF1 primer"

<400>  40
ttgtgtgtgt ggtggtgatg                                                    20


<210>  41
<211>  18
<212>  DNA
<213>  Artificial sequence

<220>
<221>  source
<223>  /note="Description of artificial sequence: DEAF1 primer"

<400>  41
gccgtgagga gaggtgag                                                      18


<210>  42
<211>  20
<212>  DNA
<213>  Artificial sequence

<220>
<221>  source
<223>  /note="Description of artificial sequence: DEAF1 primer"

<400>  42
tacgctgtga aatgcctctg                                                    20


<210>  43
<211>  20
<212>  DNA
<213>  Artificial sequence

<220>
<221>  source
<223>  /note="Description of artificial sequence: DEAF1 primer"

<400>  43
tacacctgac ccagtgacca                                                    20


<210>  44
<211>  20
<212>  DNA
<213>  Artificial sequence

<220>
<221>  source
<223>  /note="Description of artificial sequence: DEAF1 primer"

<400>  44

gaccccaagc ctaacagaga                                                    20

<210>  45
<211>  20
<212>  DNA
<213>  Artificial sequence

<220>
<221>  source
<223>  /note="Description of artificial sequence: DEAF1 primer"

<400>  45
tctcctagtt ccgggctttt                                                    20

<210>  46
<211>  22
<212>  DNA
<213>  Artificial sequence

<220>
<221>  source
<223>  /note="Description of artificial sequence: DEAF1 primer"

<400>  46
ttcagaatgc taccttgtgt ca                                                 22

<210>  47
<211>  20
<212>  DNA
<213>  Artificial sequence

<220>
<221>  source
<223>  /note="Description of artificial sequence: DEAF1 primer"

<400>  47
ggaagactca ctgcacagca                                                    20

<210>  48
<211>  20
<212>  DNA
<213>  Artificial sequence

<220>
<221>  source
<223>  /note="Description of artificial sequence: DEAF1 primer"

<400>  48
ggacggtttc ctagtcagca                                                    20

<210>  49
<211>  20
<212>  DNA
<213>  Artificial sequence

<220>
<221>  source
<223>  /note="Description of artificial sequence: DEAF1 primer"

<400>  49
tccttgtggg tcccttctaa                                                   20


<210>  50
<211>  20
<212>  DNA
<213>  Artificial sequence

<220>
<221>  source
<223>  /note="Description of artificial sequence: DEAF1 primer"

<400>  50
aggagcagaa tctggcacac                                                   20


<210>  51
<211>  20
<212>  DNA
<213>  Artificial sequence

<220>
<221>  source
<223>  /note="Description of artificial sequence: DEAF1 primer"

<400>  51
cccacaactc accagagtca                                                   20


<210>  52
<211>  21
<212>  DNA
<213>  Artificial sequence

<220>
<221>  source
<223>  /note="Description of artificial sequence: DEAF1 primer"

<400>  52
gctgcatcta gagaggttgg a                                                 21


<210>  53
<211>  22
<212>  DNA
<213>  Artificial sequence

<220>
<221>  source
<223>  /note="Description of artificial sequence: DEAF1 primer"

<400>  53
aaaaacacga agtgacaaag ca                                                22


<210>  54
<211>  20
<212>  DNA
<213>  Artificial sequence

<220>
<221>  source
<223>  /note="Description of artificial sequence: DEAF1 primer"

<400>  54
agggctttga ttcccatttc                                                    20


<210>  55
<211>  20
<212>  DNA
<213>  Artificial sequence

<220>
<221>  source
<223>  /note="Description of artificial sequence: DEAF1 primer"

<400>  55
taagcatctg cagcatctgg                                                    20


<210>  56
<211>  20
<212>  DNA
<213>  Artificial sequence

<220>
<221>  source
<223>  /note="Description of artificial sequence: DEAF1 primer"

<400>  56
tgcagaagcc ttgagatgtg                                                    20


<210>  57
<211>  20
<212>  DNA
<213>  Artificial sequence

<220>
<221>  source
<223>  /note="Description of artificial sequence: DEAF1 primer"

<400>  57
caagagcttc ttctgccaag                                                    20


<210>  58
<211>  20
<212>  DNA
<213>  Artificial sequence

<220>
<221>  source
<223>  /note="Description of artificial sequence: DEAF1 primer"

<400>  58
tgtgggtttc gcagttaaga                                                    20


<210>  59
<211>  20
<212>  DNA
<213>  Artificial sequence

<220>
<221>  source

<223>  /note="Description of artificial sequence: DEAF1 primer"

<400>  59
tgcttcacgg gtgaaaaata                                                    20


<210>  60
<211>  20
<212>  DNA
<213>  Artificial sequence

<220>
<221>  source
<223>  /note="Description of artificial sequence: DEAF1 primer"

<400>  60
ttcacccgtg aagcaaagat                                                    20


<210>  61
<211>  20
<212>  DNA
<213>  Artificial sequence

<220>
<221>  source
<223>  /note="Description of artificial sequence: DEAF1 primer"

<400>  61
ctggaccagc ctgcaaatta                                                    20


<210>  62
<211>  20
<212>  DNA
<213>  Artificial sequence

<220>
<221>  source
<223>  /note="Description of artificial sequence: DEAF1 primer"

<400>  62
tgtcccatgt cagttttcca                                                    20


<210>  63
<211>  21
<212>  DNA
<213>  Artificial sequence

<220>
<221>  source
<223>  /note="Description of artificial sequence: DEAF1 primer"

<400>  63
tgctgaaggt tcatcacaca g                                                  21


<210>  64
<211>  20
<212>  DNA
<213>  Artificial sequence

<220>

<221>  source
<223>  /note="Description of artificial sequence: DEAF1 primer"

<400>  64
gcaaatagtt ctccgccaat                                                    20


<210>  65
<211>  20
<212>  DNA
<213>  Artificial sequence

<220>
<221>  source
<223>  /note="Description of artificial sequence: DEAF1 primer"

<400>  65
ccaccagttc agatgctcct                                                    20


<210>  66
<211>  20
<212>  DNA
<213>  Artificial sequence

<220>
<221>  source
<223>  /note="Description of artificial sequence: DEAF1 primer"

<400>  66
ggagcatctg aactggtggt                                                    20


<210>  67
<211>  19
<212>  DNA
<213>  Artificial sequence

<220>
<221>  source
<223>  /note="Description of artificial sequence: DEAF1 primer"

<400>  67
acccacagct gctggaatc                                                     19


<210>  68
<211>  21
<212>  DNA
<213>  Artificial sequence

<220>
<221>  source
<223>  /note="Description of artificial sequence: DEAF1 primer"

<400>  68
ttccactggg tatagctttg g                                                  21


<210>  69
<211>  20
<212>  DNA
<213>  Artificial sequence

<220>
<221>  source
<223>  /note="Description of artificial sequence: DEAF1 primer"

<400>  69
catggaaagg gagttcctca                                                    20


<210>  70
<211>  23
<212>  DNA
<213>  Artificial sequence

<220>
<221>  source
<223>  /note="Description of artificial sequence: DEAF1 primer"

<400>  70
cctcttaaca cctaactcca tgc                                                23


<210>  71
<211>  23
<212>  DNA
<213>  Artificial sequence

<220>
<221>  source
<223>  /note="Description of artificial sequence: DEAF1 primer"

<400>  71
gatcaatacc gaggagtgat ttt                                                23


<210>  72
<211>  20
<212>  DNA
<213>  Artificial sequence

<220>
<221>  source
<223>  /note="Description of artificial sequence: DEAF1 primer"

<400>  72
cagagtccat ccctctcacc                                                    20


<210>  73
<211>  22
<212>  DNA
<213>  Artificial sequence

<220>
<221>  source
<223>  /note="Description of artificial sequence: DEAF1 primer"

<400>  73
tggctctgca gctcagatat aa                                                 22


<210>  74
<211>  20
<212>  DNA
<213>  Artificial sequence

<220>
<221>  source
<223>  /note="Description of artificial sequence: DEAF1 primer"

<400>  74
cagttgtgcc agtgtgttcc                                                    20


<210>  75
<211>  20
<212>  DNA
<213>  Artificial sequence

<220>
<221>  source
<223>  /note="Description of artificial sequence: DEAF1 primer"

<400>  75
agcaaatcct cttcctgtca                                                    20


<210>  76
<211>  21
<212>  DNA
<213>  Artificial sequence

<220>
<221>  source
<223>  /note="Description of artificial sequence: DEAF1 primer"

<400>  76
cactctttca tcctgcttcc a                                                  21


<210>  77
<211>  20
<212>  DNA
<213>  Artificial sequence

<220>
<221>  source
<223>  /note="Description of artificial sequence: DEAF1 primer"

<400>  77
ggcaaacagc atatgcaaca                                                    20


<210>  78
<211>  20
<212>  DNA
<213>  Artificial sequence

<220>
<221>  source
<223>  /note="Description of artificial sequence: DEAF1 primer"

<400>  78
gctgttcagg agcctaagga                                                    20


<210>  79
<211>  20
<212>  DNA

<213>  Artificial sequence

<220>
<221>  source
<223>  /note="Description of artificial sequence: DEAF1 primer"

<400>  79
ttcatgtgtt ctccccttc                                                    20


<210>  80
<211>  20
<212>  DNA
<213>  Artificial sequence

<220>
<221>  source
<223>  /note="Description of artificial sequence: DEAF1 primer"

<400>  80
tagtaggatt ggccccaaag                                                   20


<210>  81
<211>  20
<212>  DNA
<213>  Artificial sequence

<220>
<221>  source
<223>  /note="Description of artificial sequence: DEAF1 primer"

<400>  81
ggtccccaaa tcctgctaat                                                   20


<210>  82
<211>  22
<212>  DNA
<213>  Artificial sequence

<220>
<221>  source
<223>  /note="Description of artificial sequence: DEAF1 primer"

<400>  82
ggctcagaga taaaagggag gt                                                22


<210>  83
<211>  23
<212>  DNA
<213>  Artificial sequence

<220>
<221>  source
<223>  /note="Description of artificial sequence: DEAF1 primer"

<400>  83
cctgatctaa actccatgag gaa                                               23


<210>  84
<211>  20

<212>  DNA
<213>  Artificial sequence

<220>
<221>  source
<223>  /note="Description of artificial sequence: DEAF1 primer"

<400>  84
ctcctcacct gttgggtcat                                                    20


<210>  85
<211>  22
<212>  DNA
<213>  Artificial sequence

<220>
<221>  source
<223>  /note="Description of artificial sequence: DEAF1 primer"

<400>  85
cctcccttac agaagaggga ct                                                 22


<210>  86
<211>  22
<212>  DNA
<213>  Artificial sequence

<220>
<221>  source
<223>  /note="Description of artificial sequence: DEAF1 primer"

<400>  86
tgaagccaca tgtcttgaga tt                                                 22


<210>  87
<211>  20
<212>  DNA
<213>  Artificial sequence

<220>
<221>  source
<223>  /note="Description of artificial sequence: DEAF1 primer"

<400>  87
atcacaccac tggctcacag                                                    20


<210>  88
<211>  20
<212>  DNA
<213>  Artificial sequence

<220>
<221>  source
<223>  /note="Description of artificial sequence: DEAF1 primer"

<400>  88
tcaagcccca ttttgatagc                                                    20


<210>  89

<211>  20
<212>  DNA
<213>  Artificial sequence

<220>
<221>  source
<223>  /note="Description of artificial sequence: DEAF1 primer"

<400>  89
aacaacagtg gaggggtgac                                                    20


<210>  90
<211>  22
<212>  DNA
<213>  Artificial sequence

<220>
<221>  source
<223>  /note="Description of artificial sequence: DEAF1 primer"

<400>  90
caggtgtgat tttcaatctc ca                                                 22


<210>  91
<211>  20
<212>  DNA
<213>  Artificial sequence

<220>
<221>  source
<223>  /note="Description of artificial sequence: DEAF1 primer"

<400>  91
cagctcacgc caatgtttac                                                    20


<210>  92
<211>  20
<212>  DNA
<213>  Artificial sequence

<220>
<221>  source
<223>  /note="Description of artificial sequence: DEAF1 primer"

<400>  92
gtaaggagtg gccccaagat                                                    20


<210>  93
<211>  20
<212>  DNA
<213>  Artificial sequence

<220>
<221>  source
<223>  /note="Description of artificial sequence: DEAF1 primer"

<400>  93
ttgctctccc tctcatccat                                                    20

<210>  94
<211>  20
<212>  DNA
<213>  Artificial sequence

<220>
<221>  source
<223>  /note="Description of artificial sequence: DEAF1 primer"

<400>  94
ctgagtggtc atgtgctggt                                                    20


<210>  95
<211>  19
<212>  DNA
<213>  Artificial sequence

<220>
<221>  source
<223>  /note="Description of artificial sequence: DEAF1 primer"

<400>  95
ctggaggagg ctcccagac                                                     19


<210>  96
<211>  20
<212>  DNA
<213>  Artificial sequence

<220>
<221>  source
<223>  /note="Description of artificial sequence: DEAF1 primer"

<400>  96
tcctactgca aacctctgga                                                    20


<210>  97
<211>  18
<212>  DNA
<213>  Artificial sequence

<220>
<221>  source
<223>  /note="Description of artificial sequence: DEAF1 primer"

<400>  97
atgctgccgt ggtgagag                                                      18


<210>  98
<211>  20
<212>  DNA
<213>  Artificial sequence

<220>
<221>  source
<223>  /note="Description of artificial sequence: DEAF1 primer"

<400>  98
tgtgtctgca gggagttttg                                                    20

<210> 99
<211> 20
<212> DNA
<213> Artificial sequence

<220>
<221> source
<223> /note="Description of artificial sequence: DEAF1 primer"

<400> 99
ggcaatagat ggaggcaaga 20


<210> 100
<211> 20
<212> DNA
<213> Artificial sequence

<220>
<221> source
<223> /note="Description of artificial sequence: DEAF1 primer"

<400> 100
cacgatgagt gccagttttc 20


<210> 101
<211> 20
<212> DNA
<213> Artificial sequence

<220>
<221> source
<223> /note="Description of artificial sequence: DEAF1 primer"

<400> 101
tgctgggagg tcttgagatt 20

## Description

The present invention relates to a nucleic acid molecule contributing to or indicative of low feather pecking. The present invention also relates to a nucleic acid molecule deviating by at least one mutation from the nucleic acid molecule of the invention, wherein said mutation is contributing to or indicative of high feather pecking and is a substitution, an insertion or a deletion. The present invention further relates to methods of testing an avian for its predisposition for low or high feather pecking comprising analysing in a sample the nucleic acid molecules of the invention for nucleotide polymorphisms which are linked to said predisposition. The present invention also relates to a method for selecting an avian or part of an avian for low or high feather pecking and to a kit for the detection of avians having a predisposition for low or high feather pecking comprising a nucleic acid molecule of the invention or a fragment thereof, in one or more container.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Feather pecking is a behavioural disorder that is characterized as pecking at and pulling out of feathers of fellow birds at varying intensities. It occurs in a range of bird species, such as poultry, where feather pecking has most serious welfare and economic implications, in particular in laying hens kept in large group housing systems. It can cause extensive damage to the plumage resulting in loss of heat, higher feed consumption and consequently an impaired feed efficiency (Tauson & Svensson 1980). Feather pecking may lead to cannibalism and concomitantly to high mortality rates (Kjaer & Sorensen 2002). This behavioural disorder is hypothesised to result from redirection of exploratory pecking, a component of normal foraging behaviour, to pecking at the plumage of conspecifics (Blokhuis & Arkes 1984). The extent of the redirection depends on the genetic predisposition of the chicken, the environmental circumstances and the stress-related physiological status of the animal (Jensen *et al*. 2008).
Genes encoding the components of the serotonergic and dopaminergic neurotransmitter systems have been described as candidates for temperament traits including exploration behaviour and novelty seeking (Cloninger 1986). *DRD4,* encoding the dopamine receptor D4, has been shown to be associated with temperament behaviour in man (Reif & Lesch 2003; Ebstein 2006) as well as other mammalian species (Momozawa *et al*. 2005; Bailey *et al*. 2007; Hejjas *et al*. 2007). Recently, Fidler *et al*. (2007) reported significant association of *DRD4* polymorphisms with early exploratory behaviour in a passerine bird species *(Parus major*). A possible role of the serotonergic and dopaminergic systems in the etiology of feather pecking has also been suggested by pharmacological studies (van Hierden *et al*. 2002; Kjaer *et al*. 2004; van Hierden *et al*. 2005; Dennis *et al*. 2008).

However, the factors underlying the etiology of feather pecking have not been identified so far and no reliable method exists at present for testing avian animals for their predisposition for feather pecking as no genetic marker correlating with this behaviour is currently available.

Thus, the technical problem underlying the present invention was to provide means and methods useful for testing an avian for its predisposition for low or high feather pecking. The solution to this technical problem is achieved by providing the embodiments characterised in the claims.

Accordingly, the present invention relates to a nucleic acid molecule contributing to or indicative of low feather pecking wherein said nucleic acid molecule is selected from the group consisting of (a) a nucleic acid molecule having or comprising the nucleic acid sequence of SEQ ID NO: 1; and (b) a nucleic acid molecule of at least 12 contiguous nucleotides the complementary strand of which hybridizes under stringent conditions to the nucleic acid molecule of (a), wherein said nucleic acid molecule has (i) an adenosine at a position corresponding to position 10747, 11041, 11244, 11617, 12148, 12159, 12220, 12381, 12564, 14392, 14462, 14554, 14562, 14613, 14667, 14679, 14832, 15672, 16537, 16863, 16983, 17287, 17329, 17715, 18351, 18772, 21265, 21307, 21623, 22754, 22868, 23341, 23416, 23737, 23847, 23909, 24155 and/or 24574 of SEQ ID NO:1; (ii) a cytosine at a position corresponding to position 10252, 11238, 11271, 12184, 12327, 12338, 13286, 15154, 16911, 17369, 17435, 24051, 24213, 24247 and/or 25085 of SEQ ID NO:1; (iii) a guanosine at a position corresponding to position 10324, 11255, 11723, 12295, 13265, 13328, 14260, 14574, 14607, 14652, 14669, 14717, 14747, 14753, 14925, 15537, 15674, 16162, 17607, 18639, 18991, 19357, 22298, 23851, 23914, 25033 and/or 25062 of SEQ ID NO:1; and/or (iv) a thymidine at a position corresponding to position 10318, 11315, 11848, 12270, 12979, 14116, 14210, 14629, 14637, 14809, 17371, 17744, 18129, 18259, 21409, 23812, 24891, 25004, 25067 and/or 23850 of SEQ ID NO:1.

Nucleic acid molecules, in accordance with the present invention, include DNA, such as cDNA or genomic DNA, RNA (e.g. mRNA), also in synthetic or semisynthetic form, further synthetic or semisynthetic derivatives of DNA or RNA and mixed polymers, both sense and antisense strands. They may contain additional non-natural or derivatized nucleotide bases, as will be readily appreciated by those skilled in the art. Such nucleic acid mimicking molecules or nucleic acid derivatives according to the invention include peptide nucleic acid (PNA), phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art. For the purposes of the present invention, a peptide nucleic acid (PNA) is a polyamide type of DNA analog and the monomeric units for the derivatives of adenine, guanine, thymine and cytosine are available commercially (Perceptive Biosystems). Certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding, such as for example 12-mer, 13-mer, 14-mer, 15-mer or 16-mer probes. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

The term "contributing to or indicative of low feather pecking" refers to the fact that the nucleic acid molecule of the invention is indicative of low feather pecking behaviour and possibly also causative therefore. This nucleic acid molecule of the invention associated with low feather pecking is also referred to herein as the "wild-typ" nucleic acid molecule and is preferably represented by SEQ ID NO:1. Feather pecking is defined as pecking at and pulling out of feathers of fellow birds at varying intensities. Low feather pecking, in accordance with the present invention, encompasses gentle pecking and pulling as well as no pecking.

The term "nucleic acid molecules of the invention having [...] the nucleic acid sequence of SEQ ID NO:1" refers to nucleic acid molecules having, i.e. consisting of, the sequence of SEQ ID NO:1. The term "nucleic acid molecule [...] comprising the nucleic acid sequence of SEQ ID NO:1" as used throughout this specification refers to nucleic acid molecules that are at least 1 nucleotide longer than the nucleic acid molecule specified by the SEQ ID NO:1. At the same time, these nucleic acid molecules extend, at a maximum, 30000 nucleotides over the 5'and/or 3'end of the nucleic acid molecule of the invention specified e. g. by the SEQ ID NO:1. Such nucleic acid molecules include, but are not limited to nucleic acid molecule comprising the sequence of SEQ ID NO:1 and additional coding sequences, such as leader or secretory sequence as well as nucleic acid molecules comprising the sequence of SEQ ID NO:1 and additional non-coding sequences, e.g. 5' and 3' untranslated sequences (UTR) that are transcribed but not translated. Such additional sequences may play a role in transcription, mRNA processing (including splicing and polyadenylation signals), ribosome binding and stability of mRNA. Furthermore, the nucleic acid molecule may comprise the sequence of SEQ ID NO:1 fused to marker sequences encoding, for example, a peptide that facilitates purification of the encoded polypeptides, such as a V5- or poly-His-tag.

The term "hybridizes/hybridizing" as used herein refers to a pairing of a nucleic acid molecule to a (partially) complementary strand of this nucleic acid molecule which thereby form a hybrid.
It is well known in the art how to perform hybridization experiments with nucleic acid molecules. Correspondingly, the person skilled in the art knows what hybridization conditions she/he has to use to allow for a successful hybridization in accordance with item (i)(c), above. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).
"Stringent conditions" refers to hybridization conditions under which the nucleic acid molecules that are capable of hybridizing to the nucleic acid molecules of the invention or parts thereof do not cross hybridize to unrelated nucleic acid molecules. Appropriate stringent hybridization conditions for each nucleic acid molecule may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), Ioc. cit. , see in particular the chapter "Hybridization Strategy" by Britten & Davidson, 3 to 15. Such conditions comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Said conditions for hybridization are also known by a person skilled in the art as "highly stringent conditions for hybridization". It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado.
A hybridization complex may be formed in solution (e.g., Cot or Rot analysis) or between one nucleic acid molecule present in solution and another nucleic acid molecule immobilized on a solid support (e.g., membranes, filters, chips, pins or glass slides to which, e.g., cells have been fixed).
Thus, in accordance with item (b) above, nucleic acid molecules related but not identical in sequence with the nucleic acid molecule of item (a) are also encompassed by the invention. In addition, the invention comprises according to item (b) fragments of the nucleic acid molecule of (a). For all embodiments falling under item (b), it is essential in accordance with this embodiment, that the nucleic acid molecule contributes to or is indicative of low feather pecking.

The fragment of the invention may be of natural as well as of (semi) synthetic origin. Thus, the fragment may, for example, be a nucleic acid molecule that has been synthesized according to conventional protocols of organic chemistry. Importantly, the nucleic acid fragment of the invention comprises at least one nucleotide corresponding to the positions outlined above. The fragment of the invention may be used, for example, in assays differentiating between the wild-type and the mutant sequence. For example, the fragment of the invention may be useful as probes in Northern or Southern Blot analysis of RNA or DNA preparations, respectively, or can be used as oligonucleotide primers in PCR analysis dependent on their respective size. Also comprised by the invention are fragments which are useful for analysing DNA-Protein interactions via, e.g., electrophoretic mobility shift analysis (EMSA). The person skilled in the art is familiar with the preparation and the use of said probes (see, e.g., Sambrook and Russel "Molecular Cloning, A Laboratory Manual", Cold Spring Harbor Laboratory, N.Y. (2001)). It is further preferred that the fragment of the invention consists of at least 14, more preferred at least 15, more preferred at least 17 nucleotides, more preferred at least 21 nucleotides, and most preferred at least 25 nucleotides such as 30 nucleotides. Fragments of the present invention to be used as a probe preferably comprise at least 100, more preferably at least 200, or most preferably at least 500 nucleotides in length. The nucleotide corresponding to the positions outlined in (b) above is preferably comprised in the fragments of the present invention in a central location, wherein "central" refers to an equal number of nucleotides being located in the 5'- and 3' -direction adjacent to the referenced nucleotide position. In a different preferred embodiment at least 60, such as at least 70, preferably at least 80, more preferably at least 90 percent of nucleotides are located in the 5'- or 3'-direction adjacent to the nucleotide position referenced in (b) above and the remaining nucleotides are located in the opposite direction.

In accordance with the present invention it was discovered for the first time that specific sub-haplotypes of the gene encoding the dopamine D4 receptor (*DRD4*) are highly significantly associated with feather pecking behaviour in an experimental (P = 7.30 x 10⁻⁷) as well as in a commercial chicken line (P = 2.78 x 10⁻⁶). In the present invention, *DRD4* in the chicken genome has been annotated and was re-sequenced in 140 animals belonging to experimental layer lines divergently selected for high and low propensity to feather pecking, the unselected founder population, and two commercial lines with low and high propensity to feather pecking.
Furthermore, haplotype analysis indicated that causal variation could be located downstream of *DRD4.* Because linkage disequilibrium (LD) extends into a neighbouring gene encoding deformed epidermal autoregulatory factor 1 (*DEAF1*), the product of which regulates the transcription of the gene encoding serotonin (5-hydroxytryptamine, 5-HT) 1A receptor, *DEAF1* was considered another candidate gene for feather pecking. Thus, in accordance with the present invention five animals homozygous for the "low-pecking" sub-haplotype and of six animals homozygous for the "high-pecking" sub-haplotype were re-sequenced delineating a LD block of 14,833 bases spanning the two genes. Any of the polymorphisms tagging the associated haplotypes within the LD block can be used for selection against feather pecking behaviour in the analyzed populations. The fact that the same haplotypes are associated in genetically distant populations such as the commercial high pecking line (derived from the Rhode Island Red breed) and the selection lines (derived from the White Leghorn breed) indicates the origin of the allele predisposing to high feather pecking to predate the breeding activities of at least the last 50 years. The predisposing allele is absent in the commercial low pecking line (also originating from the White Leghorn breed). The draft sequence that was derived from Red Jungle Fowl (Hillier *et al*. 2004), the wild form of the domesticated chicken, contains the "low-pecking" allele. Thus the "low-pecking" allele may be the ancestral and the "high-pecking" allele a more recent version that, however, arose prior to modern chicken breeding. As a consequence, typing of polymorphisms tagging the haplotypes shown to be associated in accordance with the present invention can be applied to select against feather pecking in a wide range of layer and possibly also stocks bred for purposes other than egg production. Table 1 represents a summary of the polymorphisms found to be associated with pecking behaviour.

**Table 1: Characterization of DEAF1 and DRD4 polymorphisms. The left column indicates the position in SEQ ID NO:1 relative to the first nucleotide, which is numbered "1". Wild-type (low pecking) refers to the nucleotide of the invention as represented in SEQ ID NO:1. The respective nucleotides associated with low feather pecking behaviour are presented in the middle column. Mutated (high pecking) refers to the mutated nucleic acid molecule of the invention, wherein at the indicated positions in SEQ ID NO:1 nucleotide polymorphisms associated with high feather pecking behaviour were identified (see right column). del = deletion; ins = insertion; A = adenosine; C = cytosine; G = guanosine; T = thymidine.**

| **Position in SEQ ID NO:1** | **Wild-typ (low pecking)** | **Mutated (high-pecking)** |
|---|---|---|
| 10252 | C | T |
| 10318 | T | C |
| 10324 | G | A |
| 10747 | A | C |
| 11041 | A | C |
| 11238 | C | T |
| 11244 | A | G |
| 11255 | G | A |
| 11271 | C | A |
| 11315 | T | C |
| 11617 | A | G |
| 11723 | G | T |
| 11848 | T | A |
| 12148 | A | G |
| 12159 | A | G |
| 12184 | C | T |
| 12220 | A | G |
| 12270 | T | A |
| 12295 | G | C |
| 12327 | . C | T |
| 12381 | C | G |
| 12388 | A | G |
| 12564 | A | G |
| 12979 | T | G. |
| 13265 | G | A |
| 13286 | C | T |
| 13328 | G | C |
| 13388 | | ins-AA |
| 14116 | T | C |
| 14210 | T | C |
| 14260 | G | A |
| 14392 | A | G |
| 14462 | A | G |
| 14554 | A | G |
| 14562 | A | G |
| 14574 | G | C |
| 14607 | G | A |
| 14613 | A | G |
| 14629 | T | C |
| 14637 | T | C |
| 14652 | G | A |
| 14667 | A | G |
| 14669 | G | C |
| 14679 | A | G |
| 14707 | G | T |
| 14721 | | ins-ATC |
| 14747 | G | A |
| 14753 | G | A |
| 14809 | T | C |
| 14832 | A | G |
| 14925 | G | A |
| 15154 | C | G |
| 15537 | G | A |
| 15672 | A | G |
| 15674 | G | T |
| 16162 | G | A |
| 16537 | A | G |
| 16863 | A | G |
| 16911 | C | T |
| 16941 | | insT |
| 16983 | A | G |
| 17286 | A | G |
| 17329 | A | G |
| 17369 | C | G |
| 17371 | T | C |
| 17435 | C | G |
| 17607 | G | A |
| 17661-63 | | deITCT |
| 17715 | A | G |
| 17744 | T | C |
| 18129 | T | C |
| 18259 | T | C |
| 18351 | A | C |
| 18639 | G | A |
| 18678 | | delG |
| 18772 | A | G |
| 18991 | G | T |
| 19357 | G | A |
| 21265 | A | G |
| 21307 | A | G |
| 21409 | T | C |
| 21623 | A | G |
| 22298 | G | A |
| 22754 | A | G |
| 22868 | A | G |
| 23341 | A | G |
| 23416 | A | G |
| 23737 | A | C |
| 23812 | T | G |
| 23847 | A | G |
| 23850 | T | C |
| 23851 | G | A |
| 23909 | A | C |
| 23914 | G | C |
| 24051 | C | T |
| 24155 | A | G |
| 24213 | C | G |
| 24247 | C | T |
| 24568 | | delT |
| 24574 | A | G |
| 24891 | T | C |
| 25004 | T | C |
| 25033 | G | A |
| 25062 | G | A |
| 25067 | T | G |
| 25085 | T | C |

Any of the non-coding polymorphisms may be located in an unknown regulatory region and have functional consequences. Three polymorphisms in the putative 3'-UTRs of *DEAF1* and *DRD4* are of particular interest, namely the polymorphisms at position 21307, 21409 and 21623 of SEQ ID NO:1 (SNP numbers 2246, 2247 and 2248). Since 3'-UTRs of *DRD4* and *DEAF1* might overlap, there is the intriguing possibility that the transcripts from opposite strands interact at their 3' ends in a sense-antisense manner (Sun *et al*. 2005; Sanna *et al*. 2008). The extent of such an interaction could depend on structural differences of the RNA molecules conferred by the polymorphisms and could affect the translation of one or the other or both genes.

Thus, functional studies should, inter alia, be focused on polymorphisms located in the 3'-UTRs of both genes.

Further, the invention relates to a nucleic acid molecule deviating by at least one mutation from the nucleic acid molecule of the invention, wherein said mutation is contributing to or indicative of high feather pecking and is (a) a substitution; (b) an insertion; and/or (c) a deletion.

The term "mutation" in accordance with the invention refers to changes in the nucleotide sequence of the genetic material and includes deletion, insertion, or substitution of one or more nucleotides in the gene.

In accordance with the present invention, this nucleic acid molecule deviating from the nucleic acid molecule of the invention by at least one mutation is also referred to herein as the "mutant nucleic acid molecule".

The term "substitution" in accordance with the present invention, refers to point mutations including such mutations resulting in an amino acid exchange as well as mutations that are not reflected on the amino acid level.

The term "insertion" in accordance with the present invention refers to the addition of one or more nucleotides to a nucleic acid molecule, wherein the addition is not to the 5' or 3' end of the nucleic acid molecule.

The term "deletion" as used in accordance with the present invention refers to the loss of nucleotides.

As discussed above, in accordance with the present invention an LD block of 14,833 bases associated with feather pecking behaviour has been identified. Any of the polymorphisms tagging the associated haplotypes within the LD block can be used for selection against feather pecking behaviour in the analyzed populations due to their linkage to the mutation causing the phenotype. Linkage refers to the phenomenon that the DNA sequences which are close together in the genome have a tendency to be inherited together. Two or more sequences may be linked because of some selective advantage of co-inheritance. More typically, however, two or more polymorphic sequences are co-inherited because of the relative infrequency with which meiotic recombination events occur within the region between the two polymorphisms. The co-inherited polymorphic alleles are said to be in linkage disequilibrium with one another because, in a given population, they tend to either both occur together or else not occur at all in any particular member of the population. Indeed, where multiple polymorphisms in a given chromosomal region are found to be in linkage disequilibrium with one another, they define a quasi-stable genetic "haplotype." Furthermore, where a phenotype-causing mutation is found within or in linkage with this haplotype, one or more polymorphic alleles of the haplotype can be used as a diagnostic or prognostic indicator of the likelihood of developing a specific phenotype. Identification of a haplotype which spans or is linked to a phenotype-causing mutational change serves as a predictive measure of an individual's likelihood of having inherited that phenotype-causing mutation. Importantly, such prognostic or diagnostic procedures can be utilized without necessitating the identification and isolation of the actual phenotype-causing molecule. This is significant because the precise determination of the molecular basis of the establishment of a specific phenotype can be difficult and laborious, especially in the case of a multifactorial phenotype.

In a preferred embodiment of the mutated nucleic acid molecule of the invention, said substitution is an (i) adenosine to cytosine exchange at a position corresponding to position 10747, 11041, 18351, 23737 and/or 23909 of SEQ ID NO:1; (ii) cytosine to adenosine exchange at a position corresponding to position 11271 of SEQ ID NO:1; (iii) guanosine to adenosine exchange at a position corresponding to position 10324, 11255, 13265, 14260, 14607, 14652, 14747, 14753, 14925, 15537, 16162, 17607, 18639, 19357, 22298, 23851, 25033 and/or 25062 of SEQ ID NO:1; (iv) adenosine to guanosine exchange at a position corresponding to position 11244, 11617, 12148, 12159, 12220, 12381, 12564, 14392, 14462, 14554, 14562, 14613, 14667, 14679, 14832, 15672, 16537, 16863, 16983, 17287, 17329, 17715, 18772, 21265, 21307, 21623, 22754, 22868, 23341, 23416, 23847, 24155 and/or 24574 of SEQ ID NO:1; (v) thymidine to adenosine exchange at a position corresponding to position 11848 and/or 12270 of SEQ ID NO:1; (vi) guanosine to cytosine exchange at a position corresponding to position 12295, 13328, 14574, 14669 and/or 23914 of SEQ ID NO:1; (vii) cytosine to guanosine exchange at a position corresponding to position 12338, 15154, 17369, 17435 and/or 24213 of SEQ ID NO:1; (viii) thymidine to cytosine exchange at a position corresponding to position 10318, 11315, 11315, 14116, 14210, 14629, 14637, 14809, 17371, 17744, 18129, 18259, 21409, 23850, 24891, 25004 and/or 25085 of SEQ ID NO:1; (ix) cytosine to thymidine exchange at a position corresponding to position 10252, 11238, 12184, 12327, 13286, 16911, 24051 and/or 24247 of SEQ ID NO:1; (x) thymidine to guanosine exchange at a position corresponding to position 12979, 23812 and/or 25067 of SEQ ID NO:1; and/or a (xi) guanosine to thymidine exchange at a position corresponding to position 11723, 14717, 15674 and/or 18991 of SEQ ID NO:1.

In another preferred embodiment of the mutated nucleic acid molecule of the invention, said insertion is a (i) 3 nucleotide insertion of adenosine-thymidine-cytosine at a position corresponding to position 14720 of SEQ ID NO:1; (ii) 2 nucleotide insertion of adenosine-adenosine at a position corresponding to position 13388 of SEQ ID NO:1; and/or a (iii) thymidine insertion at a position corresponding to position 16940 of SEQ ID NO:1.

In an alternative preferred embodiment of the mutated nucleic acid molecule of the invention, said deletion is a (i) deletion of a guanosine at a position corresponding to position 18678 of SEQ ID NO:1; (ii) 3 nucleotide deletion of thymidine-cytosine-thymidine at a position corresponding to position 17661 to 17663 of SEQ ID NO:1; and/or a (iii) thymidine deletion at a position corresponding to position 24568 of SEQ ID NO:1.

The above recited substitutions, insertions and deletions have been summarised in Table 1 above (right column; "Mutated (high-pecking)") .

In a further preferred embodiment the nucleic acid molecule of the invention or the mutated nucleic acid molecule of the invention is DNA.

In a more preferred embodiment, the nucleic acid molecule of the invention or the mutated nucleic acid molecule of the invention is genomic DNA.

The present invention also relates to a nucleic acid molecule of at least 12 contiguous nucleotides which is complementary to the nucleic acid molecule of the invention or the mutated nucleic acid molecule of the invention.

The term "a nucleic acid molecule which is complementary" refers to a nucleic acid molecule the sequence of which is uniquely fitting to (e.g. complementary to preferably 100%) the sequence of the (wild-type) nucleic acid molecule of the invention but not to the mutated nucleic acid molecule of the invention as defined above or, alternatively, uniquely fitting to (e.g. complementary to preferably 100%) the sequence of the mutated nucleic acid molecule of the invention but not to the (wild-type) nucleic acid molecule of the invention.

Complementary nucleic acid molecules are, for example, useful in the analysis of the genetic setup in the recited positions in hybridisation assays, such as for example in Northern or Southern Blot analysis of RNA or DNA preparations, respectively. Thus, for example, a nucleic acid molecule exactly complementary either to a sequence having the low pecking allele or to a sequence having the high pecking allele associated with pecking behaviour may be used to differentiate between the polymorphic variants. This is because a nucleic acid molecule labelled with a detectable label not exactly complementary to the DNA in the analyzed sample will not give rise to a detectable signal, if appropriate hybridization and washing conditions are chosen.
In this regard, it is important to note that the nucleic acid molecule, the mutated nucleic acid molecule of the invention and the complementary nucleic acid molecule may be detectably labelled. Detectable labels include radioactive labels such as ³H, or ³²P or fluorescent labels. Labelling of nucleic acids is well understood in the art and described, for example, in Sambrook and Russel "Molecular Cloning, A Laboratory Manual", Cold Spring Harbor Laboratory, N.Y. (2001).
The person skilled in the art is familiar with the preparation and the use of such probes (see, e.g., Sambrook and Russel "Molecular Cloning, A Laboratory Manual", Cold Spring Harbor Laboratory, N.Y. (2001)). These nucleic acid molecules may be chemically synthesized or transcribed by an appropriate vector containing a chimeric gene which allows for the transcription of the nucleic acid molecule in the cell. Such complementary nucleic acid molecules may be used in any of the above described methods, such as for example in Northern or Southern Blot analysis of RNA or DNA preparations. In particular, they may be used in distinguishing nucleic acid molecules carrying the mutations indicative of and/or causative for high feather pecking from nucleic acid molecules encoding the wild-type nucleic acid molecule.
Furthermore, as the complementary nucleic acid molecules are equally associated with pecking behaviour, they may be directly analysed for the presence of the respective complementary nucleotides at the polymorphic sites of the nucleic acid molecule of the invention. For example, the presence of the respective complementary nucleotides at the positions of SEQ ID NO:1 indicated in Table 1 above may be analysed. The skilled person knows which nucleotides are the respective complementary nucleotides, namely thymidine if the nucleic acid molecule of the invention or the mutated nucleic acid molecule of the invention has at the analysed position an adenosine; adenosine if the nucleic acid molecule of the invention or the mutated nucleic acid molecule of the invention has at the analysed position a thymidine; guanosine if the nucleic acid molecule of the invention or the mutated nucleic acid molecule of the invention has at the analysed position a cytidine; and cytidine if the nucleic acid molecule of the invention or the mutated nucleic acid molecule of the invention has at the analysed position a guanosine.

The complementary nucleic acid molecule of the present invention consists most preferably of at least 12 nucleotides, but may also consist of at least 14, 15, 17, 19, 25, 50, 100, 150, 200 or more nucleotides.

In addition, the invention relates to a vector comprising the nucleic acid molecule as described above.
The vector of the invention may either contain a nucleic acid molecule comprising the wild-type sequence, a nucleic acid molecule comprising the mutated sequence or it may contain a complementary nucleic acid molecule or a fragment of the invention as described above.
The term "vector" in accordance with the present invention refers to a vector that is a plasmid, cosmid, virus, bacteriophage or another vector. Such vectors may be used e.g. in genetic engineering. Incorporation of the nucleic acid into a vector offers the possibility of introducing the nucleic acid molecule efficiently into the cells and preferably the DNA of a recipient. The recipient may be a single cell such as a cell from a cell line. Such a measure renders it possible to express, when expression vectors are chosen, the respective nucleic acid molecule in the recipient. Thus, incorporation of the nucleic acid molecule into an expression vector opens up the way to a permanently elevated level of the encoded protein in any cell or a subset of selected cells of the recipient. In a preferred embodiment, the recipient is a mammal. In a more preferred embodiment, the mammal is a human.
The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146), pBC12MI (ATCC 67109) or pcDNA3.1 (Invitrogen), preferably pcDNA3.1/V5/His or pcDNA5/FRT/TO (Invitrogen).
The nucleic acid molecule referred to above may also be inserted into vectors such that a translational fusion with another nucleic acid molecule is generated. The vectors may also contain an additional expressible polynucleotide coding for one or more chaperones to facilitate correct protein folding.
For vector modification techniques, see Sambrook and Russel ("Molecular Cloning, A Laboratory Manual", Cold Spring Harbor Laboratory, N.Y. (2001)). Generally, vectors can contain one or more origin of replication (ori) and inheritance systems for cloning or expression, one or more markers for selection in the host, e. g., antibiotic resistance, and one or more expression cassettes.
The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens, Proc. Natl. Acad. Sci. USA 2001, 98: 1471) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.
Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance genes, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).
The co-transfection with a selectable marker such as dihydrofolate reductase (dhfr), gpt, neomycin, hygromycin or G418 allows the identification and isolation of the transfected cells. The transfected nucleic acid can also be amplified to express large amounts of the encoded (poly)peptide. The DHFR marker is useful to develop cell lines that carry several hundred or even several thousand copies of the gene of interest. Another useful selection marker is the enzyme glutamine synthase (GS) (Murphy et al., Biochem J. 1991, 227:277; Bebbington et al., Bio/Technology 1992, 10:169). Using these markers, the mammalian cells are grown in selective medium and the cells with the highest resistance are selected. As indicated above, the expression vectors will preferably include at least one selectable marker.
The nucleic acid molecules as described herein may be designed for direct introduction or for introduction via liposomes, phage vectors or viral vectors (e.g. adenoviral, retroviral) into the cell. Additionally, baculoviral systems or systems based on vaccinia virus or Semliki Forest virus can be used as eukaryotic expression system for the nucleic acid molecules of the invention.

The present invention further relates to a non-human host transformed with the vector of the invention.
The host may either carry the mutant or the wild-type sequence. Upon breeding etc. the host may be heterozygous or homozygous for one or more of the polymorphisms.

The host of the invention may carry the vector of the invention either transiently or stably integrated into the genome. Methods for generating the non-human host of the invention are well known in the art. For example, conventional transfection protocols described in Sambrook et al., Ioc. cit., may be employed to generate transformed bacteria (such as E. coli) or transformed yeasts. The non-human host of the invention may be used, for example, to elucidate the functional implications of the variants. For example, the polymorphisms might be located in an unknown regulatory region and have functional consequences that may conveniently be analysed in a non-human host of the invention.

In a preferred embodiment of the invention the non-human host is a bacterium, a yeast cell, an insect cell, a fungal cell, an avian cell, a mammalian cell, a plant cell, a transgenic animal or a transgenic plant.
Whereas E. coli is a preferred bacterium, preferred yeast cells are S. cerevisiae or Pichia pastoris cells. Preferred fungal cells are Aspergillus cells and preferred insect cells include Spodoptera frugiperda cells. Preferred avian cells are neuronal cell lines or primary cells showing expression of the *DRD4* or *DEAF1* gene. Appropriate culture mediums and conditions for the above-described host cells are well known in the art.

A method for the production of a transgenic non-human animal, for example transgenic mouse, comprises introduction of the aforementioned nucleic acid molecule or targeting vector into a germ cell, an embryonic cell, stem cell or an egg or a cell derived therefrom. Production of transgenic embryos and screening of those can be performed, e. g., as described by A. L. Joyner Ed., Gene Targeting, A Practical Approach (1993), Oxford University Press. The DNA of the embryonal membranes of embryos can be analyzed using, e. g., Southern blots with an appropriate complementary nucleic acid molecule; see supra. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62: 1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326: 292-295 (1987) ), the D3 line (Doetschman et al., J. Embryo. Exp. Morph. 87: 27-45 (1985) ), the CCE line (Robertson et al., Nature 323: 445-448 (1986) ), the AK-7 line (Zhuang et al., Cell 77: 875-884 (1994) ). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are born as chimeric mice. The resultant transgenic mice are chimeric for cells having the desired nucleic acid molecule are backcrossed and screened for the presence of the correctly targeted transgene (s) by PCR or Southern blot analysis on tail biopsy DNA of offspring so as to identify transgenic mice heterozygous for the nucleic acid molecule of the invention.

Transgenesis in birds can be achieved by methods well known to the skilled person, for example by using lentiviral vectors or via genetically modified avian primordial germ cells (Sang 2004; Sang 2006; van de Lavoir *et al*. 2006).

The transgenic non-human animals may, for example, be transgenic mice, rats, hamsters, dogs, monkeys (apes), rabbits, pigs, cows or birds. Preferably, said transgenic non-human animal is a bird, more preferably a chicken. The transgenic animals of the invention are, inter alia, useful to study the phenotypic expression/outcome of the nucleic acid molecules and vectors of the present invention. It is furthermore envisaged, that the non-human transgenic animals of the invention can be employed to test for therapeutic agents/compositions.

The present invention further relates to a method of testing an avian for its predisposition for low feather pecking comprising analyzing in a sample the nucleic acid molecule of the invention for nucleotide polymorphisms which are linked to said predisposition.

The present invention also refers to a method of testing an avian for its predisposition for high feather pecking comprising analyzing in a sample the mutated nucleic acid molecule of the invention for nucleotide polymorphisms which are linked to said predisposition.

The term "predisposition" refers to a genetic precondition for either low or high feather pecking or to pass on to the offspring specific alleles or variants of a gene responsible for developing such a phenotype. In accordance with the present invention, the nucleic acid molecule of the invention representing the wild-type sequence is associated with low feather pecking whereas a difference from this wild-type sequence as characterised in the mutated nucleic acid molecule of the invention is indicative of high feather pecking.

As used herein, the term "avian" includes for example chicken, turkey, duck, goose, quail, pheasants, parrots and ratites including ostrich, emu and cassowary.

The discovery of the present invention allows to reliably predict which animals have a predisposition for either low or high feather pecking and/or may pass on this predisposition to their offspring. Based on this knowledge the welfare and economic implications of high feather pecking, which include loss of heat due to damage to the plumage, higher feed consumption and consequently an impaired feed efficiency (Tauson & Svensson 1980) as well as cannibalism and high mortality rates (Kjaer & Sorensen 2002), can be overcome in, for example, chicken farms or poultry breeding programs.

Said testing or analyzing may be performed by PCR, Southern and Northern Blotting (Sambrook et al., "Molecular Cloning, A Laboratory Manual"; ISBN: 0879695765, CSH Press, Cold Spring Harbor, 2001) but may also comprise "ligase chain reaction"( LCR, EPA320308), "cyclic probe reaction" (CPR), "strand displacement amplification" (SDA, Walker et al. 1992, Nucleic Acid Res. (7): 1691-1696), "transcription based amplification systems" (TAS, Kwoh et al. 1989, Proc. Nat. Acad. Sci. USA 86: 1173, Gingeras et al., PCT Application WO 88/10315), allele-specific hybridization, allele-specific primer extension, allele-specific oligonucleotide ligation, allele-specific cleavage of a flap probe, allele-specific enzyme cleavage, allele specific PCR, Taqman PCR, Pyrosequencing, Snapshot, Restriction Fragment length Polymorphism Analysis (RFLP) or Maldi-Tof.

Particularly preferred according to the present invention is the testing comprising a step of amplification of the nucleic acid molecule. In the present invention, the term "amplification" or "amplify" means increase in copy number and is particularly advantageous in cases where limited amounts of the sample are present. The person skilled in the art knows various methods to amplify nucleic acid molecules. Amplification methods include, but are not limited to, "polymerase chain reaction" (PCR), "ligase chain reaction" (LCR, EPA320308), "cyclic probe reaction" (CPR), "strand displacement amplification" (SDA, Walker et al. 1992, Nucleic Acid Res. (7): 1691-1696), "transcription based amplification systems" (TAS, Kwoh et al. 1989, Proc. Nat. Acad. Sci. USA 86: 1173, Gingeras et al., PCT Application WO 88/10315). Preferably, amplification of DNA is accomplished by using polymerase chain reaction (PCR). The PCR consists of many repetitions of a cycle which consists of: (a) a denaturation step, which melts both strands of a DNA molecule; (b) an annealing step, which is aimed at allowing the primers to anneal specifically to the melted strands of the DNA molecule; and (c) an extension step, which incorporates to the primers complementary to those of the strand of DNA to which the primers are annealed. The concentrations of primers, nucleotidetriphosphates, enzyme and buffers used will be apparent from and include the process parameters described in the Examples that follow. However, generally, PCR can be performed for example in a 50 µl reaction mixture containing 5 µl of 10 x PCR buffer with 1.5 mM MgCl₂, 200 µM of each deoxynucleoside triphosphate, 0.5 µl of each primer (10 µM), 0.5 µl, 30 ng of template DNA and 2.5 Units of Taq Polymerase. The primers for the amplification may be labelled or be unlabelled. DNA amplification can be performed, e.g., with a model 2400 thermal cycler (Applied Biosystems, Foster City, CA): 2 min at 94°C, followed by 35 cycles consisting of annealing (30 s at 50°C), extension (1 min at 72°C), denaturation (10 s at 94°C) and a final annealing step at 55°C for 1 min as well as a final extension step at 72°C for 5 min. However, the person skilled in the art knows how to optimize these conditions for the amplification of specific nucleic acid molecules (see for example Sambrook et al., "Molecular Cloning, A Laboratory Manual"; ISBN: 0879695765, CSH Press, Cold Spring Harbor, 2001).
A further method of nucleic acid amplification is the "reverse transcriptase polymerase chain reaction" (RT-PCR). This method is used when the nucleic acid to be amplified consists of RNA. The term "reverse transcriptase" refers to an enzyme that catalyzes the polymerisation of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. Suitable polymerases for use with a DNA template include, for example, E. coli DNA polymerase I or its Klenow fragment, T.sub.4 DNA polymerase, Tth polymerase, and Taq polymerase, a heat-stable DNA polymerase isolated from Thermus aquaticus and developed and manufactured by Hoffmann-La Roche and commercially available from Perkin Elmer. The latter enzyme is widely used in the amplification and sequencing of nucleic acids. The reaction conditions for using Taq polymerase are known in the art and are described, e.g., PCR Technology, Erlich, H. A. 1989. Stockton Press, New York or Innis, M. A., D. H. Gelfand, J. J. Sninsky, and T. J. White. 1990. PCR Protocols: A guide to methods and applications. Academic Press, New York.
High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer 4 µl, 2 µl of Oligo dT(100 µg/ml), 2µl of 10 mM dNTPs ,1 µl total RNA, 10 Units of AMV reverse transcriptase and H₂O to 20 µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample.

In a further preferred embodiment of the present invention's method of testing an avian for its predisposition for high or low feather pecking, the method of amplifying the nucleic acid by PCR comprises cycles of (a) stringent hybridization with at least two primers sufficiently complementary to bind to the two strands of target DNA under stringent conditions of hybridization, wherein at least one primer comprises at least one polymorphic position indicated in table 1, having in the polymorphic position of the nucleic acid molecule of the invention the sequence of a variant and not of wild-type; and (b) strand elongation and denaturing, wherein (i) a failure to generate a PCR product is indicative of a predisposition for low feather pecking and (ii) the generation of a PCR product is indicative of a predisposition for high feather pecking.

In another preferred embodiment of the present invention's method of testing an avian for its predisposition for high or low feather pecking, the method of amplifying the nucleic acid by PCR comprises cycles of (a) stringent hybridization with at least two primers sufficiently complementary to bind to the two strands of target DNA under stringent conditions of hybridization, wherein at least one primer comprises at least one polymorphic position indicated in table 1, but having the sequence of the nucleic acid molecule of the invention corresponding to the wild-type sequence in said position; and (b) strand elongation and denaturing, wherein (i) a failure to generate a PCR product is indicative of a predisposition for high feather pecking and (ii) the generation of a PCR product is indicative of a predisposition for low feather pecking.

In another embodiment of the method of the invention, said testing or analyzing may comprise (a) hybridizing a probe under stringent conditions to a sequence of the nucleic acid molecule of the invention or to a transcription product therefrom, wherein (i) the probe comprises at least one of the polymorphic positions of table 1, and (ii) the conditions of hybridization are such that binding can only occur if the sequence of the probe and the target nucleic acid molecule at the polymorphic position are completely complementary and (b) detecting whether hybridization has occurred, wherein hybridization of a probe comprising at least one of the polymorphic positions of table 1 is indicative of a predisposition for high feather pecking.

The choice and design of the probe particularly depends on the base composition of the target nucleic acid molecule to which the probe hybridizes. Generally, a probe is designed so that the presence of as few as one mismatch in the base pairing of the probe with the target nucleic acid molecule is detectable. Typically, the nucleotide sequence of such a probe corresponds to a nucleotide sequence in a region of the nucleic acid molecule of the invention suspected of containing a mutation. Preferentially, the probe comprises at least one of the polymorphic positions of table 1. However, the test may also comprise hybridizing a probe of wild-type sequence to a sequence of the nucleic acid molecule of the invention. In this case, an absence of hybridization is indicative of a predisposition for high feather pecking, whereas hybridization would identify an animal with a predisposition for low feather pecking.
The person skilled in the art knows various techniques for detecting mismatches in the base pairing of nucleic acid molecules. Preferably, these techniques include, but are not limited to, electrophoretic mobility shift assays or thermal mobility shift assays. Another preferred method is a mutation-sensitive PCR. The term "mutation-sensitive PCR" relates to a PCR approach comprising the use of primers which either allow or prevent the generation of amplification products. According to this technique, primers comprising at least one of the polymorphic positions outlined in the right column of table 1 would not be capable of binding to wild-type nucleic acid molecule of the invention. Hence, the absence of an amplification product would be indicative of a predisposition for low feather pecking. On the other hand, if the primers are of wild-type sequence, i.e. comprising at least one of the polymorphic positions outlined in the middle column of table 1, the presence of an amplification product would be indicative of a predisposition for low feather pecking.

In a preferred embodiment of the present invention, said probe is detectably labeled. Any of the preferred labels of the present invention (see above) may be used for labeling the probe. However, a reporter dye and a quenching dye are particularly preferred according to the present invention.

In another embodiment of the method of the present invention said testing or analyzing comprises determining the nucleic acid sequence of at least a portion of said nucleic acid molecule. The nucleic acid sequence may be determined by using any of the nucleic acid sequencing techniques known in the art (see Sambrook et al., "Molecular Cloning, A Laboratory Manual"; ISBN: 0879695765, CSH Press, Cold Spring Harbor, 2001). However, particularly preferred according to the present invention are the methods of Sanger and Maxam/Gilbert.

In a more preferred embodiment of the present invention, the determination of the nucleic acid sequence is effected by solid-phase minisequencing. The solid-phase minisequencing method is designed to detect single base changes or small deletions and insertions in amplified DNA. Solid-phase refers to the solid matrix employed to immobilise the target DNA allowing a minisequencing reaction to take place. The detection of the alteration in the base sequence occurs on the immobilised sequence by incorporation of a single residue of a labeled nucleotide. Typically streptavidin coated magnetic beads or streptavidin coated wells in microtitreplates provide a suitable support, taking advantage of the interaction between biotin and avidin. For diagnostic purposes microtitreplates are preferred as they facilitate large-scale population screening. The magnetic bead approach may be used in combination with microtitreplates.

Typically, a DNA fragment under investigation for a known mutation is enzymatically amplified by using one unbiotinylated and one 5' biotinylated primer. The PCR product carrying the biotin labelled residue is then captured at the 5' end onto the steptavidin coated solid matrix. The non-biotinylated strand and excess primers are eluted under alkaline conditions leaving the single stranded template accessible for the primer extension reaction. A specific sequencing primer is to anneal to the target DNA immediately upstream of the base change in the sequence. Primer elongation of the target DNA sequence occurs in the presence of Taq polymerase and a mixture of dNTPs and ddNTPS. Usually two different labelled dNTPs are required to detect the nucleotide variation. This allows identification of nucleotide substitutions in samples from both heterozygous and homozygous individuals. A homozygous individual will generate only one signal corresponding to the nucleotide present whereas a heterozygote will produce a signal from the reaction with both labelled dNTPs. The dNTPs may be labelled radioactively using [³H] or [³²P] or non-radioactively with digoxygenin or fluorescein. Inclusion of the ddNTPs is necessary for complete termination of the reaction. The radioactive emissions are counted in scintillation fluid in a liquid scintillation counter. If non-radioactive labelling is utilised the sequencing reaction requires a long incubation stage followed by several washing steps. Incorporation of the labelled nucleotide is detected through immunological reaction with an enzyme labelled anti-hapten conjugate and a substrate. Genotyping can be achieved by comparing photochemical signals or radioactive emissions. The solid-phase minisequencing method described here can be applied to any known point mutation, deletion or insertion. The same reaction conditions can be used to detect several mutations. The main drawback is the inability to perform multiple analyses in a single reaction. However safer and more cost-effective methods, which are preferred methods according to the present invention, have been developed including RFLP analysis and ARMS PCR and more recently automated fluorescent sequencing. These methods are more efficient at picking up known mutations and therefore more suitable for diagnostic screening purposes.

In a preferred embodiment of the method of the invention of testing an avian for its predisposition for low feather pecking, said nucleotide polymorphism is (a) an adenosine at a position corresponding to position 10747, 11041, 11244, 11617, 12148, 12159, 12220, 12381, 12564, 14392, 14462, 14554, 14562, 14613, 14667, 14679, 14832, 15672, 16537, 16863, 16983, 17287, 17329, 17715, 18351, 18772, 21265, 21307, 21623, 22754, 22868, 23341, 23416, 23737, 23847, 23909, 24155 and/or 24574 of SEQ ID NO:1; (b) a cytosine at a position corresponding to position 10252, 11238, 11271, 12184, 12327, 12338, 13286, 15154, 16911, 17369, 17435, 24051, 24213, 24247 and/or 25085 of SEQ ID NO:1; (c) a guanosine at a position corresponding to position 10324, 11255, 11723, 12295, 13265, 13328, 14260, 14574, 14607, 14652, 14669, 14717, 14747, 14753, 14925, 15537, 15674, 16162, 17607, 18639, 18991, 19357, 22298, 23851, 23914, 25033 and/or 25062 of SEQ ID NO:1; and/or (d) a thymidine at a position corresponding to position 10318, 11315, 11848, 12270, 12979, 14116, 14210, 14629, 14637, 14809, 17371, 17744, 18129, 18259, 21409, 23812, 24891, 25004, 25067 and/or 23850 of SEQ ID NO:1.

In a preferred embodiment of the method of the invention of testing an avian for its predisposition for high feather pecking, said nucleotide polymorphism is (a) a cytosine at the position corresponding to position 10318, 10747, 11041, 11315, 12295, 13328, 14116, 14210, 14574, 14629, 14637, 14669, 14809, 17371, 17744, 18129, 18259, 18351, 21409, 23737, 23850, 23909, 23914, 24891, 25004 and/or 25085 of SEQ ID NO:1; (b) an adenosine at a position corresponding to position 10324, 11255, 11271, 11848, 12270, 13265, 14260, 14607, 14652, 14747, 14753, 14925, 15537, 16162, 17607, 18639, 19357, 22298, 23851, 25033 and/or 25062 of SEQ ID NO:1 (c) a guanosine at a position corresponding to position 11244, 11617, 12148, 12159, 12220, 12338, 12381, 12564, 12979,14392, 14462, 14554, 14562, 14613, 14667, 14679, 14832, 15154, 15672, 16537, 16863, 16983, 17287, 17329, 17369, 17435, 17715, 18772, 21265, 21307, 21623, 22754, 22868, 23341, 23416, 23812, 23847, 24155, 24213, 24574 and/or 25067of SEQ ID NO:1; (d) a thymidine at a position corresponding to position 10252, 11238, 11723, 12184, 12327, 13286, 14717, 15674 16911, 18991, 24051 and/or 24247 of SEQ ID NO:1; (e) a 3 nucleotide insertion of adenosine-thymidine-cytosine at a position corresponding to position 14720 of SEQ ID NO:1; (f) a 2 nucleotide insertion of adenosine-adenosine at a position corresponding to position 13388 of SEQ ID NO:1; (g) a thymidine insertion at a position corresponding to position 16940 of SEQ ID NO:1; (h) a deletion of a guanosine at a position corresponding to position 18678 of SEQ ID NO:1; (i) a 3 nucleotide deletion of thymidine-cytosine-thymidine at a position corresponding to position 17661 to 17663 of SEQ ID NO:1; and/or (j) a thymidine deletion at a position corresponding to position 24568 of SEQ ID NO:1.

In a further preferred embodiment of the present invention, the sample is isolated from egg, semen, blood, feather, nail, skin or sputum. However, according to the present invention any sample may be used for testing an avian's predisposition for feather pecking.

The present invention also relates to a method for selecting an avian or part of an avian, including their semen or eggs, for low or high feather pecking, comprising the steps of: (a) testing for a predisposition for low or high feather pecking according to the steps of the methods of the present invention; (b) selecting the avian based on the information derived from said testing; and (c) optionally, using said information for further breeding considerations. Said breeding considerations may be breeding management considerations such as the propagation, culling or feeding of animals.

In another preferred embodiment of the present invention the avian is selected from the group consisting of chicken, turkey, pheasants, duck, goose, quail, parrots and ratites including ostrich, emu and cassowary.

Finally, the present invention relates to a kit for the detection of animals having a predisposition for high feather pecking, comprising a nucleic acid molecule of the invention, or the mutated nucleic acid molecule differing from the wild-type nucleic acid molecule of the invention by at least one polymorphism as defined above, or the complementary nucleic acid molecule or the fragment of the invention. The various components of the kit may be packaged in one or more containers such as one or more vials. The vials may, in addition to the components, comprise preservatives or buffers for storage. In addition, the kit may contain instructions for use

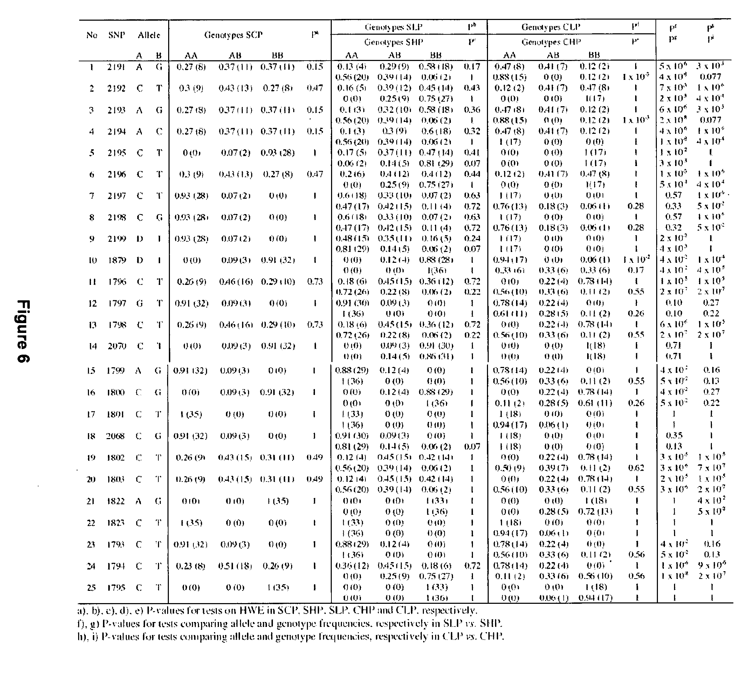

The figures show:

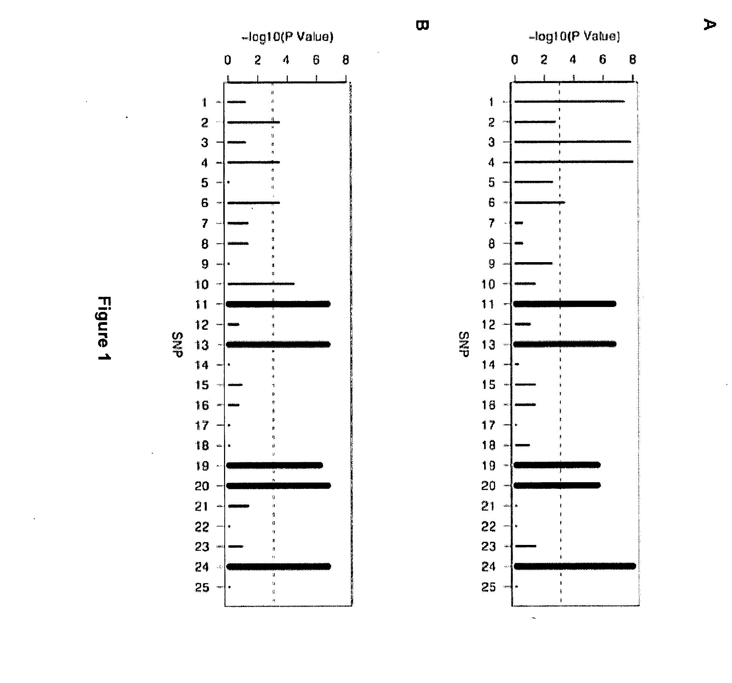

**Figure 1****.** Association of *DRD4* polymorphisms with feather pecking.
   Log transformed P values resulting from Fisher's exact test when contrasting allele frequencies: (A) selection "low-pecking" (SLP) *vs*. selection "high-pecking" (SHP) line, (B) commercial "low-pecking" (CLP) *vs*. commercial "high-pecking" (CHP) line. Dashed line indicates the Bonferroni-corrected 5% significance threshold. Thick bars indicate significant association both in the selection and in the commercial lines. The SNP numbers refer to the corresponding entries in Figure 6.

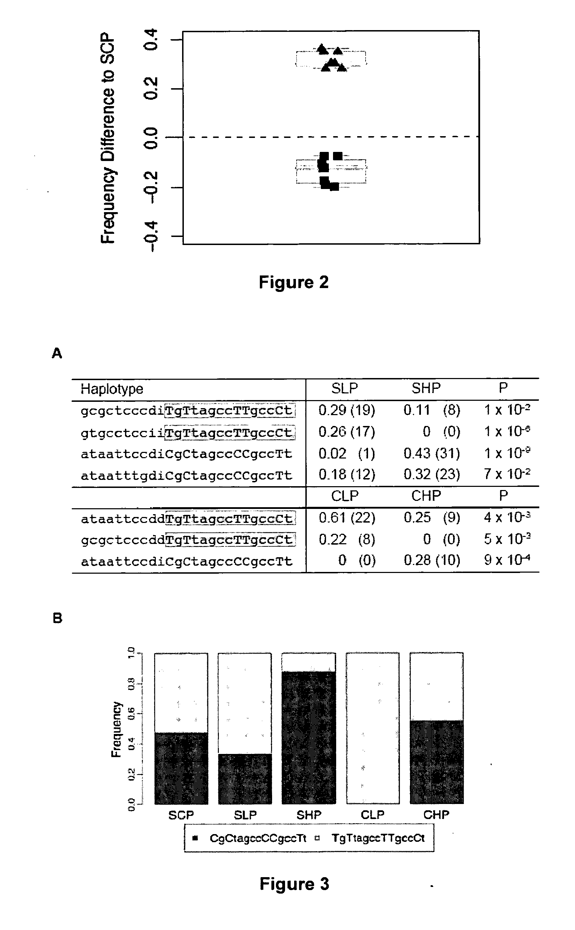

**Figure 2****.** Asymmetrical response to selection for high and low feather pecking is reflected by frequencies of *DRD4* polymorphisms. Boxplot of allele frequency differences between the "high pecking" selection line (SHP) (▲) and the "low pecking" selection line (SLP) (■), and the founding line for significantly associated SNPs.

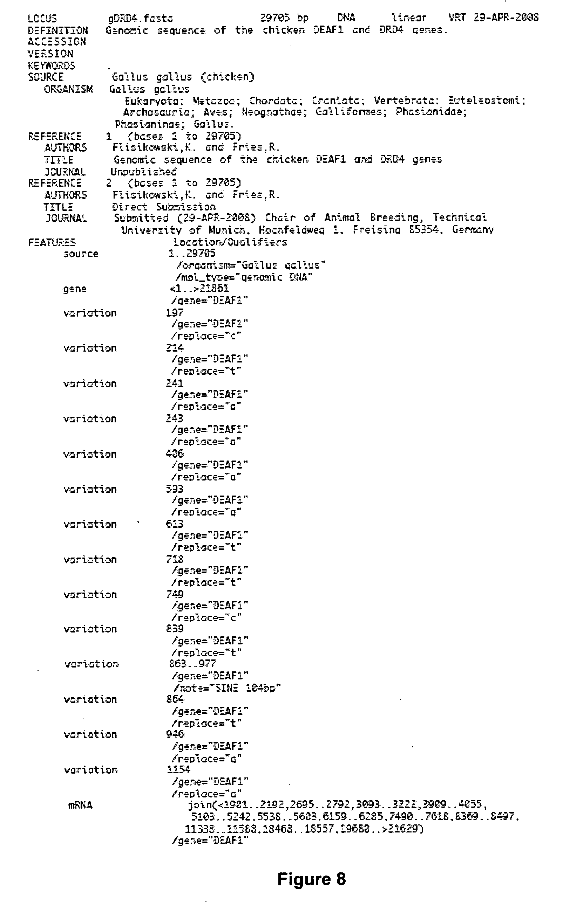

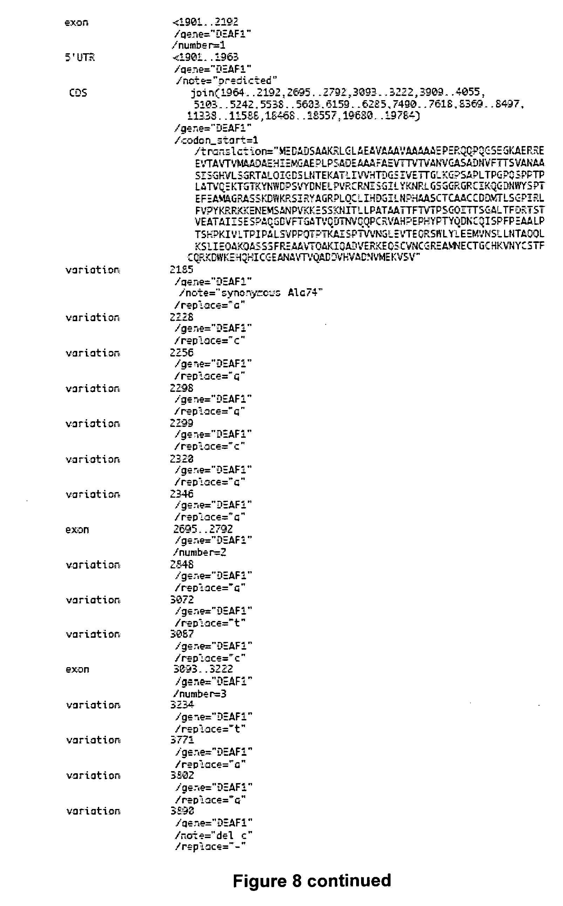

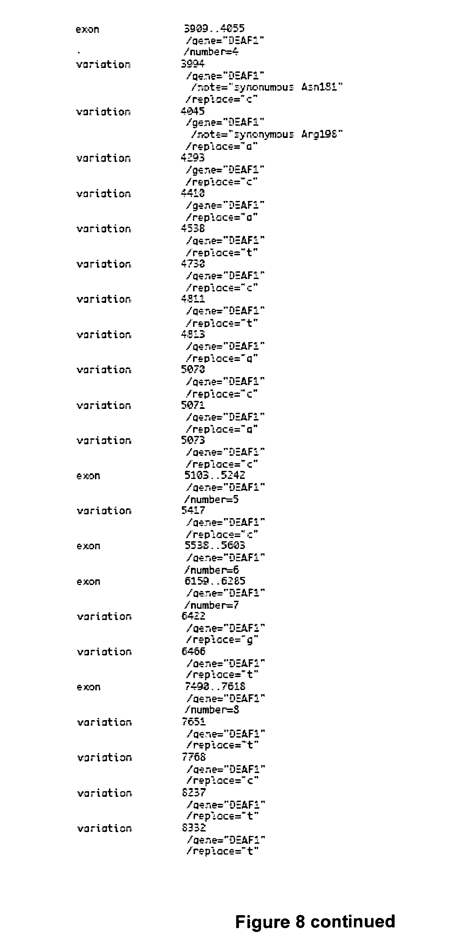

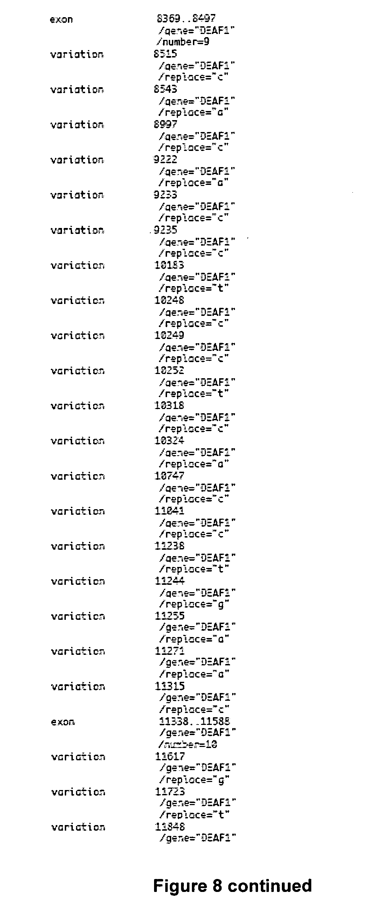

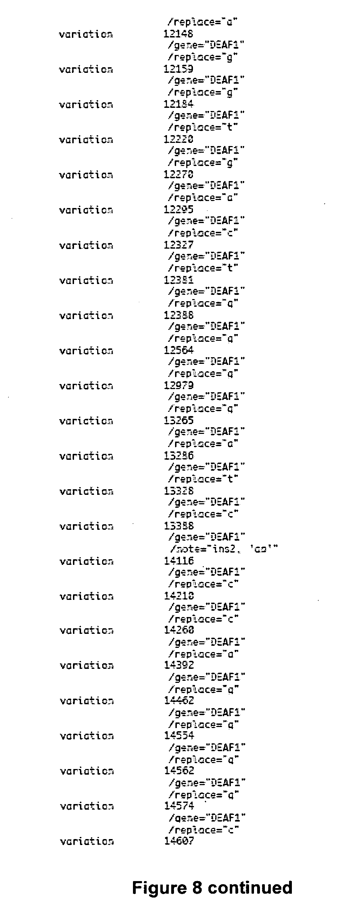

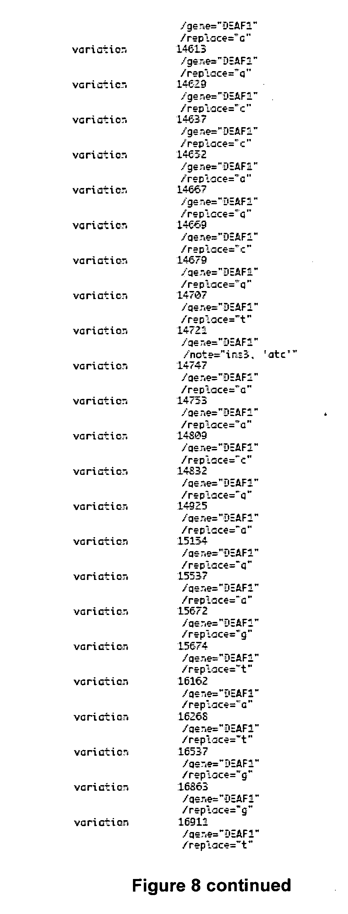

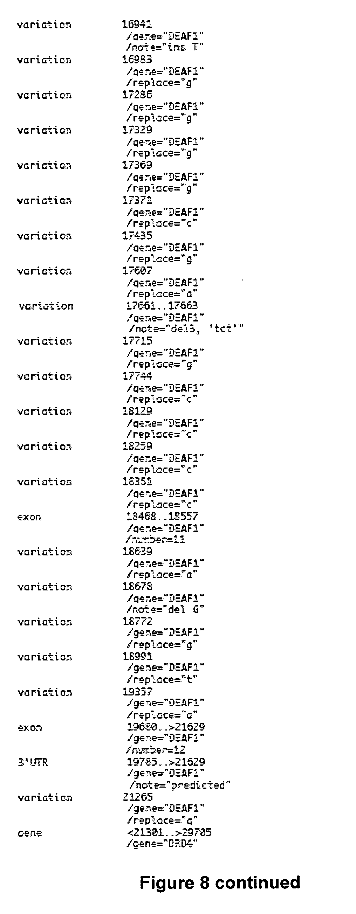

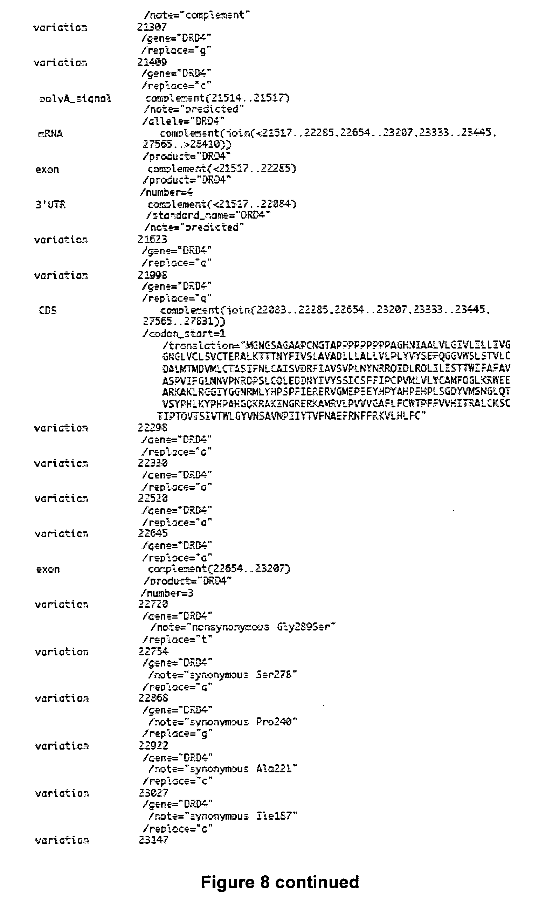

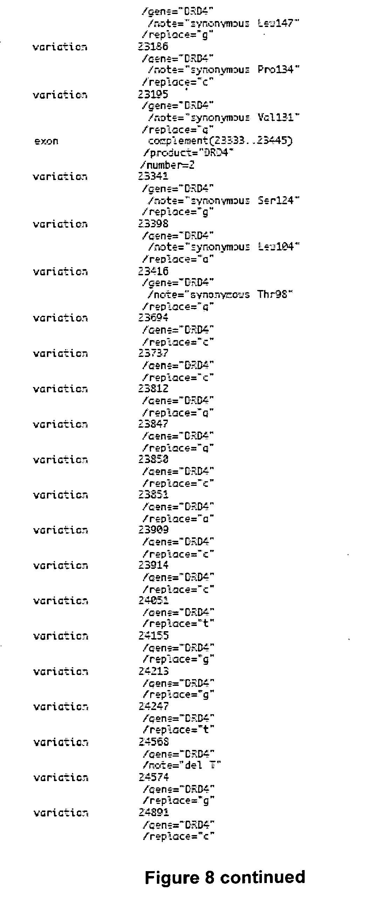

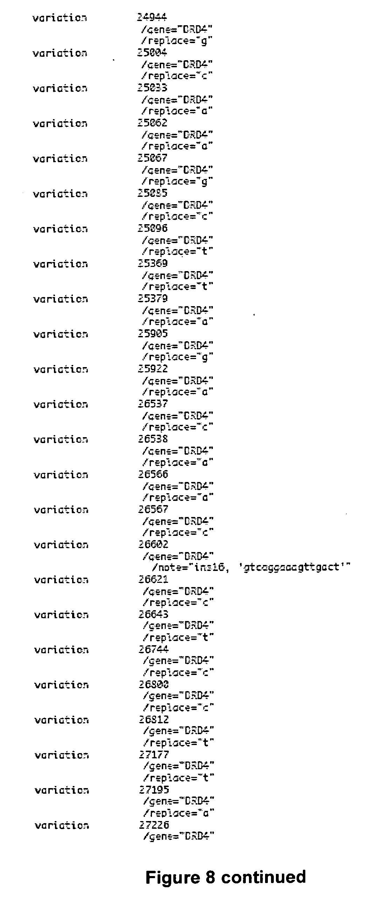

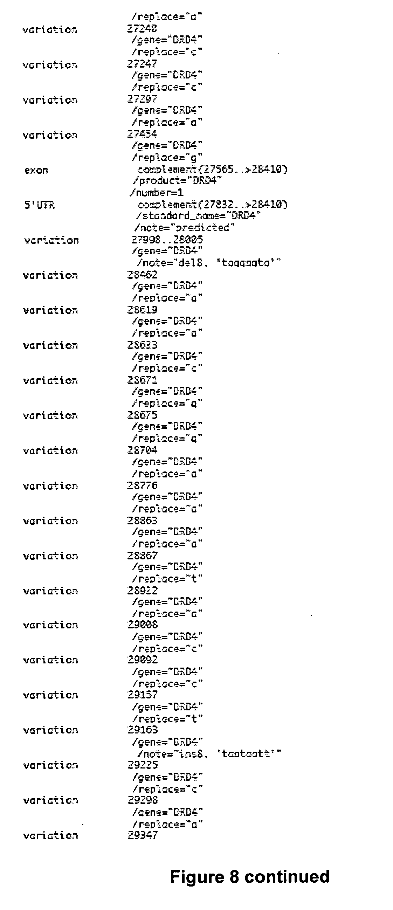

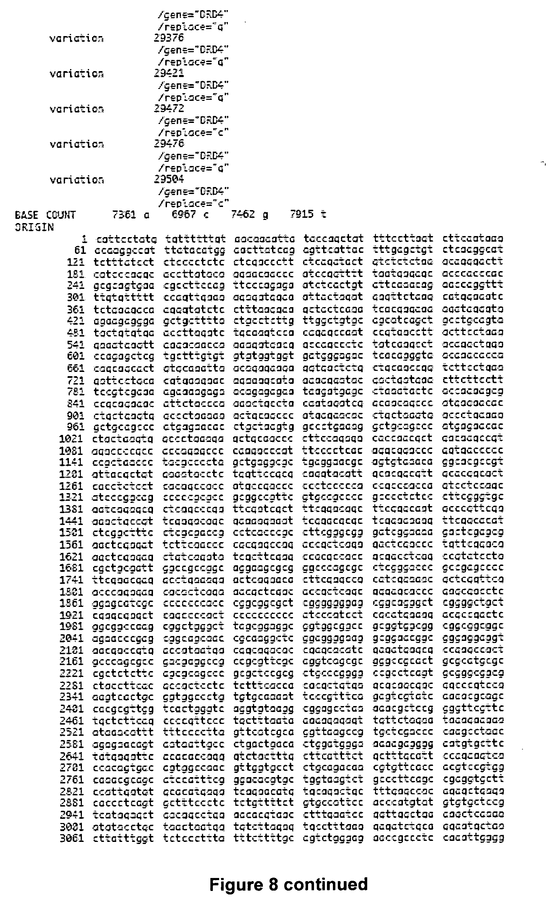

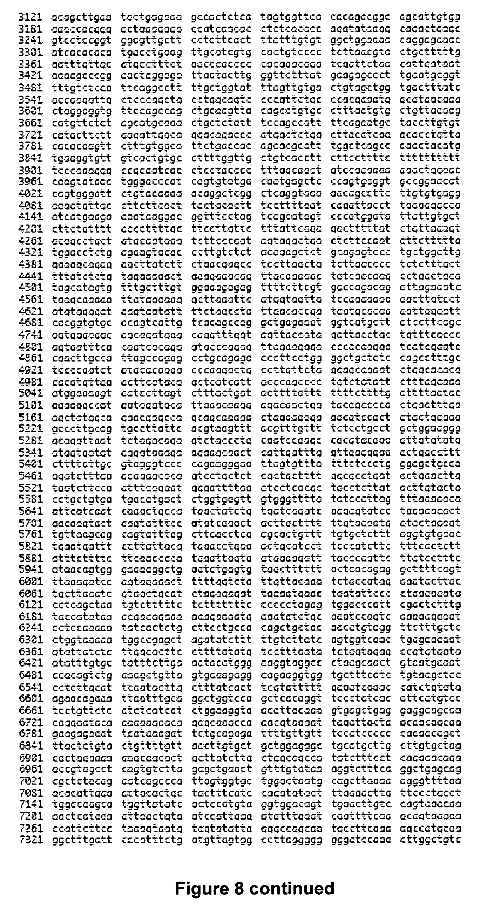

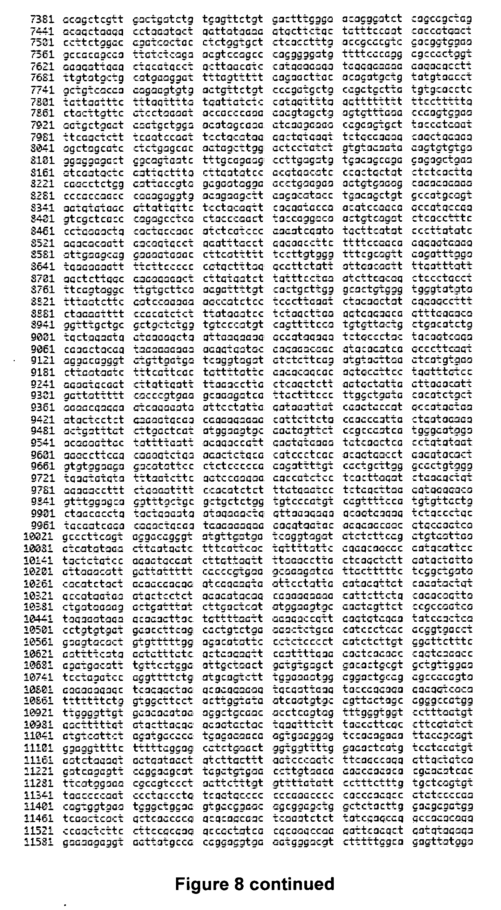

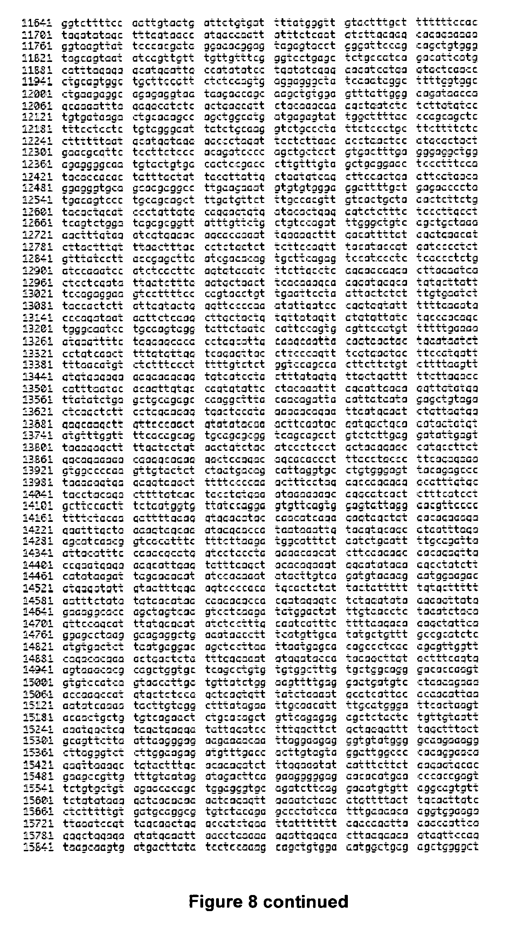

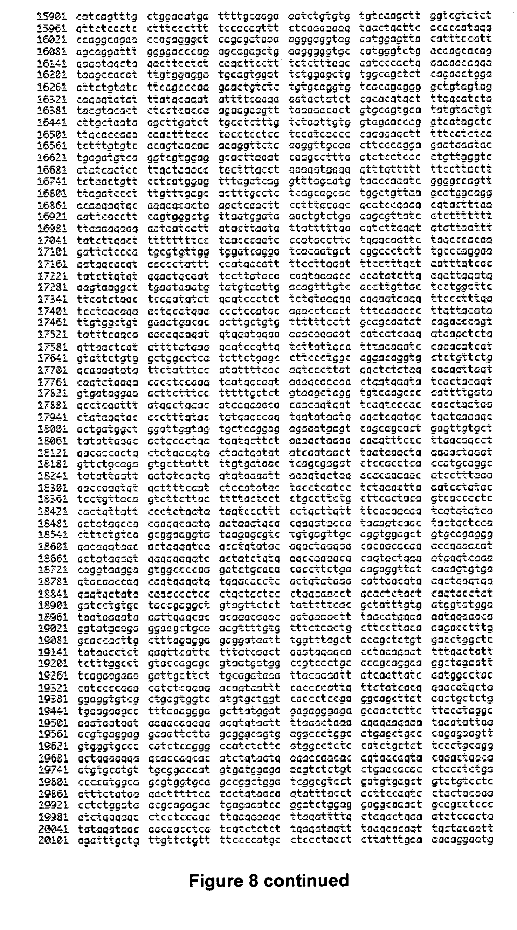

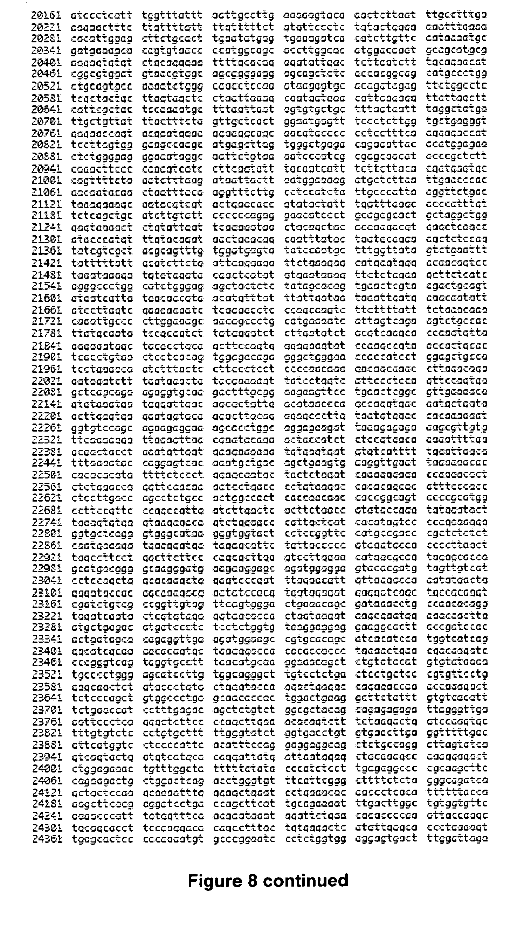

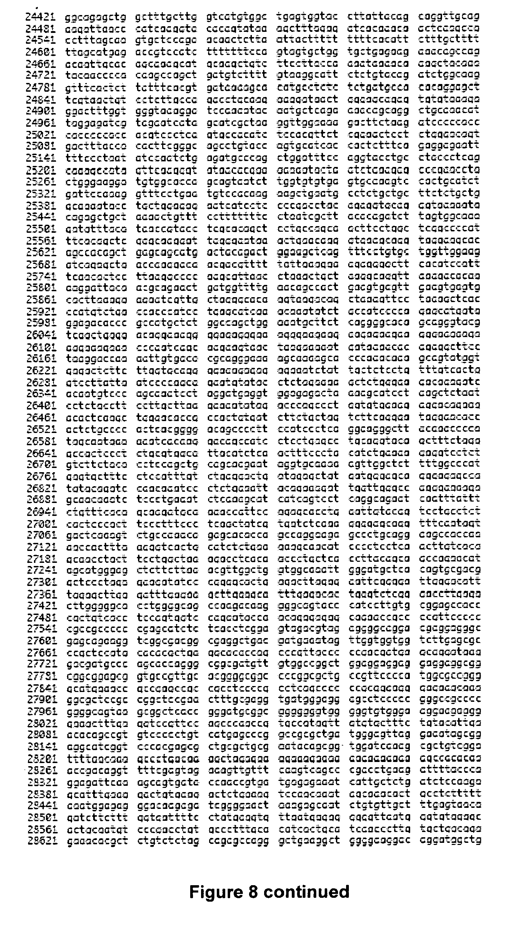

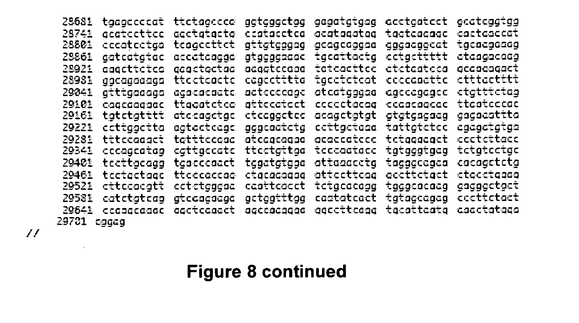

**Figure 3****.** Association of *DRD4* - haplotypes with predisposition to feather pecking. (A) Haplotype frequencies, number of animals (in parentheses) in the selection (SLP, "low-pecking"; SHP, "high-pecking") and commercial lines (CLP, "low-pecking"; CHP, "high-pecking"), and P-values from Fisher's exact test are presented. Haplotypes with a frequency greater than 5% were compared with the pooled frequencies of the remaining haplotypes. Association is accounted for by the same sub-haplotypes in the selection and commercial lines. These sub-haplotypes are shaded in dark grey if overrepresented in the "low-pecking" lines and in light grey if overrepresented in "high-pecking" lines. The alleles discriminating the "low-pecking" sub-haplotype from the "high-pecking" sub-haplotype are in uppercase letters; i and d designate an insertion or deletion, respectively. Haplotype sequences are reverse-complementary relative to the sequence of Figure 8. (B) Relative frequencies of sub-haplotypes in different lines. Columns in light shading indicate frequencies of the "low-pecking" sub-haplotype, columns in dark shading the "high-pecking" sub-haplotype. P-values resulting from Fisher's exact test are 7.30 x 10⁻⁷ for SLP compared with SHP and 2.78 x 10⁻⁶ for CLP compared with CHP.

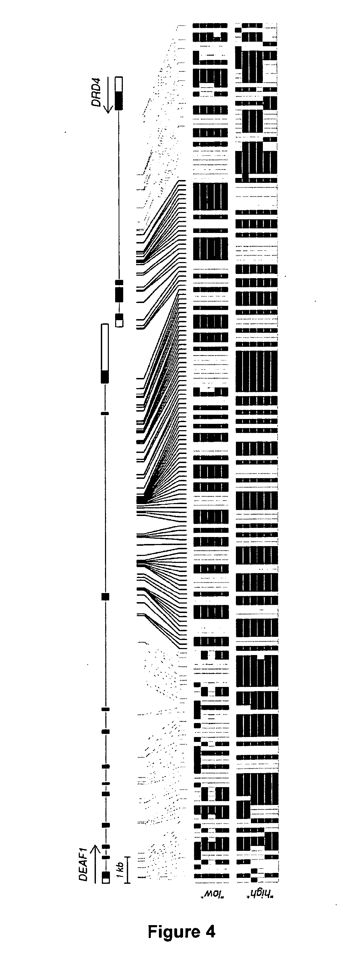

**Figure 4****.** Structure of chicken *DEAF1* and *DRD4* genes and genotype distribution at polymorphic loci. Polymorphisms (191) were identified by re-sequencing of five animals homozygous for the sub-haplotype of *DRD4* predisposing to low feather pecking ("low") and of six animals homozygous for the sub-haplotype predisposing to high feather pecking ("high"). Exons are shown as filled boxes, putative 5'- and 3'-UTRs as open boxes. Arrows indicate the orientation of the genes. Homozygosity is indicated by white and black boxes, respectively, and heterozygosity by grey boxes in the mosaic plot below the representation of the gene structure. Connecting lines indicate the position of the SNPs in *DEAF1* and *DRD4.* SNPs within a LD block as characterized by alternative homozygosity of carriers of the "low pecking" and "high pecking" *DRD4 -* sub-haplotypes, respectively, are highlighted by thicker connecting lines in black. Details of annotation of the genes and the positions of variants are indicated in Table 3 and Figure 8.

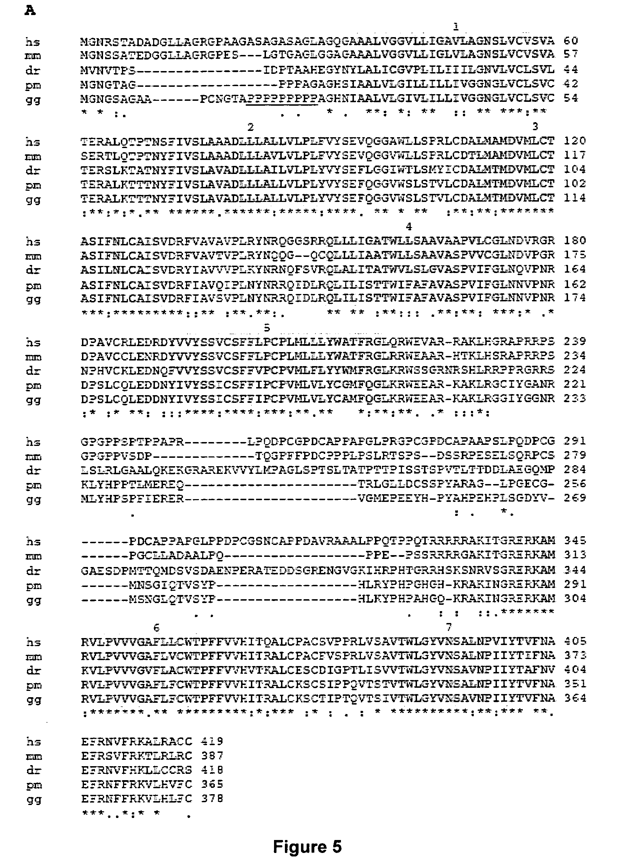

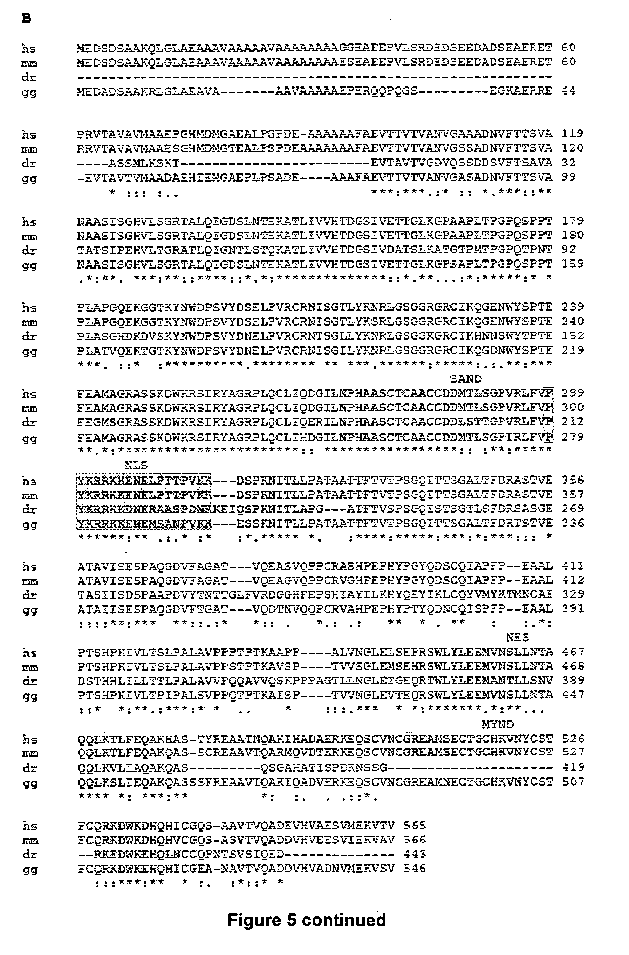

**Figure 5****:** Alignment of amino acid sequences of the chicken DRD4 (A) and DEAF1 (B) with the corresponding sequences of other species.
   The transmembrane regions of DRD4 are shaded. The proline repeat region is underlined. The main domains of DEAF1 are shaded: SAND - DNA binding domain, NLS - nuclear localization signal, NES - nuclear export signal, MYND - carboxyl-terminal zinc finger motif. DRD4 protein sequences: hs - *Homo sapiens* (GenBank accession no. NP_000788), mm *- Mus musculus* (GenBank accession no. NP_031904), dr - *Danio rerio* (GenBank accession no. NP_001012638), pm - *Parus major* (GenBank accession no. AAY56687), gg - *Gallus gallus* (GenBank accession no. XP_420947); DEAF1 protein sequences: hs - *Homo sapiens* (GenBank accession no. NP_066288), mm - *Mus musculus* (GenBank accession no. NP_058570), dr - *Danio rerio* (Ensembl: ENSDARP00000087657), gg - *Gallus gallus* (GenBank accession no. XP_420948). Prediction of seven transmembrane regions of DRD4 was done at the TMHMM Server v. 2.0 (http://www.cbs.dtu.dk/services/TMHMM). The alignment was performed using ClustalW2 (http://www.ebi.ac.uk/Tools/clustalw2). Symbols: * - identical residues, : - conserved substitutions, . - semi-conserved substitutions (for the definition of *conserved* and *semiconserved* see (http://www.ebi.ac.uk/cgi-bin/clustalw2)).
**Figure 6****:** Summary of genotype frequency analysis of *DRD4* polymorphisms. Genotype observations are in parenthesis, P-values for tests on Hardy-Weinberg-Equilibrium (HWE), P values for Fisher's exact test, comparing genotype- and allele-observations of SLP *vs*. SHP and CLP *vs* CHP, respectively. The allele symbols are reverse-complementary relative to the sequence of Figure 8. The SNP numbers refer to the corresponding entries in Table 3.

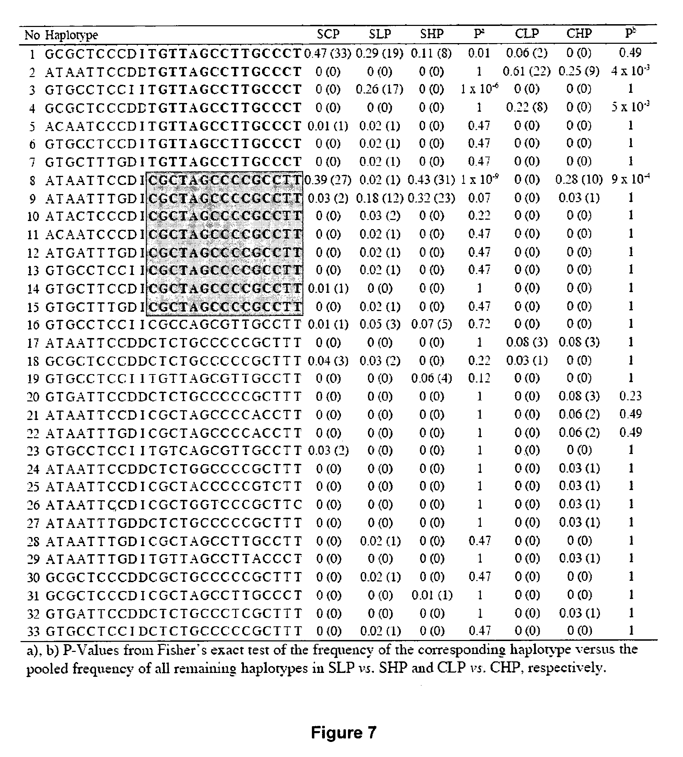

**Figure 7****:** Summary of haplotype analysis in *DRD4.* Frequencies and observations (in parentheses) of haplotypes and P-values from Fisher's exact test when contrasting haplotype frequencies of SLP *vs*. SHP and CLP *vs*. CHP. Sub-haplotypes, significantly associated with lower and higher predisposition for feather pecking are shaded in dark grey (low feather pecking) and light grey colour (high feather pecking), respectively. Haplotype sequences are reverse-complementary relative to the sequence of Figure 8.
**Figure 8****:** The annotated nucleotide sequence of chicken *DEAF1* and *DRD4.*

The examples illustrate the invention:

### Example 1. Material and Methods

### Animals and DNA preparation

We investigated hens resulting from selection for high (SHP, n = 36) and low (SLP, n = 33) feather pecking, as well as hens belonging to the unselected founding line (SCP, n = 36). Selection was carried out for eight generations and based on the number of feather pecking bouts, whereby a feather pecking bout was defined as a series of continuous pecks directed to the same recipient hen (Kjaer *et al*. 2001; Su et *al*. 2005). Hens of two commercial lines with different propensities to feather pecking were also involved in the study: commercial low pecking (CLP, n = 18) and commercial high pecking (CHP, n = 18). The selection lines and CLP originate from the White Leghorn breed and CHP is derived from the Rhode Island Red breed. Genomic DNA was prepared from whole blood by proteinase K digestion and phenolchloroform extraction. Concentration of DNA was measured and normalized to a working solution of 25 ng / µl.

### Prediction of the genomic organization of DRD4 and DEAF1

The GENOMETHREADER software tool (Gremme et al. 2005) was used to predict the genomic structure of DRD4 and DEAF1 based on the 2.1 release of the chicken draft genome sequence (Hillier et al. 2004) and the Dana-Farber Cancer Institute (DFCI) chicken gene index release 11.0 (Quackenbush et al. 2001). The GENOMETHREADER output was viewed and edited using the Apollo sequence annotation editor (Lewis et al. 2002).

### Re-Sequencing

PCR-primers (Table 2) were designed flanking all exons as well as the promoter region using PRIMER3 program (http://frodo.wi.mit.edu). The PCR (20 µl) contained 50 ng of genomic DNA, 1.5 mM MgCl₂ in 1x PCR buffer, 0.5 µM of both, forward and reverse primer, 200 µM of each nucleotide, and 0.5 U of Taq DNA polymerase (Qiagen, Hilden, Germany). The reactions were amplified under following conditions: initial denaturation at 95 C for 5 min, 30 cycles of denaturation at 95 C for 30 sec, annealing at 60 C for 1 min and elongation at 72 C for 1 min. The quality of the resulting PCR products was tested by 1% agarose gel electrophoresis. The PCR products were purified with 1 U of Exonuclase I (Fermentas, St. Leon-Rot, Germany), and 5 U of Shrimp Alkaline Phosphatase (Fermentas, St. Leon-Rot, Germany). The 10 µl of sequencing reaction mix was prepared using the forward and the reverse primer (0.5 µM), 1 µl of amplified, purified PCR-Product and BigDye Terminator v1.1 Sequencing Kit (Applied Biosystems Applera, Darmstadt, Germany). Sequencing was carried out under following conditions: initial denaturation for 15 s at 96 C, 35 cycles of 10 s at 96 C, 5 s at 51 C, and 4 min at 60 C. Thereafter, the sequencing reaction mix was purified using Multiscreen filtration plates (Millipore, Schwalbach/Ts, Germany) filled with 45 ng of Sephadex G-50 Fine (Sigma-Aldrich Chemie, Munich, Germany) and 300 µl of distilled water. Sequencing products were then mixed with 20 µl of formamide and run on an ABI 3130xI automatic sequencer (Applied Biosystems Applera, Darmstadt, Germany). The resulting chromatogram files were analyzed using the Phred / Phrap / Polyphred software suite (Nickerson et al. 1997; Ewing & Green 1998; Ewing et al. 1998) and viewed using Consed (Gordon et al. 1998).

**Table 2: Primers used for the re-sequencing of the chicken DEAF1 and DRD4.**

| **Internal_Id** | **Gene** | **Region** | **Primer Sequence** |
|---|---|---|---|
| 5864 | DRD4 | 5'END | CCCTTGGCTAGTTTGAGCTG |
| 5865 | DRD4 | 5'END | CAGCAAGAGACTGGCAGAAA |
| 5866 | DRD4 | 5'END | GGCATAAAAGGCTGGAGTGA |
| 5867 | DRD4 | 5'END | TGCTCTGATCTCCAAGAGCAT |
| 5678 | DRD4 | 5'END | CGCTATTTCCTAACTCCCATCA |
| 5679 | DRD4 | 5'END | GGGAGGGAGAAGCGAAGG |
| 5289 | DRD4 | 5'UTR | CGATCTCTTGTCCCTCTCCA |
| 5290 | DRD4 | 5'UTR | AGTTGGTGGTGGTCTTGAGC |
| 5482 | DRD4 | EX1 | CCCGTGCAACGGCACCGC |
| 5483 | DRD4 | EX1 | AGAGGGGCAGGACGAGGAG |
| 5868 | DRD4 | INT1 | GACCACTGGAGGTGACAGC |
| 5869 | DRD4 | INT1 | ATTGCAGGAAGATTGGTTGG |
| 5870 | DRD4 | INT1 | TTGATTCTGCCTGAGGACTG |
| 5871 | DRD4 | INT1 | GTCCTTATTGATCCCCAGCA |
| 5872 | DRD4 | INT1 | TCCTAGGATTTGCTGGGACA |
| 5873 | DRD4 | INT1 | CCTTCCCGGTTCAAATACTG |
| 5874 | DRD4 | INT1 | CTTCAGTGGCTGTTTGAAGC |
| 5875 | DRD4 | INT1 | CCTCTCTCTGATGCCACACA |
| 5876 | DRD4 | INT1 | CTCTGCTCCCAGTCATCCTC |
| 5877 | DRD4 | INT1 | CTTGGCTGTGGTGTTCAAAA |
| 5878 | DRD4 | INT1 | AACTTTCAGGGTGCCCAAC |
| 5879 | DRD4 | INT1 | TGAACCATCCTTTGAGACAGC |
| 5880 | DRD4 | INT1 | GCTTCAAGCTGGGGAAGAG |
| 5881 | DRD4 | INT1 | TGGGACTGAAACAGCGATAA |
| 5291 | DRD4 | EX2 | CCCAGGGGCATTTTATACAC |
| 5292 | DRD4 | EX2 | AACACAGGTGGGTCAGTGCT |
| 5293 | DRD4 | EX3 | AGCATCAAGCTGCTTCCATT |
| 5294 | DRD4 | EX3 | GGAGCGATGCTACTCTGGAT |
| 5882 | DRD4 | INT3 | GGTGGTTGTTGGTGAGTGG |
| 5883 | DRD4 | INT3 | CGTTGTGTTCAAAAGGGTTG |
| 5295 | DRD4 | EX4 | TTTTTGTCACGGAGAGATGG |
| 5296 | DRD4 | EX4 | GAGACAGAGGGCTGGGAAC |
| 5884 | DRD4 | 3'UTR | GAGATGGTAGCTCTGCATTCG |
| 5885 | DRD4 | 3'UTR | ATCTCAAGACCTCCCAGCAA |
| | | | |
| 5968 | DEAF1 | 5'END | CCAGCTGTTTTCCTTAGTCTTCA |
| 5969 | DEAF1 | 5'END | CCTCTTCCCTGTTAATCTGCAC |
| 5970 | DEAF1 | 5'UTR | TTGTGTGTGTGGTGGTGATG |
| 5971 | DEAF1 | 5'UTR | GCCGTGAGGAGAGGTGAG |
| 5936 | DEAF1 | EX1 | TACGCTGTGAAATGCCTCTG |
| 5937 | DEAF1 | EX1 | TACACCTGACCCAGTGACCA |
| 5938 | DEAF1 | EX2 | GACCCCAAGCCTAACAGAGA |
| 5939 | DEAF1 | EX3 | TCTCCTAGTTCCGGGCTTTT |
| 5940 | DEAF1 | EX4 | TTCAGAATGCTACCTTGTGTCA |
| 5941 | DEAF1 | EX4 | GGAAGACTCACTGCACAGCA |
| 5978 | DEAF1 | INT4 | GGACGGTTTCCTAGTCAGCA |
| 5979 | DEAF1 | INT4 | TCCTTGTGGGTCCCTTCTAA |
| 5942 | DEAF1 | EX5 | AGGAGCAGAATCTGGCACAC |
| 5943 | DEAF1 | EX6 | CCCACAACTCACCAGAGTCA |
| 5944 | DEAF1 | EX7 | GCTGCATCTAGAGAGGTTGGA |
| 5945 | DEAF1 | EX7 | AAAAACACGAAGTGACAAAGCA |
| 5946 | DEAF1 | EX8 | AGGGCTTTGATTCCCATTTC |
| 5947 | DEAF1 | EX8 | TAAGCATCTGCAGCATCTGG |
| 5948 | DEAF1 | EX9 | TGCAGAAGCCTTGAGATGTG |
| 5949 | DEAF1 | EX9 | CAAGAGCTTCTTCTGCCAAG |
| 5972 | DEAF1 | INT 9 | TGTGGGTTTCGCAGTTAAGA |
| 5973 | DEAF1 | INT 9 | TGCTTCACGGGTGAAAAATA |
| 5974 | DEAF1 | INT 9 | TTCACCCGTGAAGCAAAGAT |
| 5975 | DEAF1 | INT 9 | CTGGACCAGCCTGCAAATTA |
| 5980 | DEAF1 | INT 9 | TGTCCCATGTCAGTTTTCCA |
| 5981 | DEAF1 | INT 9 | TGCTGAAGGTTCATCACACAG |
| 5982 | DEAF1 | INT 9 | GCAAATAGTTCTCCGCCAAT |
| 5983 | DEAF1 | INT 9 | CCACCAGTTCAGATGCTCCT |
| 5924 | DEAF1 | EX10 | GGAGCATCTGAACTGGTGGT |
| 5925 | DEAF1 | EX10 | ACCCACAGCTGCTGGAATC |
| 5976 | DEAF1 | INT 10 | TTCCACTGGGTATAGCTTTGG |
| 5977 | DEAF1 | INT 10 | CATGGAAAGGGAGTTCCTCA |
| 5996 | DEAF1 | INT10 | CCTCTTAACACCTAACTCCATGC |
| 5997 | DEAF1 | INT10 | GATCAATACCGAGGAGTGATTTT |
| 5998 | DEAF1 | INT10 | CAGAGTCCATCCCTCTCACC |
| 5999 | DEAF1 | INT10 | TGGCTCTGCAGCTCAGATATAA |
| 6000 | DEAF1 | INT10 | CAGTTGTGCCAGTGTGTTCC |
| 6001 | DEAF1 | INT10 | AGCAAATCCTCTTCCTGTCA |
| 6002 | DEAF1 | INT10 | CACTCTTTCATCCTGCTTCCA |
| 6003 | DEAF1 | INT10 | GGCAAACAGCATATGCAACA |
| 6004 | DEAF1 | INT10 | GCTGTTCAGGAGCCTAAGGA |
| 6005 | DEAF1 | INT10 | TTCATGTGTTCTCCCCCTTC |
| 6006 | DEAF1 | INT10 | TAGTAGGATTGGCCCCAAAG |
| 6007 | DEAF1 | INT10 | GGTCCCCAAATCCTGCTAAT |
| 6008 | DEAF1 | INT10 | GGCTCAGAGATAAAAGGGAGGT |
| 6009 | DEAF1 | INT10 | CCTGATCTAAACTCCATGAGGAA |
| 6010 | DEAF1 | INT10 | CTCCTCACCTGTTGGGTCAT |
| 6011 | DEAF1 | INT10 | CCTCCCTTACAGAAGAGGGACT |
| 6012 | DEAF1 | INT10 | TGAAGCCACATGTCTTGAGATT |
| 6013 | DEAF1 | INT10 | ATCACACCACTGGCTCACAG |
| 6014 | DEAF1 | INT10 | TCAAGCCCCATTTTGATAGC |
| 6015 | DEAF1 | INT10 | AACAACAGTGGAGGGGTGAC |
| 5926 | DEAF1 | EX11 | CAGGTGTGATTTTCAATCTCCA |
| 5927 | DEAF1 | EX11 | CAGCTCACGCCAATGTTTAC |
| 6016 | DEAF1 | INT11 | GTAAGGAGTGGCCCCAAGAT |
| 6017 | DEAF1 | INT11 | TTGCTCTCCCTCTCATCCAT |
| 5928 | DEAF1 | EX12 | CTGAGTGGTCATGTGCTGGT |
| 5929 | DEAF1 | EX12 | CTGGAGGAGGCTCCCAGAC |
| 5930 | DEAF1 | 3'UTR | TCCTACTGCAAACCTCTGGA |
| 5931 | DEAF1 | 3'UTR | ATGCTGCCGTGGTGAGAG |
| 5932 | DEAF1 | 3'UTR | TGTGTCTGCAGGGAGTTTTG |
| 5933 | DEAF1 | 3'UTR | GGCAATAGATGGAGGCAAGA |
| 5934 | DEAF1 | 3'UTR | CACGATGAGTGCCAGTTTTC |
| 5935 | DEAF1 | 3'UTR | TGCTGGGAGGTCTTGAGATT |

### Statistical analysis

The open source language and environment for statistical computing R was used for statistical analysis extended by the package GENETICS, v1.2.0 (www.cran.r-project.org). Fisher's exact test was applied to test for deviations from Hardy-Weinberg equilibrium. Genotype- and allele observations between two lines were compared via contingency tables and tested by Fisher's exact test. For 2 x 3 (genotype) tables, P-values were obtained by 10⁶ Monte Carlo simulations. Haplotypes were inferred within each population using FASTPHASE (v1.2) (Scheet & Stephens 2006). The frequency of each haplotype was compared with the pooled frequency of the remaining haplotypes in two lines using 2 x 2 contingency tables and tested by Fisher's exact test.

The probability that an observed difference in SNP allele frequencies in the selection lines is caused by random genetic drift was assessed by Monte Carlo simulation: Two populations were simulated, each consisting of 10 males and 30 females in accordance with the actual selection experiment (Kjaer et al. 2001; Su et al. 2005). Starting allele frequencies were 0.5 in both populations and sexes. The simulated individuals were randomly mated for eight generations. For each of 10⁵ random repetitions, the observed deviation in the allelic frequencies of the populations in generation 8 was recorded and used to calculate probabilities that the observed allelic difference between lines was caused by genetic drift.

### Example 2: Annotation of DRD4

A BLAST search of the chicken draft sequence with the human DRD4 RefSeq (GenBank accession no. NM_000797) indicated up to 89% identity in an interval spanning bases 524,469 to 529,322 of chicken chromosome 5. 50,000 bases centred to this interval were subject to EST-based gene prediction using the GENOMETHREADER software. The derived amino acid sequence of a gene predicted in the centre of the analyzed genomic interval was 99% identical with the predicted sequence of Gallus gallus DRD4 (GenBank accession no. XP_420947) and 85% identical with the Parus major DRD4 amino acid sequence (GenBank accession no. AAY56686) and moderately identical to the DRD4 amino acid sequences of a number of other vertebrate species, among them Homo sapiens ((GenBank accession no. NP_000788), 55% identical). The predicted gene is structured into 4 exons. Comparison of the derived amino acid sequence with the DRD4 sequence of other species revealed a gap in the draft sequence corresponding to the 5'-part of the first exon. Part of the missing genomic sequence has been deposited under GenBank accession nos. (AB125363-AB125364). We obtained the entire missing sequence after PCR with primers flanking the gap and sequencing of the resulting PCR product. A possible transcription start was computationally predicted (http://www.fruitf!y.org/seq_toots/promoter.html) with a promoter score = 1 at position - 579 of the translation start. Based on the mouse polyadenylation signal database, polyadq (http://rulai.cshl.org/tools/polyadq/polyadq_form.html), (Tabaska & Zhang 1999) predicts a polyadenylation signal at bases 566 to 572 after the stop codon. Prediction of transmembrane helices in the derived chicken protein (http://www.cbs.dtu.dk/services/TMHMM/) indicates a structure of seven transmembrane domains that is typical for dopamine receptors (Missale et al. 1998). The alignment of the chicken amino acid sequence with the corresponding sequences of other vertebrate species indicates a high degree of conservation of the transmembrane domains and little conservation of the 3^{rd} intracellular loop (Fig. 5 A).

### Example 3: Polymorphism and association analysis of DRD4

To detect sequence polymorphisms, part of the promoter and the four exons including flanking segments were re-sequenced in hens selected for high (SHP) and low (SLP) feather pecking, in hens belonging to the founder line (SCP) and in hens of two commercial lines with different propensities to feather pecking (CHP, CLP). In total, 3244 basepairs were re-sequenced in 140 animals resulting in the detection of 25 variants, specifically, a non-synonymous SNP leading to the exchange of a glycine by a serine, eight SNPs in the putative promoter, 12 synonymous and two intronic SNPs, one SNP in the 3'-UTR as well as two insertion / deletion polymorphisms in the putative promoter and in the 5'-UTR, respectively. Genotype and allele frequencies of several polymorphisms differ significantly between both selected lines, i.e., SHP vs. SLP, and also between animals of the commercial lines, i.e., CHP vs. CLP (Fig. 1, Figure 6). However, allelic differences between selection lines don't necessarily imply association. Such differences could actually result from genetic drift due to the small number of selected chickens in each generation. However, simulations indicate, that a difference of 0.47 between the SHP and SLP line, as observed for one of the significantly associated SNPs, has a probability of 0.16 to result from genetic drift. Furthermore, the highly significant allelic differences between the two selection lines parallel those observed in commercial lines, i.e., an allele overrepresented in SHP is also overrepresented in the CHP and the corresponding alternative allele is underrepresented in SLP and CLP (Figure 6). Concordant allelic differences in genetically distant populations (see description of animals in Material and Methods) are unlikely to be population genetic artefacts. Response to selection for high and low feather pecking is not symmetrical (Kjaer et al. 2001; Su et al. 2005). This seems to be reflected by the extent of the allelic difference between the founding line and the high and low pecking line, respectively, another indication for association of DRD4 variation and feather pecking (Fig. 2). The significantly associated variants are either synonymous or intronic SNPs. A polymorphism in the putative promoter involving the insertion / deletion of 8 basepairs is associated in the commercial lines only. SNPs that are significantly associated in the selection as well as in the commercial lines cluster in 3'- half of the gene, indicating a linkage disequilibrium (LD) block to span this region of DRD4 (Fig. 1).

### Example 4: Haplotype analysis of DRD4

To obtain detailed information on the linkage disequilibrium structure, haplotypes were derived (Figure 7). Association analysis showed two haplotypes to be significantly overrepresented in the selection high pecking line (SHP) as well as two haplotypes in the commercial high pecking line (CHP). Two haplotypes are overrepresented in the selection low pecking line (SLP) and one haplotype in the commercial low pecking line (CLP). Comparison of associated haplotypes reveals that association with feather pecking is accounted for by the same two sub-haplotypes in the selection as well as in the commercial lines (Fig. 3A). Frequencies of sub-haplotypes differ significantly between SLP and SHP (P = 7.30 × 10⁻⁷) and between CLP and CHP (P = 2.78 × 10⁻⁶) (Fig. 3B). The associated sub-haplotypes are delineated by an SNP in exon 2 of DRD4 and the 3'-UTR of the gene. Thus, causal variation may potentially be confined to this region of the gene or be located in sequence downstream of DRD4.

### Example 5: Annotation and re-sequencing of DEAF1

We extended the re-sequencing and polymorphism analysis to a not yet closed gap in the draft sequence in intron 1 of DRD4 and to the sequence downstream of DRD4. We first annotated the genomic draft sequence 3' to DRD4 based on the GENOMETHREADER output obtained in the course of annotating DRD4 (see above). This revealed a gene transcribed from the opposite strand. The derived amino acid sequence is 100% identical with the predicted sequence of chicken deformed epidermal autoregulatory factor 1 (DEAF1, (GenBank accession no. XP_420948)) and 80% identical with the sequence of human DEAF1 (GenBank accession no. NP_066288). DEAF1 is involved in the regulation of the gene encoding serotonin (5-hydroxytryptamine, 5-HT) 1A receptor, a key component of the serotonergic system (Huggenvik et al. 1998; Lemonde et al. 2003; Albert & Lemonde 2004; Czesak et al. 2006). Since both the dopaminergic and the serotonergic systems have been shown to be involved in chicken behaviour, DEAF1 may be considered a functional candidate gene for feather pecking. DEAF1 is structured into 12 exons. Alignment with an EST locates a putative transcription start at position -62 bp of the translation start. Polyadenylation signal prediction was done as shown above for DRD4 and locates a putative site at bases 1845 to 1851 after the stop codon. According to this annotation, the 3'-UTRs of DEAF1 and DRD4 overlap for 112 bases (Fig. 8). The alignment of the derived DEAF1 amino acid sequence with the corresponding sequences of other vertebrate species indicates a high degree of conservation of motifs characteristic for transcription factors, i.e., the DNA binding domain SAND, and a carboxyl-terminal zinc finger motif called the MYND domain (Fig. 5 B) (Jensik et al. 2004). A conserved nuclear export signal (NES) sequence can also be identified (Fig. 5 B) (Jensik et al. 2004).

We chose five animals homozygous for the sub-haplotype associated with low propensity to feather pecking and six animals homozygous for the alternative sub-haplotype for re-sequencing of the not yet analyzed part of intron 1 of DRD4 and the entire DEAF1. Re-sequencing reveals a LD block of 14,833 bases as manifested by alternative homozygosity at 106 of 107 polymorphic sites in the animals carrying the "low-pecking" sub-haplotype of DRD4 and in the animals carrying the "high-pecking" sub-haplotype (Fig. 4, Table 3). The exceptional SNP could be the result of a recent mutation. The LD block delimits the associated haplotypes within intron 1 of DRD4 and intron 9 of DEAF1.

**Table 3: Characterization of DEAF1 and DRD4 polymorphisms.**

| **no** | **snp_ id** | **gene** | **type** | **"high-pecking"** | **localization** | **position** | **comments** | **part of LD Block (NO/ YES)** |
|---|---|---|---|---|---|---|---|---|
| 1 | 2314 | DEAF1 | C>T | | 5'END | 197 | | NO |
| 2 | 2315 | | C>T | | 5'END | 214 | | NO |
| 3 | 2316 | | A>G | | 5'END | 241 | | NO |
| 4 | 2317 | | A>G | | 5'END | 243 | | NO |
| 5 | 2318 | | A>G | | 5'END | 406 | | NO |
| 6 | 2319 | | A>G | | 5'END | 593 | | NO |
| 7 | 2320 | | C>T | | 5'END | 613 | | NO |
| 8 | 2361 | | G>T | | 5'END | 718 | | NO |
| 9 | 2362 | | C>T | | 5'END | 749 | | NO |
| 10 | 2363 | | C>T | | 5'END | 839 | | NO |
| 11 | 2364 | | C>T | | 5'END | 864 | | NO |
| 12 | 2366 | | A>G | | 5'END | 946 | | NO |
| 13 | 2337 | | . A>G | | EXON 1 | 1154 | syn Ala74 | NO |
| 14 | 2338 | | C>T | | INTRON 1 | 2185 | | NO |
| 15 | 2339 | | C>G | | INTRON 1 | 2256 | | NO |
| 16 | 2340 | | C>G | | INTRON 1 | 2298 | | NO |
| 17 | 2482 | | T>C | | INTRON 1 | 2299 | | NO |
| 18 | 2341 | | A>G | | INTRON 1 | 2320 | | NO |
| 19 | 2342 | | A>G | | INTRON 1 | 2346 | | NO |
| 20 | 2283 | | A>G | | INTRON 2 | 2848 | | NO |
| 21 | 2284 | | C>T | | INTRON 2 | 3072 | | NO |
| 22 | 2285 | | C>T | | INTRON 2 | 3087 | | NO |
| 23 | 2286 | | A>T | | INTRON 3 | 3234 | | NO |
| 24 | 2287 | | A>G | | INTRON 3 | 3771 | | NO |
| 25 | 2288 | | G>T | | INTRON 3 | 3802 | | NO |
| 26 | 2289 | | delC | | INTRON 3 | 3890 | | NO |
| 27 | 2290 | | C>T | | EXON 4 | 3994 | syn | NO |
| | | | | | | | Asn181 | |
| 28 | 2291 | | A>G | | EXON 4 | 4045 | syn Arg198 | NO |
| 29 | 2321 | | C>T | | INTRON 4 | 4293 | | NO |
| 30 | 2322 | | A>C | | INTRON 4 | 4410 | | NO |
| 31 | 2323 | | C>T | | INTRON 4 | 4538 | | NO |
| 32 | 2324 | | C>T | | INTRON 4 | 4730 | | NO |
| 33 | 2325 | | C>T | | INTRON 4 | 4811 | | NO |
| 34 | 2326 | | A>G | | INTRON 4 | 4813 | | NO |
| 35 | 2269 | | T>C | | INTRON 4 | 5070 | | NO |
| 36 | 2483 | | A>G | | INTRON 4 | 5071 | | NO |
| 37 | 2270 | | C>T | | INTRON 4 | 5073 | | NO |
| 38 | 2271 | | C>T | | INTRON 5 | 5417 | | NO |
| 39 | 2272 | | G>T | | INTRON 7 | 6422 | | NO |
| 40 | 2273 | | C>T | | INTRON 7 | 6466 | | NO |
| 41 | 2274 | | C>T | | INTRON 8 | 7651 | | NO |
| 42 | 2275 | | C>T | | INTRON 8 | 7768 | | NO |
| 43 | 2279 | | C>T | | INTRON 8 | 8237 | | NO |
| 44 | 2280 | | C>T | | INTRON 8 | 8332 | | NO |
| 45 | 2281 | | C>T | | INTRON 9 | 8515 | | NO |
| 46 | 2282 | | A>G | | INTRON 9 | 8543 | | NO |
| 47 | 2343 | | C>T | | INTRON 9 | 8997 | | NO |
| 48 | 2344 | | A>G | | INTRON 9 | 9222 | | NO |
| 49 | 2345 | | C>G | | INTRON 9 | 9233 | | NO |
| 50 | 2346 | | C>T | | INTRON 9 | 9235 | | NO |
| 51 | 2347 | | C>T | | INTRON 9 | 10183 | | NO |
| 52 | 2348 | | T>C | | INTRON 9 | 10248 | | NO |
| 53 | 2484 | | T>C | | INTRON 9 | 10249 | | NO |
| 54 | 2349 | | C>T | T | INTRON 10 | 10252 | | YES |
| 55 | 2350 | | C>T | C | INTRON 9 | 10318 | | YES |
| 56 | 2351 | | A>G | A | INTRON 9 | 10324 | | YES |
| 57 | 2352 | | A>C | C | INTRON 9 | 10747 | | YES |
| 58 | 2353 | | A>C | C | INTRON 9 | 11041 | | YES |
| 59 | 2231 | | C>T | T | INTRON 9 | 11238 | | YES |
| 60 | 2232 | | A>G | G | INTRON 9 | 11244 | | YES |
| 61 | 2233 | | A>G | A | INTRON 9 | 11255 | | YES |
| 62 | 2234 | | A>C | A | INTRON 9 | 11271 | | YES |
| 63 | 2235 | | C>T | C | INTRON 9 | 11315 | | YES |
| 64 | 2236 | | A>G | G | INTRON 9 | 11617 | | YES |
| 65 | 2237 | | G>T | T | INTRON 10 | 11723 | | YES |
| 66 | 2327 | | A>T | A | INTRON 10 | 11848 | | YES |
| 67 | 2328 | | A>G | G | INTRON 10 | 12148 | | YES |
| 68 | 2329 | | A>G | G | INTRON 10 | 12159 | | YES |
| 69 | 2330 | | C>T | T | INTRON 10 | 12184 | | YES |
| 70 | 2331 | | A>G | G | INTRON 10 | 12220 | | YES |
| 71 | 2332 | | A>T | A | INTRON 10 | 12270 | | YES |
| 72 | 2333 | | C>G | C | INTRON 10 | 12295 | | YES |
| 73 | 2334 | | C>T | T | INTRON 10 | 12327 | | YES |
| 74 | 2335 | | C>G | G | INTRON 10 | 12381 | | YES |
| 75 | 2336 | | A>G | G | INTRON 10 | 12388 | | YES |
| 76 | 2371 | | A>G | G | INTRON 10 | 12564 | | YES |
| 77 | 2372 | | G>T | G | INTRON 10 | 12979 | | YES |
| 78 | 2373 | | A>G | A | INTRON 10 | 13265 | | YES |
| 79 | 2374 | | C>T | T | INTRON 10 | 13286 | | YES |
| 80 | 2375 | | C>G | | C INTRON 10 | 13328 | | YES |
| 81 | 2410 | | insA | INS | INTRON 10 | 13388 | | YES |
| | | | A | | | | | |
| 82 | 2385 | | C>T | C | INTRON 10 | 14116 | | YES |
| 83 | 2386 | | C>T | C | INTRON 10 | 14210 | | YES |
| 84 | 2387 | | A>G | A | INTRON 10 | 14260 | | YES |
| 85 | 2388 | | A>G | G | INTRON 10 | 14392 | | YES |
| 86 | 2389 | | A>G | G | INTRON 10 | 14462 | | YES |
| 87 | 2390 | | A>G | G | INTRON 10 | 14554 | | YES |
| 88 | 2391 | | A>G | G | INTRON 10 | 14562 | | YES |
| 89 | 2392 | | C>G | C | INTRON 10 | 14574 | | YES |
| 90 | 2393 | | A>G | A | INTRON 10 | 14607 | | YES |
| 91 | 2394 | | A>G | G | INTRON 10 | 14613 | | YES |
| 92 | 2395 | | C>T | | C INTRON 10 | 14629 | | YES |
| 93 | 2396 | | C>T | C | INTRON 10 | 14637 | | YES |
| 94 | 2397 | | A>G | A | INTRON 10 | 14652 | | YES |
| 95 | 2398 | | A>G | G | INTRON 10 | 14667 | | YES |
| 96 | 2399 | | C>G | | C INTRON 10 | 14669 | | YES |
| 97 | 2400 | | A>G | G | INTRON 10 | 14679 | | YES |
| 98 | 2401 | | G>T | T | INTRON 10 | 14707 | | YES |
| 99 | 2402 | | delA | INS | INTRON 10 | 14721 | | YES |
| | | | TC | | | | | |
| 100 | 2403 | | A>G | A | INTRON 10 | 14747 | | YES |
| 101 | 2404 | | A>G | A | INTRON 10 | 14753 | | YES |
| 102 | 2378 | | C>T | C | INTRON 10 | 14809 | | YES |
| 103 | 2379 | | A>G | G | INTRON 10 | 14832 | | YES |
| 104 | 2380 | | A>G | A | INTRON 10 | 14925 | | YES |
| 105 | 2381 | | C>G | G | INTRON 10 | 15154 | | YES |
| 106 | 2382 | | A>G | A | INTRON 10 | 15537 | | YES |
| 107 | 2383 | | A>G | G | INTRON 10 | 15672 | | YES |
| 108 | 2384 | | G>T | T | INTRON 10 | 15674 | | YES |
| 109 | 2376 | | A>G | A | INTRON 10 | 16162 | | YES |
| 110 | 2406 | | A>T | | INTRON 10 | 16268 | | NO |
| 111 | 2377 | | A>G | G | INTRON 10 | 16537 | | YES |
| 112 | 2411 | | A>G | G | INTRON 10 | 16863 | | YES |
| 113 | 2412 | | C>T | T | INTRON 10 | 16911 | | YES |
| 114 | 2413 | | insT | INS | INTRON 10 | 16941 | | YES |
| 115 | 2414 | | A>G | G | INTRON 10 | 16983 | | YES |
| 116 | 2415 | | A>G | G | INTRON 10 | 17286 | | YES |
| 117 | 2416 | | A>G | G | INTRON 10 | 17329 | | YES |
| 118 | 2417 | | C>G | G | INTRON 10 | 17369 | | YES |
| 119 | 2418 | | C>T | C | INTRON 10 | 17371 | | YES |
| 120 | 2419 | | C>G | G | INTRON 10 | 17435 | | YES |
| 121 | 2420 | | A>G | A | INTRON 10 | 17607 | | YES |
| 122 | 2421 | | delT | DEL | INTRON 10 | 17661-63 | | YES |
| | | | CT | | | | | |
| 123 | 2422 | | A>G | G | INTRON 10 | 17715 | | YES |
| 124 | 2423 | | C>T | | C INTRON 10 | 17744 | | YES |
| 125 | 2407 | | C>T | C | INTRON 10 | 18129 | | YES |
| 126 | 2408 | | C>T | | C INTRON 10 | 18259 | | YES |
| 127 | 2409 | | A>C | C | INTRON 10 | 18351 | | YES |
| 128 | 2276 | | A>G | A | INTRON 10 | 18639 | | YES |
| 129 | 2277 | | delG | DEL | INTRON 11 | 18678 | | YES |
| 130 | 2278 | | A>G | G | INTRON 11 | 18772 | | YES |
| 131 | 2424 | | G>T | T | INTRON 11 | 18991 | | YES |
| 132 | 2425 | | A>G | A | INTRON 11 | 19357 | | YES |
| 133 | 2245 | | A>G | G | INTRON 11 | 21265 | | YES |
| 134 | 2246 | | A>G | G | 3'UTR | 21307 | | YES |
| 135 | 2247 | | C>T | C | 3'UTR | 21409 | | YES |
| 136 | 2248 | DEAF1/DRD4 | A>G | G | 3'UTR | 21623 | | YES |
| 137 | 1795 | DRD4 | A>G | | 3'UTR | 21998 | | NO |
| 138 | 1794 | | A>G | A | INTRON 3 | 22298 | | YES |
| 139 | 1793 | | A>G | | INTRON 3 | 22330 | | NO |
| 140 | 1823 | | A>G | | INTRON 3 | 22520 | | NO |
| 141 | 1822 | | C>T | | INTRON 3 | 22720 | nonsyn | NO |
| | | | | | | | Gly289Ser | |
| 142 | 1803 | | A>G | G | EXON 3 | 22754 | syn Ser278 | YES |
| 143 | 1802 | | A>G | G | EXON 3 | 22868 | syn Pro240 | YES |
| 144 | 2068 | | C>G | | EXON 3 | 22922 | syn Ala221 | NO |
| 145 | 1801 | | A>G | | EXON 3 | 23027 | syn Ile187 | NO |
| 146 | 1800 | | C>G | | EXON 3 | 23147 | syn | NO |
| | | | | | | | Leu147 | |
| 147 | 1799 | | C>T | | EXON 3 | 23186 | syn Pro134 | NO |
| 148 | 2070 | | A>G | | EXON 3 | 23195 | syn Val131 | NO |
| 149 | 1798 | | A>G | G | EXON 3 | 23341 | syn Ser124 | YES |
| 150 | 1797 | | A>C | | EXON 2 | 23398 | Syn | NO |
| | | | | | | | Leu104 | |
| 151 | 1796 | | A>G | G | EXON 2 | 23416 | syn Thr98 | YES |
| 152 | 2225 | | A>C | C | EXON 2 | 23737 | | YES |
| 153 | 2224 | | G>T | G | INTRON 1 | 23812 | | YES |
| 154 | 2223 | | A>G | G | INTRON 1 | 23847 | | YES |
| 155 | 2222 | | C>T | C | INTRON 1 | 23850 | | YES |
| 156 | 2481 | | A>G | A | INTRON 1 | 23851 | | YES |
| 157 | 2220 | | A>C | C | INTRON 1 | 23909 | | YES |
| 158 | 2219 | | C>G | C | INTRON 1 | 23914 | | YES |
| 159 | 2218 | | C>T | T | INTRON 1 | 24051 | | YES |
| 160 | 2217 | | A>G | G | INTRON 1 | 24155 | | YES |
| 161 | 2216 | | C>G | G | INTRON 1 | 24213 | | YES |
| 162 | 2215 | | C>T | T | INTRON 1 | 24247 | | YES |
| 163 | 2439 | | delT | DEL | INTRON 1 | 24568 | | YES |
| 164 | 2438 | | A>G | G | INTRON 1 | 24574 | | YES |
| 165 | 2437 | | C>T | C | INTRON 1 | 24891 | | YES |
| 166 | 2436 | | C>G | | INTRON 1 | 24944 | | NO |
| 167 | 2435 | | C>T | C | INTRON 1 | 25004 | | YES |
| 168 | 2434 | | A>G | A | INTRON 1 | 25033 | | YES |
| 169 | 2433 | | A>G | A | INTRON 1 | 25062 | | YES |
| 170 | 2432 | | G>T | G | INTRON 1 | 25067 | | YES |
| 171 | 2431 | | C>T | C | INTRON 1 | 25085 | | YES |
| 172 | 2430 | | C>T | | INTRON 1 | 25096 | | NO |
| 173 | 2429 | | G>T | | INTRON 1 | 25369 | | NO |
| 174 | 2428 | | A>T | | INTRON 1 | 25379 | | NO |
| 175 | 2427 | | A>G | | INTRON 1 | 25905 | | NO |
| 176 | 2426 | | A>C | | INTRON 1 | 25922 | | NO |
| 177 | 2485 | | C>G | | INTRON 1 | 26537 | | NO |
| 178 | 2370 | | A>G | | INTRON 1 | 26538 | | NO |
| 179 | 2486 | | A>G | | INTRON 1 | 26566 | | NO |
| 180 | 2369 | | C>G | | INTRON 1 | 26567 | | NO |
| 181 | 2368 | | ins16 | | INTRON 1 | 26602 | | NO |
| 182 | 2358 | | C>T | | INTRON 1 | 26621 | | NO |
| 183 | 2367 | | C>T | | INTRON 1 | 26643 | | NO |
| 184 | 2356 | | C>T | | INTRON 1 | 26744 | | NO |
| 185 | 2355 | | G>T | | INTRON 1 | 26800 | | NO |
| 186 | 2190 | | C>T | | INTRON 1 | 26812 | | NO |
| 187 | 2189 | | A>G | | INTRON 1 | 27177 | | NO |
| 188 | 2187 | | C>T | | INTRON 1 | 27195 | | NO |
| 189 | 2186 | | C>T | | INTRON 1 | 27226 | | NO |
| 190 | 2185 | | C>T | | INTRON 1 | 27240 | | NO |
| 191 | 2184 | | C>G | | INTRON 1 | 27247 | | NO |
| 192 | 1879 | | del8 | | INTRON 1 | 27998-05 | | NO |
| 193 | 2212 | | C>G | | 5'UTR | 28462 | | NO |
| 194 | 2211 | | A>G | | 5'END | 28619 | | NO |
| 195 | 2210 | | C>G | | 5'END | 28633 | | NO |
| 196 | 2209 | | A>G | | 5'END | 28671 | | NO |
| 197 | 2208 | | G>T | | 5'END | 28675 | | NO |
| 198 | 2207 | | A>G | | 5'END | 28704 | | NO |
| 199 | 2206 | | A>G | | 5'END | 28776 | | NO |
| 200 | 2205 | | A>T | | 5'END | 28863 | | NO |
| 201 | 2204 | | G>T | | 5'END | 28867 | | NO |
| 202 | 2203 | | A>G | | 5'END | 28922 | | NO |
| 203 | 2199 | | ins8 | | 5'END | 29163 | | NO |
| 204 | 2198 | | C>G | | 5'END | 29225 | | NO |
| 205 | 2197 | | A>G | | 5'END | 29298 | | NO |
| 206 | 2196 | | A>G | | 5'END | 29347 | | NO |
| 207 | 2195 | | A>G | | 5'END | 29376 | | NO |
| 208 | 2194 | | G>T | | 5'END | 29421 | | NO |
| 209 | 2193 | | C>T | | 5'END | 29472 | | NO |
| 210 | 2192 | | A>G | | 5'END | 29476 | | NO |
| 211 | 2191 | | C>T | | 5'END | 29504 | | NO |

### References

Albert P.R. & Lemonde S. (2004) 5-HT1A receptors, gene repression, and depression: guilt by association. Neuroscientist, 10, 575-93 Bailey J.N., Breidenthal S.E., Jorgensen M.J., McCracken J.T. & Fairbanks L.A. (2007) The association of DRD4 and novelty seeking is found in a nonhuman primate model. Psychiatr Genet, 17, 23-7Blokhuis H.J. & Arkes J.G. (1984) Some observations on the development of feather-pecking in poultry. Appl Anim Behav Sci, 12, 145-157 Blum K., Braverman E.R., Holder J.M., Lubar J.F., Monastra V.J., Miller D., Lubar J.O., Chen T.J. & Comings D.E. (2000) Reward deficiency syndrome: a biogenetic model for the diagnosis and treatment of impulsive, addictive, and compulsive behaviors. J Psychoactive Drugs, 32 Suppl, i-iv, 1-112 Bordnick P.S., Thyer B.A. & Ritchie B.W. (1994) Feather picking disorder and trichotillomania: an avian model of human psychopathology. J Behav Ther Exp Psychiatry, 25, 189-96 Callier S., Snapyan M., Le Crom S., Prou D., Vincent J.D. & Vernier P. (2003) Evolution and cell biology of dopamine receptors in vertebrates. Biol Cell, 95, 489-502 Cloninger C.R. (1986) A unified biosocial theory of personality and its role in the development of anxiety states. Psychiatr Dev, 4, 167-226Czesak M., Lemonde S., Peterson E.A., Rogaeva A. & Albert P.R. (2006) Cell-specific repressor or enhancer activities of Deaf-1 at a serotonin 1A receptor gene polymorphism. J Neurosci, 26, 1864-71 Dennis R.L., Chen Z.Q. & Cheng H.W. (2008) Serotonergic mediation of aggression in high and low aggressive chicken strains. Poult Sci, 87, 612-20 Ebstein R.P. (2006) The molecular genetic architecture of human personality: beyond self-report questionnaires. Mol Psychiatry, 11, 427-45Ewing B. & Green P. (1998) Base-calling of automated sequencer traces using phred. II. Error probabilities. Genome Res., 8, 186-194 Ewing B., Hillier L., Wendl M.C. & Green P. (1998) Base-calling of automated sequencer traces using phred. I. Accuracy assessment. Genome Res., 8, 175-185 Fidler A.E., van Oers K., Drent P.J., Kuhn S., Mueller J.C. & Kempenaers B. (2007) Drd4 gene polymorphisms are associated with personality variation in a passerine bird. Proc Biol Sci Gordon D., Abajian C. & Green P. (1998) Consed: a graphical tool for sequence finishing. Genome Res., 8, 195-202 Gremme G., Brendel V., Sparks M.E. & Kurtz S. (2005) Engineering a software tool for gene structure prediction in higher organisms. Information and Software Technology, 47, 965-978 Hejjas K., Vas J., Topal J., Szantai E., Ronai Z., Szekely A., Kubinyi E., Horvath Z., Sasvari-Szekely M. & Miklosi A. (2007) Association of polymorphisms in the dopamine D4 receptor gene and the activity-impulsivity endophenotype in dogs. Anim Genet, 38, 629-33 Hillier L.W., Miller W., Birney E., Warren W., Hardison R.C., Ponting C.P., Bork P., Burt D.W., Groenen M.A., Delany M.E., Dodgson J.B., Fingerprint Map S., Assembly G., Chinwalla A.T., Cliften P.F., Clifton S.W., Delehaunty K.D., Fronick C., Fulton R.S., Graves T.A., Kremitzki C., Layman D., Magrini V., McPherson J.D., Miner T.L., Minx P., Nash W.E., Nhan M.N., Nelson J.O., Oddy L.G., Pohl C.S., Randall-Maher J., Smith S.M., Wallis J.W., Yang S.P., Romanov M.N., Rondelli C.M., Paton B., Smith J., Morrice D., Daniels L., Tempest H.G., Robertson L., Masabanda J.S., Griffin D.K., Vignal A., Fillon V., Jacobbson L., Kerje S., Andersson L., Crooijmans R.P., Aerts J., van der Poel J.J., Ellegren H., Sequencing C., Caldwell R.B., Hubbard S.J., Grafham D.V., Kierzek A.M., McLaren S.R., Overton I.M., Arakawa H., Beattie K.J., Bezzubov Y., Boardman P.E., Bonfield J.K., Croning M.D., Davies R.M., Francis M.D., Humphray S.J., Scott C.E., Taylor R.G., Tickle C., Brown W.R., Rogers J., Buerstedde J.M., Wilson S.A., Sequencing, Libraries O., Stubbs L., Ovcharenko I., Gordon L., Lucas S., Miller M.M., Inoko H., Shiina T., Kaufman J., Salomonsen J., Skjoedt K., Wong G.K., Wang J., Liu B., Yu J., Yang H., Nefedov M., Koriabine M., Dejong P.J., And Annotation A., Goodstadt L., Webber C., Dickens N.J., Letunic I., Suyama M., Torrents D., von Mering C., Zdobnov E.M., Makova K., Nekrutenko A., Elnitski L., Eswara P., King D.C., Yang S., Tyekucheva S., Radakrishnan A., Harris R.S., Chiaromonte F., Taylor J., He J., Rijnkels M., Griffiths-Jones S., Ureta-Vidal A., Hoffman M.M., Severin J., Searle S.M., Law A.S., Speed D., Waddington D., Cheng Z., Tuzun E., Eichler E., Bao Z., Flicek P., Shteynberg D.D., Brent M.R., Bye J.M., Huckle E.J., Chatterji S., Dewey C., Pachter L., Kouranov A., Mourelatos Z., Hatzigeorgiou A.G., Paterson A.H., Ivarie R., Brandstrom M., Axelsson E., Backstrom N., Berlin S., Webster M.T., Pourquie O., Reymond A., Ucla C., Antonarakis S.E., Long M., Emerson J.J., Betran E., Dupanloup I., Kaessmann H., Hinrichs A.S., Bejerano G., Furey T.S., Harte R.A., Raney B., Siepel A., Kent W.J., Haussler D., Eyras E., Castelo R., Abril J.F., Castellano S., Camara F., Parra G., Guigo R., Bourque G., Tesler G., Pevzner P.A., Smit A., Management P., Fulton L.A., Mardis E.R. & Wilson R.K. (2004) Sequence and comparative analysis of the chicken genome provide unique perspectives on vertebrate evolution. Nature, 432, 695-716 Huggenvik J.I., Michelson R.J., Collard M.W., Ziemba A.J., Gurley P. & Mowen K.A. (1998) Characterization of a nuclear deformed epidermal autoregulatory factor-1 (DEAF-1)-related (NUDR) transcriptional regulator protein. Mol Endocrinol, 12, 1619-39 Jensen P., Buitenhuis B., Kjaer J., Zanella A., Mormede P. & Pizzari T. (2008) Genetics and genomics of animal behaviour and welfare - Challenges and possibilities. Appl Anim Behav Sci Jensik P.J., Huggenvik J.I. & Collard M.W. (2004) Identification of a nuclear export signal and protein interaction domains in deformed epidermal autoregulatory factor-1 (DEAF-1). J Biol Chem, 279, 32692-9 Kjaer J.B., Hjarvard B.M., Jensen K.H., Hansen-Møller J. & Naesbye Larsen (2004) Effects of haloperidol, a dopamine D2 receptor antagonist, in feather pecking behaviour in laying hens. Appl Anim Behav Sci, 86, 77-91 Kjaer J.B. & Sorensen P. (2002) Feather pecking and cannibalism in free-range laying hens as affected by genotype, dietary level of methionine + cystein, light intensity during rearing and age at first access to the range area. Appl Anim Behav Sci, 76, 21-39 Kjaer J.B., Sorensen P. & Su G. (2001) Divergent selection on feather pecking behaviour in laying hens (Gallus gallus domesticus). Appl Anim Behav Sci, 71, 229-239 Lemonde S., Turecki G., Bakish D., Du L., Hrdina P.D., Bown C.D., Sequeira A., Kushwaha N., Morris S.J., Basak A., Ou X.M. & Albert P.R. (2003) Impaired repression at a 5-hydroxytryptamine 1A receptor gene polymorphism associated with major depression and suicide. J Neurosci, 23, 8788-99 Lewis S.E., Searle S.M., Harris N., Gibson M., Lyer V., Richter J., Wiel C., Bayraktaroglir L., Birney E., Crosby M.A., Kaminker J.S., Matthews B.B., Prochnik S.E., Smithy C.D., Tupy J.L., Rubin G.M., Misra S., Mungall C.J. & Clamp M.E. (2002) Apollo: a sequence annotation editor. Genome Biol, 3, RESEARCH0082 Missale C., Nash S.R., Robinson S.W., Jaber M. & Caron M.G. (1998) Dopamine receptors: from structure to function. Physiol Rev, 78, 189-225 Momozawa Y., Takeuchi Y., Kusunose R., Kikusui T. & Mori Y. (2005) Association between equine temperament and polymorphisms in dopamine D4 receptor gene. Mamm Genome, 16, 538-44 Nickerson D.A., Tobe V.O. & Taylor S.L. (1997) PolyPhred: automating the detection and genotyping of single nucleotide substitutions using fluorescence-based resequencing. Nucleic Acids Res., 25, 2745-2751 Quackenbush J., Cho J., Lee D., Liang F., Holt I., Karamycheva S., Parvizi B., Pertea G., Sultana R. & White J. (2001) The TIGR Gene Indices: analysis of gene transcript sequences in highly sampled eukaryotic species. Nucleic Acids Res, 29, 159-64 Reif A. & Lesch K.P. (2003) Toward a molecular architecture of personality. Behav Brain Res, 139, 1-20 Sanna C.R., Li W.H. & Zhang L. (2008) Overlapping genes in the human and mouse genomes. BMC Genomics, 9, 169 Sang H. (2004) Prospects for transgenesis in the chick. Mech Dev 121: 1179-1186. Sang H. (2006) Transgenesis sunny-side up. Nat Biotechnol 24: 955-956. Scheet P. & Stephens M. (2006) A fast and flexible statistical model for large-scale population genotype data: applications to inferring missing genotypes and haplotypic phase. Am J Hum Genet, 78, 629-44 Su G., Kjaer J.B. & Sorensen P. (2005) Variance components and selection response for feather-pecking behavior in laying hens. Poult Sci, 84, 14-21 Sugiyama A., Inoue-Murayama M., Miwa M., Ohashi R., Kayang B.B., Mizutani M., Nirasawa K., Odai M., Minezawa M., Watanabe S. & Ito S. (2004) Polymorphism of dopamine receptor D4 exon I corresponding region in chicken. Zoolog Sci, 21, 941-6 Sun M., Hurst L.D., Carmichael G.G. & Chen J. (2005) Evidence for a preferential targeting of 3'-UTRs by cis-encoded natural antisense transcripts. Nucleic Acids Res, 33, 5533-43Tabaska J.E. & Zhang M.Q. (1999) Detection of polyadenylation signals in human DNA sequences. Gene, 231, 77-86 Tauson R. & Svensson S.A. (1980) Influence of plumage condition on the hen's feed requirement. Swedish Journal of Agricultural Research, 10, 35-39 van de Lavoir, M. C., J. H. Diamond, P. A. Leighton, C. Mather-Love, B. S. Heyer et al., (2006) Germline transmission of genetically modified primordial germ cells. Nature 441: 766-769. van Hierden Y.M., Koolhaas J.M., Kost'al L., Vyboh P., Sedlackova M., Rajman M., Jurani M. & Mechiel Korte S. (2005) Chicks from a high and low feather pecking line of laying hens differ in apomorphine sensitivity. Physiol Behav, 84, 471-7 van Hierden Y.M., Korte S.M., Ruesink E.W., van Reenen C.G., Engel B., Korte-Bouws G.A., Koolhaas J.M. & Blokhuis H.J. (2002) Adrenocortical reactivity and central serotonin and dopamine turnover in young chicks from a high and low feather-pecking line of laying hens. Physiol Behav, 75, 653-9

## Claims

1. A nucleic acid molecule contributing to or indicative of low feather pecking wherein said nucleic acid molecule is selected from the group consisting of:
a) a nucleic acid molecule having or comprising the nucleic acid sequence of SEQ ID NO: 1; and
b) a nucleic acid molecule of at least 12 contiguous nucleotides the complementary strand of which hybridizes under stringent conditions to the nucleic acid molecule of (a), wherein said nucleic acid molecule has
i) an adenosine at a position corresponding to position 10747, 11041, 11244, 11617, 12148, 12159, 12220, 12381, 12564, 14392, 14462, 14554, 14562, 14613, 14667, 14679, 14832, 15672, 16537, 16863, 16983, 17287, 17329, 17715, 18351, 18772, 21265, 21307, 21623, 22754, 22868, 23341, 23416, 23737, 23847, 23909, 24155 and/or 24574 of SEQ ID NO:1;
ii) a cytosine at a position corresponding to position 10252, 11238, 11271, 12184, 12327, 12338, 13286, 15154, 16911, 17369, 17435, 24051, 24213, 24247 and/or 25085 of SEQ ID NO:1;
iii) a guanosine at a position corresponding to position 10324, 11255, 11723, 12295, 13265, 13328, 14260, 14574, 14607, 14652, 14669, 14717, 14747, 14753, 14925, 15537, 15674, 16162, 17607, 18639, 18991, 19357, 22298, 23851, 23914, 25033 and/or 25062 of SEQ ID NO:1; and/or
iv) a thymidine at a position corresponding to position 10318, 11315, 11848, 12270, 12979, 14116, 14210, 14629, 14637, 14809, 17371, 17744, 18129, 18259, 21409, 23812, 24891, 25004, 25067 and/or 23850 of SEQ ID NO:1;

2. A nucleic acid molecule deviating by at least one mutation from the nucleic acid molecule of claim 1, wherein said mutation is contributing to or indicative of high feather pecking and is:
c) a substitution;
d) an insertion; and/or
e) a deletion.

3. The nucleic acid molecule of claim 2, wherein said substitution is a
i) adenosine to cytosine exchange at a position corresponding to position 10747, 11041, 18351, 23737 and/or 23909 of SEQ ID NO:1;
ii) cytosine to adenosine exchange at a position corresponding to position 11271 of SEQ ID NO:1;
iii) guanosine to adenosine exchange at a position corresponding to position 10324, 11255, 13265, 14260, 14607, 14652, 14747, 14753, 14925, 15537, 16162, 17607, 18639, 19357, 22298, 23851, 25033 and/or 25062 of SEQ ID NO:1;
iv) adenosine to guanosine exchange at a position corresponding to position 11244, 11617, 12148, 12159, 12220, 12381, 12564, 14392, 14462, 14554, 14562, 14613, 14667, 14679, 14832, 15672, 16537, 16863, 16983, 17287, 17329, 17715, 18772, 21265, 21307, 21623, 22754, 22868, 23341, 23416, 23847, 24155 and/or 24574 of SEQ ID NO:1;
v) thymidine to adenosine exchange at a position corresponding to position 11848 and/or 12270 of SEQ ID NO:1;
vi) guanosine to cytosine exchange at a position corresponding to position 12295, 13328, 14574, 14669 and/or 23914 of SEQ ID NO:1;
vii) cytosine to guanosine exchange at a position corresponding to position 12338, 15154, 17369, 17435 and/or 24213 of SEQ ID NO:1;
viii) thymidine to cytosine exchange at a position corresponding to position 10318, 11315, 11315, 14116, 14210, 14629, 14637, 14809, 17371, 17744, 18129, 18259, 21409, 23850, 24891, 25004 and/or 25085 of SEQ ID NO:1;
ix) cytosine to thymidine exchange at a position corresponding to position 10252, 11238, 12184, 12327, 13286, 16911, 24051 and/or 24247 of SEQ ID NO:1;
x) thymidine to guanosine exchange at a position corresponding to position 12979, 23812 and/or 25067 of SEQ ID NO:1; and/or a
xi) guanosine to thymidine exchange at a position corresponding to position 11723, 14717, 15674 and/or 18991 of SEQ ID NO:1.

4. The nucleic acid molecule of claim 2, wherein said insertion is a
i) 3 nucleotide insertion of adenosine-thymidine-cytosine at a position corresponding to position 14720 of SEQ ID NO:1;
ii) 2 nucleotide insertion of adenosine-adenosine at a position corresponding to position 13388 of SEQ ID NO:1; and/or a
iii) thymidine insertion at a position corresponding to position 16940 of SEQ ID NO:1.

5. The nucleic acid molecule of claim 2, wherein said deletion is a
i) deletion of a guanosine at a position corresponding to position 18678 of SEQ ID NO:1;
ii) 3 nucleotide deletion of thymidine-cytosine-thymidine at a position corresponding to position 17661 to 17663 of SEQ ID NO:1; and/or a
iii) thymidine deletion at a position corresponding to position 24568 of SEQ ID NO:1.

6. The nucleic acid molecule of any one of claims 1 to 5 which is DNA.

7. The nucleic acid molecule of any one of claims 1 to 6 which is genomic DNA.

8. A nucleic acid molecule of at least 12 contiguous nucleotides which is complementary to the nucleic acid molecule of any of claims 1 to 7.

9. A vector comprising the nucleic acid molecule of any one of claims 1 to 8.

10. A non-human host transformed with the vector of claim 9.

11. The non-human host of claim 10 which is a bacterium, a yeast cell, an insect cell, a fungal cell, an avian cell, a mammalian cell, a plant cell, a transgenic animal or a transgenic plant.

12. A method of testing an avian for its predisposition for low feather pecking comprising analyzing in a sample the nucleic acid molecule of claim 1 for nucleotide polymorphisms which are linked to said predisposition.

13. A method of testing an avian for its predisposition for high feather pecking comprising analyzing in a sample the nucleic acid molecule of any one of claims 2 to 5 for nucleotide polymorphisms which are linked to said predisposition.

14. The method of claim 12 or 13, wherein the sample is isolated from egg, semen, blood, feather, nail, skin or sputum of an avian.

15. A method for selecting an avian or part of an avian, including their semen or eggs, for low or high feather pecking, comprising the steps of:
a) testing for a predisposition for low or high feather pecking according to claim 12 or 13;
b) selecting the avian based on the information derived from said testing; and
c) optionally, using said information for further breeding considerations.

16. The method of any one of claims 12 to 15, wherein the avian is selected from the group consisting of chicken, turkey, pheasants, duck, goose, quail, parrots and ratites including ostrich, emu and cassowary.

17. A kit for the detection of an avian having a predisposition for low or high feather pecking, comprising a nucleic acid molecule according to any one of claims 1 to 8, in one or more container.
